(19) Europäisches Patentamt
European Patent Office
Office européen des brevets

(11) EP 4 211 598 B1

(12) **EUROPEAN PATENT SPECIFICATION**

(45) Date of publication and mention of the grant of the patent:
**07.01.2026 Bulletin 2026/02**

(21) Application number: **21772839.3**

(22) Date of filing: **11.09.2021**

(51) International Patent Classification (IPC):
***G06F 18/2413*** *(2023.01)*

(52) Cooperative Patent Classification (CPC):
**G06V 20/698; G06F 18/24133; G06V 10/454; G06V 10/751**

(86) International application number:
**PCT/IB2021/058273**

(87) International publication number:
**WO 2022/054009 (17.03.2022 Gazette 2022/11)**

(54) **A SCORING METHOD FOR AN ANTI-HER2 ANTIBODY-DRUG CONJUGATE THERAPY**

BEWERTUNGSVERFAHREN FÜR EINE ANTI-HER2-ANTIKÖRPER-WIRKSTOFF-KONJUGATTHERAPIE

PROCÉDÉ DE NOTATION DE THÉRAPIE PAR CONJUGUÉ ANTICORPS ANTI-HER2-MÉDICAMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.09.2020 US 202063077604 P**

(43) Date of publication of application:
**19.07.2023 Bulletin 2023/29**

(73) Proprietors:
- **AstraZeneca UK Limited**
  **Cambridge Biomedical Campus**
  **Cambridge CB2 0AA (GB)**
- **Daiichi Sankyo Company, Limited**
  **Tokyo 103-8426 (JP)**

(72) Inventors:
- **SCHMIDT, Guenter**
  **80999 Munich (DE)**
- **BRIEU, Nicolas**
  **81543 Munich (DE)**
- **SPITZMUELLER, Andreas**
  **83052 Bruckmuehl (DE)**
- **KAPIL, Ansh**
  **80339 Munich (DE)**

(74) Representative: **Marks & Clerk LLP**
**15 Fetter Lane**
**London EC4A 1BW (GB)**

(56) References cited:
**WO-A1-2019/020556**
**WO-A1-2019/110583**
**WO-A1-2019/224153**
**WO-A1-2020/023213**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

CROSS-REFERENCE TO RELATED APPLICATION

This application claims priority to U.S. Provisional Application No. 63/077,604, filed on September 12, 2020.

REFERENCE TO SEQUENCE LISTING SUBMITTED ELECTRONICALLY

The content of the electronically submitted sequence listing (Name: DSADC_400_Seqlisting.txt: Size: 24,662 bytes; and Date of Creation: September 9, 2021) is herein incorporated by reference in its entirety.

TECHNICAL FIELD

The present invention relates to a method for computing a score indicative of how a cancer patient will respond to a therapy that uses an antibody-drug conjugate having a drug conjugated to an anti-HER2 antibody via a linker structure.

BACKGROUND

Assessing a cancer patient's response probability to a given treatment is an essential step in determining a cancer patient's treatment regimen. Such an assessment is often based on histological analysis of tissue samples from a cancer patient and involves identifying and classifying cancers using standard grading schemes. Immunohistochemical (IHC) staining can be used to distinguish marker-positive cells that express a particular protein from marker-negative cells that do not express the protein. IHC staining typically involves multiple dyes, which includes one or more dyes connected to protein-specific antibodies and another dye that is a counterstain. A common counterstain is hematoxylin, which labels DNA and thus stains nuclei.

A protein specific stain or biomarker can be used to identify the regions of the tissue of the cancer patient that are likely to exhibit a response to a predetermined therapy. For example, a biomarker that stains epithelial cells can help to identify the suspected tumor regions. Then other protein specific biomarkers are used to characterize the cells within the cancerous tissue. The cells stained by a specific biomarker can be identified and quantified, and subsequently a score indicating the number of positively stained cells and negatively stained cells can be visually estimated by pathologists. This score can then be compared to scores of other cancer patients that have been calculated in the same way. If the response of these other patients to a given cancer treatment is known, the pathologist can predict, based on a comparison of the score calculated for the cancer patient with the scores of the other patients, how likely the cancer patient is to respond to a given treatment. However, visual assessment by pathologists is prone to variability and subjectivity.

One promising cancer treatment involves an antibody-drug conjugate (ADC) having a drug with cytotoxicity conjugated to an antibody, whose antigen is expressed on the surface of cancer cells. The ADC binds to the antigen and undergoes cellular internalization so as to deliver the drug selectively to cancer cells and to accumulate the drug within those cancer cells and kill them. A computer-based method is sought for generating a repeatable and objective score indicating a cancer patient's response to a treatment involving a therapeutic HER2 antibody-drug conjugate.

SUMMARY

A method for predicting how a cancer patient will respond to a therapy involving an antibody drug conjugate (ADC) involves computing a response score based on single-cell ADC scores for each cancer cell. The ADC includes an ADC payload and an ADC antibody that targets a protein on each cancer cell. A tissue sample is immunohistochemically stained using a dye linked to a diagnostic antibody that binds to the protein on the cancer cells in the tissue sample. A digital image of the tissue sample is acquired. Image analysis is performed on the digital image to detect the cancer cells using a convolutional neural network. For each cancer cell, a single-cell ADC score is computed based on the staining intensities of the dye in the membrane and/or cytoplasm of the cancer cell and/or in the membranes and cytoplasms of other cancer cells that are closer than a predefined distance to the cancer cell. A response score is generated that predicts the response of the cancer patient to the ADC therapy by aggregating all single-cell ADC scores of the tissue sample using a statistical operation. Patients having a response score higher than a predetermined threshold are recommended for a therapy involving the ADC.

In a non-claimed embodiment, a method of generating a score indicative of a survival probability of a cancer patient treated with an antibody drug conjugate (ADC) involves computing single-cell ADC scores. A tissue sample of the cancer patient is immunohistochemically stained using a dye linked to a diagnostic antibody. The ADC includes an ADC payload and an ADC antibody that targets a human epidermal growth factor receptor 2 (HER2) protein on cancer cells. The diagnostic antibody binds to the HER2 protein on cancer cells in the tissue sample. A digital image of the tissue sample is

acquired, and cancer cells in the digital image are detected using image analysis. For each cancer cell, a single-cell ADC score is computed based on the staining intensity of the dye in the membrane. The single-cell ADC score may also optionally be based on the staining intensity of the dye in the cytoplasm of the cancer cell, as well as on the staining intensities of the dye in the membranes and the cytoplasms of other cancer cells that are closer than a predefined distance to the cancer cell. The resulting Quantitative Continuous Score (QCS), which is indicative of the survival probability of the cancer patient, is generated by aggregating all single-cell ADC scores of the tissue sample using a statistical operation. The aggregating of all single-cell ADC scores is performed by determining the mean, determining the median, or determining a quantile with a predefined percentage. In another aspect, the aggregation of all single-cell ADC scores involves a threshold operation using a predefined threshold. All cells having a single-cell ADC score larger than the predefined threshold are labeled single-cell ADC positive. The aggregation is performed by determining the number of single-cell ADC positive cells divided by the number of all cancer cells.

In a non-claimed embodiment, a method of predicting a response of a cancer patient to an antibody drug conjugate (ADC) involves detecting cancer cells and computing a single-cell ADC score for each cancer cell. The ADC includes an ADC payload and an ADC antibody that targets a protein on a cancer cell. The protein is human epidermal growth factor receptor 2 (HER2). A tissue sample is immunohistochemically stained using a dye linked to a diagnostic antibody. The diagnostic antibody binds to the protein on the cancer cells in the tissue sample. A digital image of the tissue sample is acquired, and cancer cells are detected in the digital image. For each cancer cell, a single-cell ADC score is computed based on the staining intensity of the dye in the membrane. The single-cell ADC score may also optionally be based on the staining intensity of the dye in the cytoplasm of the cancer cell, and based on the staining intensities of the dyes in the membranes and cytoplasms of other cancer cells that are closer than a predefined distance to the cancer cell. The staining intensity of each membrane is computed based on the average optical density of a brown diaminobenzidine (DAB) signal in pixels of the membrane, and the staining intensity of each cytoplasm is computed based on the average optical density of the brown DAB signal in pixels of the cytoplasm. The response of the cancer patient to the ADC is predicted based on an aggregation of all single-cell ADC scores of the tissue sample using a statistical operation.

In another non-claimed embodiment, a method of identifying a cancer patient who will exhibit a predetermined response to an antibody drug conjugate (ADC) involves generating a response score. The ADC includes an ADC payload and an ADC antibody that targets a protein on a cancer cell. A tissue sample of the cancer patient is immunohistochemically stained using a dye linked to a diagnostic antibody that binds to the protein on the cancer cells in the tissue sample. A digital image of the tissue sample is acquired, and cancer cells in the digital image are detected using a convolutional neural network. For each cancer cell, a single-cell ADC score is computed based on staining intensity of the dye in the membrane. The single-cell ADC score may also optionally be based on the staining intensity of the dye in the cytoplasm of the cancer cell, and on the staining intensities of the dye in the membranes and the cytoplasms of other cancer cells that are closer than a predefined distance to the cancer cell for which the single-cell score is computed. The QCS score is a response score generated by aggregating all single-cell ADC scores of the tissue sample using a statistical operation. The cancer patient is identified as one who will exhibit the predetermined response to the ADC based on whether the QCS score exceeds a threshold. Patients exhibiting a QCS score larger than the threshold are considered QCS Positive (QCS+), and all other patients are considered QCS Negative (QCS-). The predetermined response is a reduction in average tumor size. In another aspect, the difference between the QCS score to the threshold is indicative of the probability that the cancer patient is correctly identified as one who will exhibit the predetermined response to the ADC. A small difference indicates a low probability, whereas a large difference indicates a high probability that the cancer patient is accurately identified. In another embodiment, the predetermined response is indicated by patient survival so that patient death within a pre-defined time span is considered as non-response. In one aspect, the pre-defined time span is twice the average survival time of patients treated according to the Standard Of Care.

In one embodiment, a method of treating a cancer patient with an antibody drug conjugate (ADC) involves generating the QCS score in the form of a treatment score. The ADC includes an ADC payload and an ADC antibody that targets a protein on a cancer cell. The protein is human epidermal growth factor receptor 2 (HER2). A tissue sample of the cancer patient is immunohistochemically stained using a dye linked to a diagnostic antibody that binds to the protein on the cancer cells in the tissue sample. A digital image of the tissue sample is acquired, and cancer cells in the digital image are detected. For each cancer cell, a single-cell ADC score is calculated based on the staining intensity of the dye in the membrane. The single-cell ADC score may also optionally be based on the staining intensity of the dye in the cytoplasm of the cancer cell, as well as on the staining intensities of the dyes in the membranes and cytoplasms of other cancer cells that are closer than a predefined distance to the cancer cell. The treatment score is generated by aggregating all single-cell ADC scores of the tissue sample using a statistical operation. A therapy involving the ADC is administered to the cancer patient if the treatment score exceeds a predetermined threshold. In another aspect, a therapy involving the ADC is administered to a cancer patient only if the patient’s pre-treatment, HER2 stained tissue sample is scored as QCS Positive (QCS+).

In another embodiment, a method of treating a cancer patient with an antibody drug conjugate (ADC) is performed by a clinician who administers a therapy. The ADC includes an ADC payload and an ADC antibody that

targets a protein on a cancer cell. The protein is human epidermal growth factor receptor 2 (HER2). The therapy involving the ADC is administered to the cancer patient if a response score exceeds a predetermined threshold. The QCS response score was generated by aggregating single-cell ADC scores of a tissue sample of the cancer patient using a statistical operation. Each single-cell ADC score was computed for each cancer cell based on staining intensity of a dye in the membrane. The single-cell ADC score may also optionally be based on the staining intensity of the dye in the cytoplasm of each cancer cell and also based on the staining intensities of the dye in the membranes and cytoplasms of other cancer cells that are closer than a predefined distance to each cancer cell for which the single-cell ADC score was computed. The cancer cells were detected in a digital image of the tissue sample of the cancer patient. The tissue sample was immunohistochemically stained using the dye linked to a diagnostic antibody that binds to the protein on the cancer cells in the tissue sample.

Other embodiments and advantages are described in the detailed description below. This summary does not purport to define the invention. The invention is defined by the claims.

BRIEF DESCRIPTION OF THE DRAWINGS

The accompanying drawings, where like numerals indicate like components, illustrate embodiments of the invention.

FIG. 1 shows the amino acid sequence (SEQ ID No: 1) of the HER2 protein.
FIG. 2 shows the amino acid sequence (SEQ ID No: 2) of a heavy chain of the anti-HER2 antibody.
FIG. 3 shows the amino acid sequence (SEQ ID No: 3) of a light chain of the anti-HER2 antibody.
FIG. 4 shows the amino acid sequence (SEQ ID No: 4) of CDRH1 of the anti-HER2 antibody.
FIG. 5 shows the amino acid sequence (SEQ ID No: 5) of CDRH2 of the anti-HER2 antibody.
FIG. 6 shows the amino acid sequence (SEQ ID No: 6) of CDRH3 of the anti-HER2 antibody.
FIG. 7 shows the amino acid sequence (SEQ ID No: 7) of CDRL1 of the anti-HER2 antibody.
FIG. 8 shows the amino acid sequence (SEQ ID No: 8) of CDRL2 (SAS) of the anti-HER2 antibody.
FIG. 9 shows the amino acid sequence (SEQ ID No: 9) of CDRL3 of the anti-HER2 antibody.
FIG. 10 shows the amino acid sequence (SEQ ID No: 10) of a heavy chain variable region of the anti-HER2 antibody.
FIG. 11 shows the amino acid sequence (SEQ ID No: 11) of a light chain variable region of the anti-HER2 antibody.
FIG. 12 shows the amino acid sequence (SEQ ID No: 12) of a heavy chain of the anti-HER2 antibody.
FIG. 13 illustrates the anti-HER2 antibody-drug conjugate trastuzumab deruxtecan with eight drug-linker units.
FIG. 14 is a flowchart of steps by which an analysis system analyzes digital images of tissue from a cancer patient and predicts how the cancer patient will likely respond to a therapy involving an anti-HER2 antibody-drug conjugate.
FIG. 15 shows digital images illustrating the image analysis process of step 2 of FIG. 14.
FIG. 16 shows digital images of another embodiment of the image analysis process of step 2 of FIG. 14.
FIG. 17 illustrates image analysis steps in which nucleus objects of cancer cells are detected.
FIG. 18 illustrates image analysis steps in which nucleus objects are used to detect membranes.
FIG. 19 illustrates image analysis steps in which membrane objects of cancer cells are detected.
FIG. 20 is a screenshot of the results of the image analysis steps in an image analysis software environment.
FIG. 21 shows a script used for image analysis segmentation and the cell object measurements obtained using the script.
FIG. 22 shows sample quantitative results of staining intensities from image analysis using gray values of membrane and cytoplasm pixels.
FIG. 23 lists the exemplary quantitative amounts of staining on the membranes and in the cytoplasms of the image of FIG. 22.
FIG. 24 illustrates the mechanism by which an anti-HER2 ADC therapy kills cancer cells.
FIG. 25 illustrates the calculation of the single-cell ADC score for each of the three cells shown in FIG. 23 based on cell separation to account for the uptake of the ADC payload into neighboring cells.
FIG. 26 shows a formula by which a single-cell score is calculated.
FIG. 27 is a diagram comparing how consistently pathologists annotate membranes compared to one another.
FIG. 28 is a diagram comparing how membranes detected using image analysis of the method of FIG. 14 correlate to membranes identified by pathologists.
FIG. 29 is a diagram showing whether each of fifty patients with HER2 IHC 1+ and HER2 IHC 2+/ISH- scores had a progressive disease, a stable disease or exhibited a response to the anti-HER2 ADC therapy.
FIG. 30 shows the response in terms of tumor shrinkage of patients whose scores from the novel scoring method were categorized as "HER2 Positive".
FIG. 31 shows that a subgroup of patients from the "HER2 Negative" category of IHC scoring still exhibited a favorable objective response rate to the anti-HER2 ADC therapy.

FIG. 32 shows the stratification of patients from the conventional "HER2 Negative" category into "QCS Positive" and "QCS Negative" using the novel scoring method of FIG. 14.

FIG. 33 illustrates the stratification of 151 patients into "QCS Positive" and "QCS Negative" using the novel scoring method of FIG. 14.

FIG. 34A is a bar graph showing the novel response score for breast cancer patients whose conventional HER2 IHC score is indicated by the shading of the bar.

FIG. 34B is a table showing the objective response rate (ORR) and the median progression-free survival (mPFS) period of the QCS Positive and QCS Negative patients stratified in FIG. 34A.

FIG. 35 is a graph of Kaplan-Meyer curves of progression-free survival for two groups of HER2-negative patients identified using an embodiment of the novel method in which single-cell-ADC-score negative cancer cells that neighbor single-cell-ADC-score positive cancer cells are also considered.

FIG. 36 is a graph of Kaplan-Meier curves of progression-free survival for two patient groups of the entire J101 trial identified using the novel QCS scoring method.

FIG. 37 is a table of features used in the novel QCS scoring method to stratify the two groups of patients shown in the Kaplan-Meier curve of FIG. 36.

FIG. 38 is a table of tumor infiltrating lymphocytes (TIL)-based and HER2-staining-based features of a model used to stratify 151 breast cancer patients into those exhibiting longer and shorter progression free survival (PFS) in response to an anti-HER2 ADC therapy.

FIG. 39A is a Kaplan-Meier curve for the TIL density feature listed in FIG. 38.

FIG. 39B is a Kaplan-Meier curve for the HER2+ cell density feature listed in FIG. 38.

FIG. 39C is a Kaplan-Meier curve for the HER2+ neighborhood score feature listed in FIG. 38.

FIG. 40A is a Kaplan-Meier curve for the TIL density feature listed in FIG. 38 for just 72 patients designated as HER2 positive from among the 151 patients of the J101 trial.

FIG. 40B is a Kaplan-Meier curve for the HER2+ cell density feature for just 72 HER2 positive patients of the total 151 patients in the J101 trial.

FIG. 40C is a Kaplan-Meier curve for the HER2+ neighborhood score feature for just 72 HER2 positive patients of the total 151 patients in the J101 trial.

FIG. 41A is a Kaplan-Meier curve for the TIL density feature for just 65 HER2 negative patients of the total 151 patients in the J101 trial.

FIG. 41B is a Kaplan-Meier curve for the HER2+ cell density feature for just 65 HER2 negative patients of the total 151 patients in the J101 trial.

FIG. 41C is a Kaplan-Meier curve for the HER2+ neighborhood score feature for just 65 HER2 negative patients of the total 151 patients in the J101 trial.

FIG. 42 is a bar graph showing the novel response score for gastric cancer patients whose conventional HER2 IHC score is indicated by the shading of the bar.

FIG. 43 is a table of HER2-staining-based features of a model used to stratify gastric cancer patients into those exhibiting longer and shorter progression free survival (PFS) in response to an anti-HER2 ADC therapy.

FIG. 44 is a table of HER2-staining-based features of a model used to stratify gastric cancer patients into those exhibiting longer and shorter overall survival (OS) in response to an anti-HER2 ADC therapy.

FIG. 45 is a Kaplan-Meier curve for the feature membOD_density_10 listed in FIG. 43 based on PFS for 32 gastric cancer patients of the J101 trial.

FIG. 46 is a Kaplan-Meier curve for the feature membOD_density_10 listed in FIG. 44 based on OS for 32 gastric cancer patients of the J101 trial.

## DETAILED DESCRIPTION

The present invention provides a novel method for computing a score indicative of how a cancer patient will respond to a therapy that uses an anti-HER2 antibody-drug conjugate. Another aspect of the invention relates to a method for computing a score for a cancer patient indicative of a survival probability of a cancer patient treated with the ADC. Another aspect of the invention relates to a method for predicting a response of a cancer patient to the ADC. Another aspect of the invention relates to identifying a cancer patient who will exhibit a predetermined response to the ADC. Yet another aspect of the invention relates to a method of treating a cancer patient by administering a therapy involving the ADC if a treatment score exceeds a predetermined threshold.

### I. **Definitions.**

To facilitate an understanding of the present invention, a number of terms and phrases are defined below. The term "cancer" is used to have the same meaning as that of the term "tumor".

In the present invention, "HER2" is synonymous with human epidermal growth factor receptor 2 (which may also be referred to as neu or ErbB-2) and is a transmembrane receptor belonging to the epidermal growth factor receptor (EGFR) subfamily of receptor protein tyrosine kinases together with HER1 (EGFR or ErbB-1), HER3 (ErbB-3), and HER4 (ErbB-4). HER2 is known to play an important role in cell proliferation, differentiation, and survival in normal cells and tumor cells by being activated by autophosphorylation of intercellular tyrosine residues due to heterodimer formation with HER1, HER3, or HER4. In the present invention, the term "HER2 protein" is used in the same meaning as HER2. The expression of the HER2 protein can be detected using a method well known to those skilled in the art, such as immunohistochemistry (IHC) or immunofluorescence (IF).

FIG. 1 shows the amino acid sequence (SEQ ID No. 1) of the HER2 protein. In SEQ ID No. 1, the amino acid sequence consisting of amino acid residues 1 through 652 is referred to as the "extracellular domain of the HER2 protein", the amino acid sequence consisting of amino acid residues 653 through 675 is referred to as the "transmembrane domain of the HER2 protein", and the amino acid sequence consisting of amino acid residues 676 through 1255 is referred to as the "intercellular domain of the HER2 protein".

In the present invention, "anti-HER2 antibody" means an antibody that specifically binds to HER2. The anti-HER2 antibody has an activity of binding to HER2 and is thereby internalized into HER2-expressing cells, such that after exhibiting the activity of binding to HER2, the antibody moves into the HER2 expressing cells. The anti-HER2 antibody targets tumor cells, binds to the tumor cells, internalizes into the tumor cells, exhibits cytocidal activity against the tumor cells, and can be conjugated with a drug having antitumor activity via a linker to form an antibody-drug conjugate.

FIG. 2 shows the amino acid sequence (SEQ ID No. 2) of a heavy chain of the anti-HER2 antibody, and FIG. 3 shows the amino acid sequence (SEQ ID No. 3) of a light chain of the anti-HER2 antibody.

FIG. 4 shows the amino acid sequence (SEQ ID No: 4) of CDRH1 of the anti-HER2 antibody, FIG. 5 shows the amino acid sequence (SEQ ID No: 5) of CDRH2 of the anti-HER2 antibody, and FIG. 6 shows the amino acid sequence (SEQ ID No: 6) of CDRH3 of the anti-HER2 antibody.

FIG. 7 shows the amino acid sequence (SEQ ID No: 7) of CDRL1 of the anti-HER2 antibody, FIG. 8 shows the amino acid sequence (SEQ ID No: 8) of CDRL2 (SAS) of the anti-HER2 antibody, and FIG. 9 shows the amino acid sequence (SEQ ID No: 9) of CDRL3 of the anti-HER2 antibody.

FIG. 10 shows the amino acid sequence (SEQ ID No: 10) of a heavy chain variable region of the anti-HER2 antibody, and FIG. 11 shows the amino acid sequence (SEQ ID No: 11) of a light chain variable region of the anti-HER2 antibody. FIG. 12 shows the amino acid sequence (SEQ ID No: 12) of another heavy chain of the anti-HER2 antibody.

An anti-HER2 antibody of the anti-HER2 antibody-drug conjugate used in the present invention is preferably an antibody comprising a heavy chain comprising CDRH1 consisting of an amino acid sequence represented by SEQ ID No. 4 (an amino acid sequence consisting of amino acid residues 26 through 33 of SEQ ID No. 2), CDRH2 consisting of an amino acid sequence represented by SEQ ID No. 5 (an amino acid sequence consisting of amino acid residues 51 through 58 of SEQ ID No. 2) and CDRH3 consisting of an amino acid sequence represented by SEQ ID No. 6 (an amino acid sequence consisting of amino acid residues 97 through 109 of SEQ ID No. 2), and a light chain comprising CDRL1 consisting of an amino acid sequence represented by SEQ ID No. 7 (an amino acid sequence consisting of amino acid residues 27 through 32 of SEQ ID No. 3), CDRL2 consisting of an amino acid sequence consisting of amino acid residues 1 through 3 of SEQ ID No. 8 (an amino acid sequence consisting of amino acid residues 50 to 52 of SEQ ID No. 3) and CDRL3 consisting of an amino acid sequence represented by SEQ ID No. 9 (an amino acid sequence consisting of amino acid residues 89 through 97 of SEQ ID No. 3), and more preferably an antibody comprising a heavy chain variable region consisting of an amino acid sequence represented by SEQ ID NO: 10 (an amino acid sequence consisting of amino acid residues 1 through 120 of SEQ ID No. 2) and a light chain variable region consisting of an amino acid sequence represented by SEQ ID No. 11 (an amino acid sequence consisting of amino acid residues 1 through 107 of SEQ ID No. 3), and even more preferably an antibody comprising a heavy chain consisting of an amino acid sequence represented by SEQ ID No. 2 and a light chain consisting of an amino acid sequence represented by SEQ ID No. 3; or an antibody comprising a heavy chain consisting of an amino acid sequence represented by SEQ ID No. 12 (an amino acid sequence consisting of amino acid residues 1 through 449 of SEQ ID No. 2) and a light chain consisting of an amino acid sequence represented by SEQ ID No. 3.

In the present invention, the terms "HER2-positive" and "HER2-overexpressing" refer to cancer tissue given a score of 3+ for the expression of HER2 in an immunohistochemical method, as well as cancer that both is given a score of 2+ for the expression of HER2 in an immunohistochemical method and is determined as positive for the expression of HER2 in an in situ hybridization method. The in situ hybridization method of the present invention includes a fluorescence in situ hybridization method (FISH) and a dual color in situ hybridization method (DISH).

In the present invention, the term "HER2-negative" refers to cancer tissue given a score of 0 for the expression of HER2 in an immunohistochemical method, cancer given a score of 1+ for the expression of HER2 in an immunohistochemical method, and cancer that both is given a score of 2+ for the expression of HER2 in an immunohistochemical method and is determined as negative for the expression of HER2 in an in situ hybridization method.

**In** the present invention, the term "HER2-low" refers to cancer tissue given a score of 0+ (meaning >0 and <1+) for the expression of HER2 in an immunohistochemical method, cancer given a score of 1+ for the expression of HER2 in an

immunohistochemical method, and cancer that both is given a score of 2+ for the expression of HER2 in an immunohistochemical method and is determined as negative for the expression of HER2 in an in situ hybridization method. Tissue that is categorized as 0+ exhibits a very weak but non-noise HER2 expression.

**In** the present invention, the term "QCS Positive" (QCS+) refers to cancer that is likely to show a response to an anti-HER2 ADC therapy. The term "QCS Negative" (QCS-) refers to cancer that is unlikely to show a response to an anti-HER2 ADC therapy. The acronym QCS stands for Quantitative Continuous Score. The result of the novel predictive method of the present invention is generally referred to as a Quantitative Continuous Score and may be a response score, a treatment score or an indication of predicted survival time. The QCS score is obtained by performing statistical operations on all of the single-cell ADC scores obtained for a patient. Applying a predetermined threshold to the QCS scores discriminates between "QCS Positive" and "QCS Negative" patients. Stratifying the cancer patients into the QCS Positive and QCS Negative groups enables the identification of those QCS+ patients who likely benefit from the therapy which involves the ADC.

**II. The anti-HER2 antibody-drug conjugate.**

In the present invention, a partial structure consisting of a linker and the drug of the anti-HER2 antibody-drug conjugate is referred to as the "drug-linker". The drug-linker can be conjugated to the anti-HER2 antibody via a thioether bond. Thus, the drug-linker can be connected to a thiol group (the sulfur atom of a cysteine residue) formed at an interchain disulfide bond site (two sites between heavy chains and two sites between a heavy chain and a light chain) of the antibody. The anti-HER2 antibody-drug conjugate used in the present invention is preferably an anti-HER2 antibody-drug conjugate in which the drug-linker is represented by the following formula:

[Formula 1]

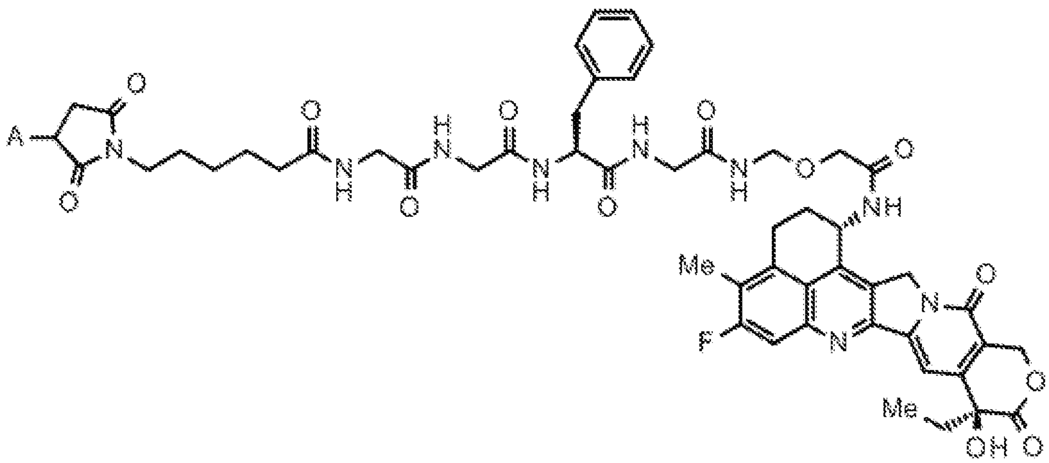

wherein A represents a connecting position to the anti-HER2 antibody.

The drug-linker of the present invention includes exatecan (IUPAC name: (1S,9S)-1-amino-9-ethyl-5-fluoro-1,2,3,9,12,15-hexahydro-9-hydroxy-4-methyl-10H,13H-benzo[de]pyrano[3',4':6,7]indolizino[1,2-b]quinoline-10,13-dione, (also expressed as chemical name: (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione)), which is a topoisomerase I inhibitor. Exatecan is the cytotoxic payload of the anti-body drug conjugate and has an antitumor effect. Exatecan is a camptothecin derivative represented by the following formula:

[Formula 2]

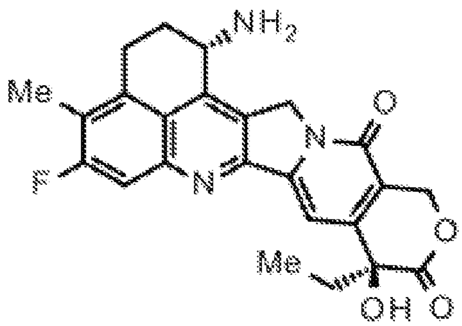

The anti-HER2 antibody-drug conjugate used in the present invention is preferably trastuzumab deruxtecan, also known as DS-8201. The anti-HER2 antibody-drug conjugate used in the present invention can also be represented by the following formula:

[Formula 3]

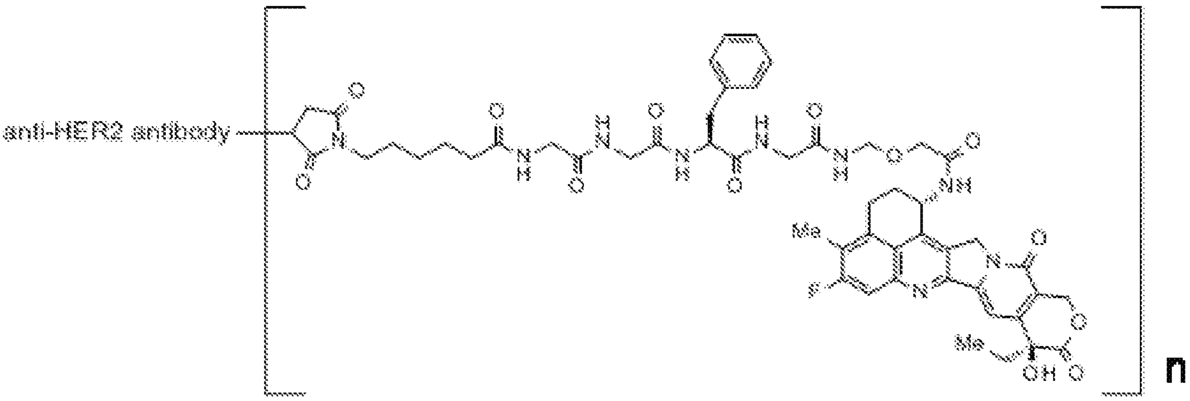

The drug-linker shown in the square brackets is conjugated to the anti-HER2 antibody via a thioether bond. The meaning of n is the same as that of what is called the average number of conjugated drug molecules (the drug-to-antibody ratio DAR), and indicates the average number of units of the drug-linker conjugated per antibody molecule.

The average number of units of the drug-linker conjugated per antibody molecule in the anti-HER2 antibody-drug conjugate used in the present invention is preferably 2 to 8, more preferably 3 to 8, even more preferably 7 to 8, and even more preferably about 8.

FIG. 13 illustrates the anti-HER2 antibody-drug conjugate trastuzumab deruxtecan (DS-8201) with eight drug-linker units designated as "DL". The trastuzumab portion of trastuzumab deruxtecan shown in FIG. 13 is humanized anti-HER2 IgG1 mAb, wherein IgG1 indicates the isotype of the anti-HER2 antibody.

After undergoing cellular internalization into cancer cells, the anti-HER2 antibody-drug conjugate used in the present invention is cleaved at a linker moiety and releases the compound represented by the following formula:

[Formula 4]

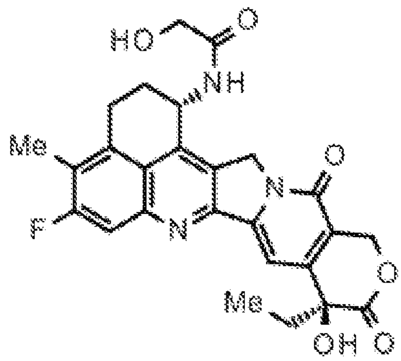

The compound shown above is the primary source of antitumor activity of the anti-HER2 antibody-drug conjugate used in the present invention, and has a topoisomerase I inhibitory effect.

The anti-HER2 antibody-drug conjugate used in the present invention also has a bystander effect in which the anti-HER2 antibody-drug conjugate is internalized into cancer cells that express the target protein HER2, and the compound shown above then also exerts an antitumor effect on neighboring cancer cells that do not express the target protein HER2.

### III. Production of the anti-HER2 antibody.

The anti-HER2 antibody-drug conjugate used in the present invention can be produced as disclosed in International Publication No. WO 2015/115091.

The anti-HER2 antibody used in the present invention can be obtained by immunizing animals with any polypeptide selected from HER2 serving as an antigen or the amino acid sequence of HER2, collecting an antibody produced in vivo, and then purifying the antibody. The origin of the antigen is not limited to humans, and animals may be immunized with an antigen derived from a non-human animal such as a mouse, a rat and the like. In this case, the cross-reactivity of antibodies binding to the obtained heterologous antigen with human antigens can be tested to screen for an anti-HER2 antibody applicable to a human disease.

Alternatively, antibody-producing cells that produce antibodies against the antigen are fused with myeloma cells to establish hybridomas, from which monoclonal antibodies are in turn obtained.

The antigen can be obtained by genetically engineering host cells to produce a gene that encodes the antigenic protein. Specifically, vectors that permit expression of the antigen gene are prepared and transferred to host cells so that the gene is expressed. The antigen thus expressed can be purified. The antibody can also be obtained by a method of immunizing animals with the above-described genetically engineered antigen-expressing cells or a cell line expressing the antigen.

The anti-HER2 antibody used in the present invention is a recombinant antibody obtained by artificial modification for the purpose of decreasing heterologous antigenicity to humans such as a chimeric antibody or a humanized antibody, or is an antibody having only the gene sequence of an antibody derived from a human, that is, a human antibody. As the chimeric antibody, an antibody in which antibody variable and constant regions are derived from different species, for example, a chimeric antibody in which a mouse-derived or rat-derived antibody variable region is connected to a human-derived antibody constant region can be exemplified.

For the humanized antibody, the antibody is obtained by integrating only the complementarity determining region (CDR) of a heterologous antibody into a human-derived antibody, or the antibody is obtained by grafting a part of the amino acid residues of the framework of a heterologous antibody as well as the CDR sequence of the heterologous antibody to a human antibody by a CDR-grafting method (WO 90/07861), or the antibody is humanized using a gene conversion mutagenesis strategy (U.S. Patent No. 5821337).

For the human antibody, the antibody is generated by using a human antibody-producing mouse having a human chromosome fragment including genes of a heavy chain and light chain of a human antibody. As an alternative, the antibody is obtained by phage display, the antibody being selected from a human antibody library.

In the present invention, modified variants of the anti-HER2 antibody can also be used. A modified variant refers to a variant obtained by subjecting the antibody according to the present invention to chemical or biological modification. Examples of the chemically modified variant include variants including a linkage of a chemical moiety to an amino acid skeleton, variants including a linkage of a chemical moiety to an N-linked or O-linked carbohydrate chain. Examples of the biologically modified variant include variants obtained by post-translational modification (such as N-linked or O-linked glycosylation, N-terminal or C-terminal processing, deamidation, isomerization of aspartic acid, or oxidation of methionine), and variants in which a methionine residue has been added to the N terminus by being expressed in a prokaryotic

host cell. Furthermore, an antibody labeled so as to enable the detection or isolation of the anti-HER2 antibody used in the present invention or the antigen, for example, an enzyme-labeled antibody, a fluorescence-labeled antibody, and an affinity-labeled antibody are also included in the meaning of the modified variant. Such a modified variant of the anti-HER2 antibody used in the present invention is useful for improving the stability and blood retention of the antibody, reducing the antigenicity thereof, and detecting or isolating an antibody or an antigen.

In addition, by regulating the modification of a glycan that is linked to the anti-HER2 antibody used in the present invention (glycosylation, defucosylation, etc.), it is possible to enhance antibody-dependent cellular cytotoxic activity. Techniques for regulating the modification of a glycan of antibodies are disclosed in WO99/54342, WO00/61739 and WO02/31140. However, the techniques are not limited thereto. Antibodies in which the modification of a glycan is regulated can also be used as the anti-HER2 antibody of the present invention.

It is known that a lysine residue at the carboxyl terminus of the heavy chain of an antibody produced in a cultured mammalian cell is deleted, and it is also known that two amino acid residues (glycine and lysine) at the carboxyl terminus of the heavy chain of an antibody produced in a cultured mammalian cell are deleted and a proline residue newly located at the carboxyl terminus is amidated. However, such deletion and modification of the heavy chain sequence do not affect the antigen-binding affinity and the effector function (the activation of complement, antibody-dependent cellular cytotoxicity, etc.) of the antibody. Therefore, in the anti-HER2 antibody used in the present invention, antibodies subjected to such modification and functional fragments of the antibody are also included, and deletion variants in which one or two amino acids have been deleted at the carboxyl terminus of the heavy chain, variants obtained by amidation of deletion variants (for example, a heavy chain in which the carboxyl terminal proline residue has been amidated), and the like are also included. The type of deletion variant having a deletion at the carboxyl terminus of the heavy chain of the anti-HER2 antibody used in the present invention is not limited to the above variants as long as the antigen-binding affinity and the effector function are conserved. The two heavy chains constituting the anti-HER2 antibody used in the present invention may be of one type selected from the group consisting of a full-length heavy chain and the above-described deletion variant, or may be of two types in combination selected therefrom. The ratio of the amount of each deletion variant can be affected by the type of cultured mammalian cells that produce the anti-HER2 antibody used in the present invention and the culture conditions. However, an antibody in which one amino acid residue at the carboxyl terminus has been deleted in both of the two heavy chains in the anti-HER2 antibody can also be used in the present invention.

**IV. Producing the anti-HER2 antibody-drug conjugate.**

A drug-linker intermediate that can be used in the production of the anti-HER2 antibody-drug conjugate of the present invention is represented by the following formula.

[Formula 5]

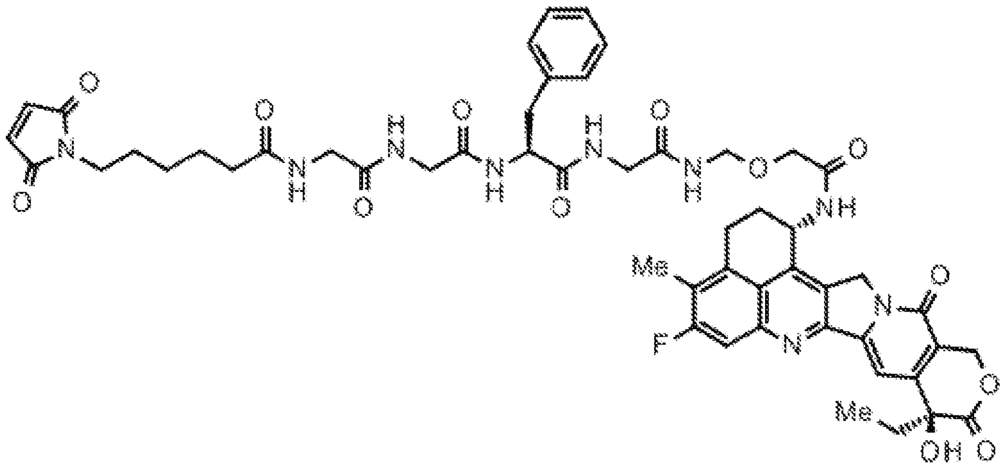

The drug-linker intermediate can be expressed as a chemical name, N-[6-(2,5-dioxo-2,5-dihydro-1H-pyrrole-1-yl)hexanoyl]glycylglycyl-L-phenylalanyl-N-[(2-{[(1S,9S)-9-ethyl-5-fluoro-9-hydroxy-4-methyl-10,13-dioxo-2,3,9,10,13, 15-hexahydro-1H,12H- benzo[de]pyrano[3',4':6,7]indolizino [1,2-b]quinoline-1-yl]amino}-2-oxoethoxy)methyl]glycine amide and can be produced based on the disclosure of International Publication No. WO2015/115091. The anti-HER2 antibody-drug conjugate used in the present invention can be produced by having the above-described drug-linker intermediate react with an anti-HER2 antibody having a thiol group (alternatively referred to as sulfhydryl group).

The anti-HER2 antibody having a sulfhydryl group can be obtained by a method well known to those skilled in the

art. For example, by using 0.3 to 3 molar equivalents of a reducing agent, such as tris(2-carboxyethyl)phosphine hydrochloride (TCEP), per interchain disulfide within the antibody and reacting with the anti-HER2 antibody in a buffer solution containing a chelating agent such as ethylenediaminetetraacetic acid (EDTA), an anti-HER2 antibody having a sulfhydryl group with partially or completely reduced interchain disulfides within the antibody can be obtained.

In addition, by using 2 to 20 molar equivalents of the drug-linker intermediate per anti-HER2 antibody having a sulfhydryl group, an anti-HER2 antibody-drug conjugate in which 2 to 8 drug molecules are conjugated per antibody molecule can be produced.

The average number of conjugated drug molecules per antibody molecule of the anti-HER2 antibody-drug conjugate produced can be determined, for example, by a method of calculation based on measurement of UV absorbance for the anti-HER2 antibody-drug conjugate and the conjugation precursor thereof at two wavelengths of 280nm and 370nm (UV method), or a method of calculation based on quantification through HPLC measurement for fragments obtained by treating the antibody-drug conjugate with a reducing agent (HPLC method).

Conjugation between the anti-HER2 antibody and the drug-linker intermediate, and calculation of the average number of conjugated drug molecules per antibody molecule of the anti-HER2 antibody-drug conjugate can be performed according to the disclosure of International Publication No. WO2015/115091.

V. Uses of the anti-HER2 antibody-drug conjugate.

The antibody-drug conjugate of the present invention can retard cancer cell growth, suppress its proliferation, and further disrupt cancer cells. These actions can accomplish relief from a symptom caused by cancer, improvement of quality of life (QOL) in a cancer patient, and preserves the life of a cancer patient, thus achieving a therapeutic effect. Even in failing to lead to disruption of cancer cells, suppression or control of cancer cell proliferation can result in achieving longer-term survival as well as accomplishing higher QOL in a cancer patient.

The antibody-drug conjugate of the present invention can be expected to exert a therapeutic effect by application as systemic therapy to patients, and additionally, by local application to cancer tissues.

The antibody-drug conjugate may be administered as a pharmaceutical composition with one or more pharmaceutically compatible components contained. The pharmaceutically compatible component can be appropriately selected from formulation additives or the like that are generally used in the art, in view of the dosage, administration concentration or the like of the anti-HER2 antibody-drug conjugate. For example, the anti-HER2 antibody-drug conjugate used in the present invention may be administered as a pharmaceutical composition containing a buffering agent such as a histidine buffering agent, an excipient such as sucrose, and a surfactant such as polysorbate 80. The pharmaceutical composition containing the anti-HER2 antibody-drug conjugate used in the present invention can be used as an injection, as an aqueous injection or a lyophilized injection, or even as a lyophilized injection.

If the pharmaceutical composition containing the anti-HER2 antibody-drug conjugate used in the present invention is an aqueous injection, it can be diluted with a suitable diluent and then given as an intravenous infusion. Examples of the diluent can include dextrose solution and physiological saline.

If the pharmaceutical composition containing the anti-HER2 antibody-drug conjugate used in the present invention is a lyophilized injection, it can be dissolved with injection-grade water, then diluted for a requisite amount with a suitable diluent and then given as an intravenous infusion. Examples of the diluent include dextrose solution and physiological saline.

Examples of the administration route possibly used for administering the pharmaceutical composition of present invention can include intravenous, intradermal, subcutaneous, intramuscular, and intraperitoneal routes.

The anti-HER2 antibody-drug conjugate used in the present invention can be administered to a human with intervals of 1 to 180 days, can be administered with intervals of several weeks, and can preferably be administered with intervals of three weeks. The anti-HER2 antibody-drug conjugate used in the present invention can be administered in a dose of about 0.001 to 100 mg/kg per administration, and can be preferably administered in a dose of 0.8 to 12.4 mg/kg per administration. The anti-HER2 antibody-drug conjugate can preferably be administered once every three weeks at a dose of 0.8 mg/kg to 8 mg/kg, and can more preferably be administered at 5.4 mg/kg to 6.4 mg/kg per dose once every three weeks.

**VI. Method of predicting a patient response to an anti-HER2 antibody-drug conjugate.**

FIG. 14 is a flowchart of steps 1-6 of a method 7 by which an analysis system analyzes a digital image of tissue from a cancer patient and predicts how the cancer patient will likely respond to a therapy involving an anti-HER2 antibody-drug conjugate (ADC). In one embodiment, the method predicts the response to an ADC of a patient having a cancer selected from the group consisting of breast cancer, gastric cancer, colorectal cancer, lung cancer, esophageal cancer, head-and-neck cancer, esophagogastric junction cancer, biliary tract cancer, Paget's disease, pancreatic cancer, ovarian cancer, uterine cancer sarcoma, bladder cancer, prostate cancer, urothelial cancer, gastrointestinal stromal tumor, uterine

cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, sarcoma, osteosarcoma, and melanoma. In one embodiment, the method predicts the response to an ADC of patient having a cancer selected from the group consisting of breast cancer, gastric cancer, colorectal cancer, non-small cell lung cancer, esophageal cancer, head-and-neck cancer, esophagogastric junction adenocarcinoma, biliary tract cancer, Paget's disease, pancreatic cancer, ovarian cancer, uterine carcinosarcoma, bladder cancer, and prostate cancer. In one embodiment, the method predicts the response to an ADC of a patient with breast cancer. In another embodiment, the method predicts the ADC response of a patient with gastric cancer. In yet another embodiment, the method predicts the ADC response of a patient with lung cancer.

In a first step 1, a high-resolution digital image is acquired of a tissue slice from the cancer patient that has been stained using one or more biomarkers or stains.

To predict the efficacy of the ADC therapy, a diagnostic biomarker with an attached dye is used that targets the same protein as that targeted by the ADC therapy. In one embodiment, the anti-HER2 ADC comprises an anti-HER2 antibody conjugated to a drug-linker via a thioether bond, wherein the drug-linker is represented by the following formula:

[Formula 1]

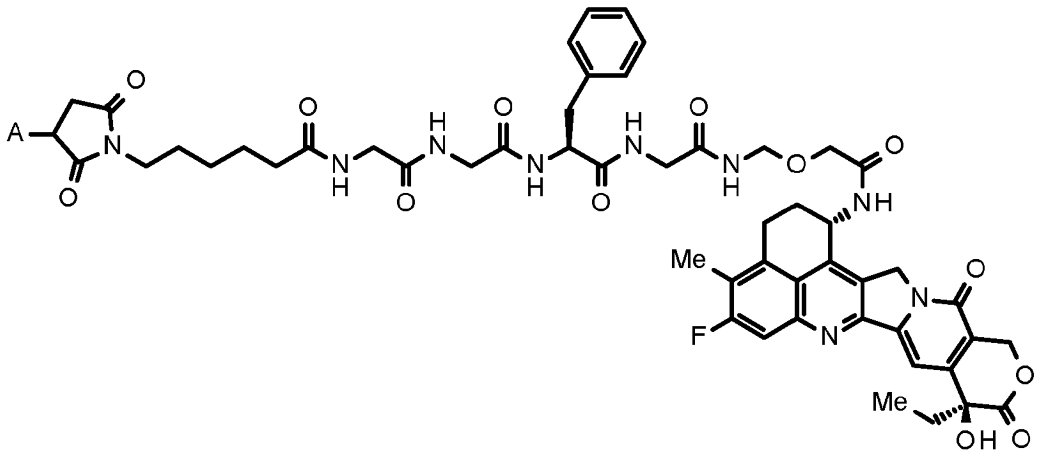

,

wherein A represents the connecting position to the anti-HER2 antibody. In some embodiments, the anti-HER2 antibody is an antibody with a heavy chain comprising CDRH1 consisting of an amino acid sequence represented by SEQ ID NO: 4, CDRH2 consisting of an amino acid sequence represented by SEQ ID NO: 5 and CDRH3 consisting of an amino acid sequence represented by SEQ ID NO: 6, and a light chain comprising CDRL1 consisting of an amino acid sequence represented by SEQ ID NO: 7, CDRL2 consisting of an amino acid sequence consisting of amino acid residues 1 to 3 of SEQ ID NO: 8 and CDRL3 consisting of an amino acid sequence represented by SEQ ID NO: 9.

In some further embodiments, the anti-HER2 antibody is an antibody comprising a heavy chain variable region consisting of the amino acid sequence represented by SEQ ID NO: 10, and light chain variable region consisting of the amino acid sequence represented by SEQ ID NO: 11. In some further embodiments, the anti-HER2 antibody is an antibody comprising a heavy chain consisting of the amino acid sequence represented by SEQ ID NO: 12, and a light chain consisting of the amino acid sequence represented by SEQ ID NO: 3. In certain embodiments, the anti-HER2 antibody is an antibody comprising a heavy chain consisting of the amino acid sequence represented by SEQ ID NO: 2, and a light chain consisting of the amino acid sequence represented by SEQ ID NO: 3. In one embodiment, the anti-HER2 ADC to which the scoring is directed is trastuzumab deruxtecan, as represented by Formula 3 above. Thus, in embodiments, the diagnostic biomarker also targets the HER2 protein.

In step 2, a pretrained convolutional neural network processes a digital image of tissue of the cancer patient that has been stained with the diagnostic antibody linked to the dye, such as 3,3'-Diaminobenzidine (DAB). The staining intensity of the dye in the membrane of a cancer cell is determined based on the mean staining intensity of the dye of all pixels associated with the corresponding segmented membrane object. Moreover, the staining intensity of the dye in a single pixel is computed based on the red, green and blue color components of the pixel. The result of the image analysis processing is two posterior image layers representing, for each pixel in the digital image, the probability that the pixel belongs to a cell nucleus and the probability that the pixel belongs to a cell membrane.

**In** another embodiment of step 2, two pretrained convolutional networks process the digital image of tissue. The result of the processing by the first network is a posterior image layer representing, for each pixel in the digital image, the

likelihood that the pixel belongs to a cell nucleus. The result of the processing by the second network is a posterior image layer representing, for each pixel in the digital image, the likelihood that the pixel belongs to a cell membrane.

In step 3, individual cancer cells are detected based on a heuristic image analysis of the posterior layers for nuclei and membranes. Cancer cell objects are generated that include cell membrane objects and optionally also cell cytoplasm objects.

**In** step 4, a single-cell ADC score is determined for each cancer cell. The single-cell ADC score is based on (1) the amount of DAB in the cell membrane, and a surrogate measurement for (2) the amount of ADC payload uptake. The amount of DAB is determined by the staining intensity of each membrane based on the average optical density of the brown diaminobenzidine (DAB) signal in the pixels of the membrane. The amount of ADC payload uptake is estimated based on the amount of DAB in the cell membrane and optionally also in the cell cytoplasm, and further optionally also on the amount of DAB in the membranes and cytoplasm of neighboring cells to the cell for which the score is determined. The amount DAB in the cell cytoplasm is determined by the staining intensity of each cytoplasm, which is computed based on the average optical density of the brown DAB signal in pixels of the cytoplasm. The amount of DAB in neighboring cells is determined for those cancer cells within a predefined distance of the cell for which the score is being determined.

**In** step 5, a patient score QCS is computed for the digital image of tissue based on a statistical operation on the single-cell ADC score of all cancer cells in the digital image. The patient score is indicative of how the cancer patient will respond to a therapy involving an anti-HER2 ADC. The parameters of the single-cell ADC score in step 4 and the type of statistical operation in step 5 are optimized using a training cohort of patients with known responses to the ADC therapy. Optimization goals are low p-values in a Kaplan Meier analysis of the groups of score-positive versus score-negative patients in the training cohort.

In step 6, the therapy involving the anti-HER2 ADC is recommended to score-positive patients if the score is larger than a predetermined threshold.

In some embodiments, a patient is QCS Positive if at least 90% of tumor cells have a membrane optical density of 5 or greater. In some embodiments, a patient is QCS Positive if at least 90% of tumor cells have a membrane optical density of 6 or greater. In some embodiments, a patient is QCS Positive if at least 90% of tumor cells have a membrane optical density of 7 or greater. In some embodiments, a patient is QCS Positive if at least 90% of tumor cells have a membrane optical density of 8 or greater. In some embodiments, a patient is QCS Positive if at least 90% of tumor cells have a membrane optical density of 8.4 or greater. In some embodiments, a patient is QCS Positive if at least 90% of tumor cells have a membrane optical density of 9 or greater. In some embodiments, a patient is QCS Positive if at least 90% of tumor cells have a membrane optical density of 10 or greater. In some embodiments, a patient is QCS Positive if at least 90% of tumor cells have a membrane optical density of 15 or greater. In some embodiments, a patient is QCS Positive if at least 90% of tumor cells have a membrane optical density of 20 or greater. In some embodiments a patient is QCS Positive if at least 90% of tumor cells have a membrane optical density of 25 or greater.

In some embodiments, a patient is QCS Positive if at least 50% of tumor cells have a membrane optical density of 5 or greater, 8 or greater, or 25 or greater. In some embodiments, a patient is QCS Positive if at least 50% of tumor cells have a membrane optical density of 8 or greater. In some embodiments, a patient is QCS Positive if at least 60% of tumor cells have a membrane optical density of 5 or greater, 8 or greater, or 25 or greater. In some embodiments, a patient is QCS Positive if at least 60% of tumor cells have a membrane optical density of 8 or greater. In some embodiments, a patient is QCS Positive if at least 70% of tumor cells have a membrane optical density of 5 or greater, 8 or greater, or 25 or greater. In some embodiments, a patient is QCS Positive if at least 70% of tumor cells have a membrane optical density of 8 or greater. In some embodiments, a patient is QCS Positive if at least 80% of tumor cells have a membrane optical density of 5 or greater, 8 or greater, or 25 or greater. In some embodiments, a patient is QCS Positive if at least 80% of tumor cells have a membrane optical density of 8 or greater. In some embodiments, a patient is QCS Positive if at least 90% of tumor cells have a membrane optical density of 5 or greater, 8 or greater, or 25 or greater. In some embodiments, a patient is QCS Positive if at least 90% of tumor cells have a membrane optical density of 8 or greater. In some embodiments, a patient is QCS Positive if at least 95% of tumor cells have a membrane optical density of 5 or greater, 8 or greater, or 25 or greater. In some embodiments, a patient is QCS Positive if at least 95% of tumor cells have a membrane optical density of 8 or greater.

In some embodiments, a patient is QCS Positive if the density of tumor cells with a membrane optical density of at least 8 within the tumor area is 500 cells/$mm^2$ or greater. In some embodiments, a patient is QCS Positive if the density of tumor cells with a membrane optical density of at least 8 within the tumor area is 1000 cells/$mm^2$ or greater. In some embodiments, a patient is QCS Positive if the density of tumor cells with a membrane optical density of at least 8 within the tumor area is 1250 cells/$mm^2$ or greater. In some embodiments, a patient is QCS Positive if the density of tumor cells with a membrane optical density of at least 8 within the tumor area is 1500 cells/$mm^2$ or greater. In some embodiments, a patient is QCS Positive if the density of tumor cells with a membrane optical density of at least 8 within the tumor area is 1600 cells/$mm^2$ or greater. In some embodiments, a patient is QCS Positive if the density of tumor cells with a membrane optical density of at least 8 within the tumor area is 1670 cells/$mm^2$ or greater. In some embodiments, a patient is QCS Positive if the density of tumor cells with a membrane optical density of at least 8 within the tumor area is 1700 cells/$mm^2$ or greater. In some embodiments, a patient is QCS Positive if the density of tumor cells with a membrane optical density of at least 8

within the tumor area is 1800 cells/$mm^2$ or greater. In some embodiments, a patient is QCS Positive if the density of tumor cells with a membrane optical density of at least 8 within the tumor area is 1900 cells/$mm^2$ or greater. In some embodiments, a patient is QCS Positive if the density of tumor cells with a membrane optical density of at least 8 within the tumor area is 2000 cells/$mm^2$ or greater. In some embodiments, a patient is QCS Positive if the density of tumor cells with a membrane optical density of at least 8 within the tumor area is 3000 cells/$mm^2$ or greater.

In some embodiments, a patient is QCS Positive if the density of tumor cells with a membrane optical density of at least 20 within the tumor area is 500 cells/$mm^2$ or greater. In some embodiments, a patient is QCS Positive if the density of tumor cells with a membrane optical density of at least 20 within the tumor area is 1000 cells/$mm^2$ or greater. In some embodiments, a patient is QCS Positive if the density of tumor cells with a membrane optical density of at least 20 within the tumor area is 1500 cells/$mm^2$ or greater. In some embodiments, a patient is QCS Positive if the density of tumor cells with a membrane optical density of at least 20 within the tumor area is 2000 cells/$mm^2$ or greater. In some embodiments, a patient is QCS Positive if the density of tumor cells with a membrane optical density of at least 20 within the tumor area is 2500 cells/$mm^2$ or greater. In some embodiments, a patient is QCS Positive if the density of tumor cells with a membrane optical density of at least 20 within the tumor area is 2750 cells/$mm^2$ or greater. In some embodiments, a patient is QCS Positive if the density of tumor cells with a membrane optical density of at least 20 within the tumor area is 3000 cells/$mm^2$ or greater. In some embodiments, a patient is QCS Positive if the density of tumor cells with a membrane optical density of at least 20 within the tumor area is 3250 cells/$mm^2$ or greater. In some embodiments, a patient is QCS Positive if the density of tumor cells with a membrane optical density of at least 20 within the tumor area is 3500 cells/$mm^2$ or greater. In some embodiments, a patient is QCS Positive if the density of tumor cells with a membrane optical density of at least 20 within the tumor area is 4000 cells/$mm^2$ or greater. In some embodiments, a patient is QCS Positive if the density of tumor cells with a membrane optical density of at least 20 within the tumor area is 5000 cells/$mm^2$ or greater.

In some embodiments, a patient is QCS Positive if the binary spatial proximity score is 90 or greater. In some embodiments, a patient is QCS Positive if the binary spatial proximity score is 91 or greater. In some embodiments, a patient is QCS Positive if the binary spatial proximity score is 92 or greater. In some embodiments, a patient is QCS Positive if the binary spatial proximity score is 93 or greater. In some embodiments, a patient is QCS Positive if the binary spatial proximity score is 94 or greater. In some embodiments, a patient is QCS Positive if the binary spatial proximity score is 95 or greater. In some embodiments, a patient is QCS Positive if the binary spatial proximity score is 96 or greater. In some embodiments, a patient is QCS Positive if the binary spatial proximity score is 97 or greater. In some embodiments, a patient is QCS Positive if the binary spatial proximity score is 98 or greater. In some embodiments, a patient is QCS Positive if the binary spatial proximity score is 99 or greater. In some embodiments, a patient is QCS Positive if the binary spatial proximity score is 99.5 or greater. In some embodiments, a patient is QCS Positive if the binary spatial proximity score is 99.8 or greater. In the above embodiments, the binary spatial proximity score is calculated as follows: spatial proximity score = ([number of tumor cells with OD>8] + [number of tumor cells with OD<=8 which are in the 50$\mu$m neighborhood of at least one tumor cell with OD>8])/[number of all tumor cells].

In some embodiments, a patient is QCS Positive if the continuous spatial proximity score is 20 or greater. In some embodiments, a patient is QCS Positive if the continuous spatial proximity score is 30 or greater. In some embodiments, a patient is QCS Positive if the continuous spatial proximity score is 40 or greater. In some embodiments, a patient is QCS Positive if the continuous spatial proximity score is 50 or greater. In some embodiments, a patient is QCS Positive if the continuous spatial proximity score is 60 or greater. In some embodiments, a patient is QCS Positive if the continuous spatial proximity score is 70 or greater. In some embodiments, a patient is QCS Positive if the continuous spatial proximity score is 80 or greater. In some embodiments, a patient is QCS Positive if the continuous spatial proximity score is 90 or greater. In the above embodiments, the continuous spatial proximity score is 10% quantile of the cumulative optical density of each tumor cell and any neighboring tumor cell within 25 $\mu$m, weighted by the distance of the neighbors.

In some embodiments, a patient is QCS Positive if the density of tumor infiltrating lymphocytes in the stroma is 50 cells/$mm^2$ or greater. In some embodiments, a patient is QCS Positive if the density of tumor infiltrating lymphocytes in the stroma is 100 cells/$mm^2$ or greater. In some embodiments, a patient is QCS Positive if the density of tumor infiltrating lymphocytes in the stroma is 125 cells/$mm^2$ or greater. In some embodiments, a patient is QCS Positive if the density of tumor infiltrating lymphocytes in the stroma is 150 cells/$mm^2$ or greater. In some embodiments, a patient is QCS Positive if the density of tumor infiltrating lymphocytes in the stroma is 160 cells/$mm^2$ or greater. In some embodiments, a patient is QCS Positive if the density of tumor infiltrating lymphocytes in the stroma is 165 cells/$mm^2$ or greater. In some embodiments, a patient is QCS Positive if the density of tumor infiltrating lymphocytes in the stroma is 168 cells/$mm^2$ or greater. In some embodiments, a patient is QCS Positive if the density of tumor infiltrating lymphocytes in the stroma is 170 cells/$mm^2$ or greater. In some embodiments, a patient is QCS Positive if the density of tumor infiltrating lymphocytes in the stroma is 175 cells/$mm^2$ or greater. In some embodiments, a patient is QCS Positive if the density of tumor infiltrating lymphocytes in the stroma is 180 cells/$mm^2$ or greater. In some embodiments, a patient is QCS Positive if the density of tumor infiltrating lymphocytes in the stroma is 190 cells/$mm^2$ or greater. In some embodiments, a patient is QCS Positive if the density of tumor infiltrating lymphocytes in the stroma is 200 cells/$mm^2$ or greater.

In some embodiments, a patient is QCS Positive if the density of tumor infiltrating lymphocytes in the stroma is

300 cells/$mm^2$ or greater. In some embodiments, a patient is QCS Positive if the density of tumor infiltrating lymphocytes in the stroma is 400 cells/$mm^2$ or greater. In some embodiments, a patient is QCS Positive if the density of tumor infiltrating lymphocytes in the stroma is 500 cells/$mm^2$ or greater. In some embodiments, a patient is QCS Positive if the density of tumor infiltrating lymphocytes in the stroma is 600 cells/$mm^2$ or greater. In some embodiments, a patient is QCS Positive if the density of tumor infiltrating lymphocytes in the stroma is 700 cells/$mm^2$ or greater. In some embodiments, a patient is QCS Positive if the density of tumor infiltrating lymphocytes in the stroma is 735 cells/$mm^2$ or greater. In some embodiments, a patient is QCS Positive if the density of tumor infiltrating lymphocytes in the stroma is 750 cells/$mm^2$ or greater.

In some embodiments, a patient is QCS Positive if the density of tumor infiltrating lymphocytes in the stroma is 800 cells/$mm^2$ or greater. In some embodiments, a patient is QCS Positive if the density of tumor infiltrating lymphocytes in the stroma is 900 cells/$mm^2$ or greater. In some embodiments, a patient is QCS Positive if the density of tumor infiltrating lymphocytes in the stroma is 1000 cells/$mm^2$ or greater. In some embodiments, a patient is QCS Positive if the density of tumor infiltrating lymphocytes in the stroma is 2000 cells/$mm^2$ or greater. In some embodiments, a patient is QCS Positive if the density of tumor infiltrating lymphocytes in the stroma is 3000 cells/$mm^2$ or greater. In some embodiments, a patient is QCS Positive if the density of tumor infiltrating lymphocytes in the stroma is 4000 cells/$mm^2$ or greater. In some embodiments, a patient is QCS Positive if the density of tumor infiltrating lymphocytes in the stroma is 5000 cells/$mm^2$ or greater.

In some embodiments, a patient is QCS Positive if a) at least 90% of tumor cells have a membrane optical density of 8.4 or greater and b) if the density of tumor cells with a membrane optical density of at least 20 within the tumor area is 3000 cells/$mm^2$ or greater. In some embodiments, a patient is score positive if a) at least 90% of tumor cells have a membrane optical density of 8.4 or greater and b) if the binary spatial proximity score is 99.8 or greater. In some embodiments, a patient is score positive if a) at least 90% of tumor cells have a membrane optical density of 8.4 or greater and b) if the continuous spatial proximity score is 50 or greater. In some embodiments, a patient is score positive if a) at least 90% of tumor cells have a membrane optical density of 8.4 or greater and b) if the density of tumor infiltrating lymphocytes in the stroma is 735 cells/$mm^2$ or greater.

In some embodiments, a patient is QCS Positive if a) at least 90% of tumor cells have a membrane optical density of 8.4 or greater; b) if the density of tumor cells with a membrane optical density of at least 20 within the tumor area is 3000 cells/$mm^2$ or greater; and c) if the binary spatial proximity score is 99.8 or greater or if the continuous spatial proximity score is 50 or greater. In some embodiments, a patient is QCS Positive if a) at least 90% of tumor cells have a membrane optical density of 8.4 or greater; b) if the density of tumor cells with a membrane optical density of at least 20 within the tumor area is 3000 cells/$mm^2$ or greater; c) if the binary spatial proximity score is 99.8 or greater; and d) if the continuous spatial proximity score is 50 or greater. In some embodiments, a patient is QCS Positive if a) at least 90% of tumor cells have a membrane optical density of 8.4 or greater; b) if the density of tumor cells with a membrane optical density of at least 20 within the tumor area is 3000 cells/$mm^2$ or greater; c) if the binary spatial proximity score is 99.8 or greater or if the continuous spatial proximity score is 50 or greater; and d) if the density of tumor infiltrating lymphocytes in the stroma is 735 cells/$mm^2$ or greater. In some embodiments, a patient is QCS Positive if a) at least 90% of tumor cells have a membrane optical density of 8.4 or greater; b) if the density of tumor cells with a membrane optical density of at least 20 within the tumor area is 3000 cells/$mm^2$ or greater; c) if the binary spatial proximity score is 99.8 or greater; d) if the continuous spatial proximity score is 50 or greater; and e) if the density of tumor infiltrating lymphocytes in the stroma is 735 cells/$mm^2$ or greater.

## VII. Examples of prediction and scoring method.

### A. Image analysis of stained tissue.

The method of FIG. 14 is now described in relation to a particular image of stained cancer tissue.

In step 1, a tissue sample is immunohistochemically stained using a dye linked to a diagnostic antibody that binds to the associated protein on the cancer cells in the tissue sample. FIG. 15 (upper-left image) is a digital image 17 of a portion of stained tissue that was acquired in step 1. Image 17 shows tissue from a cancer patient that has been immunohistochemically stained with an anti-Her2 diagnostic antibody linked to a dye. In this example, the diagnostic antibody is the Ventana PATHWAY anti-HER-2/neu (4B5) Rabbit Monoclonal Primary Antibody, which targets the protein HER2. In other examples, the diagnostic antibody may be the Dako HercepTest primary antibody, which is a rabbit anti-human antibody which also targets the protein HER2. The anti-Her2/neu antibody binds to the membrane protein Her2/neu so that the 3,3'-Diaminobenzidine (DAB) stain indicates the location of the protein Her2/neu in the tissue sample.

In step 2, image analysis is performed on the digital image 17 to generate posterior image layers of cancer cell nuclei and membranes using a convolutional neural network. The image analysis is used to detect the cancer cells and their components, such as the nuclei, the membrane and the cytoplasm. FIG. 15 illustrates the image analysis process of step 2. The convolutional neural network generates posterior layers (gray value images) that indicate, for each pixel of digital image 17, the probability that each pixel belongs to either the nucleus (FIG. 15 upper-right image) or the membrane

(FIG. 15 lower-left image) of the cell. High probabilities are shown in black, low probabilities in white.

FIG. 16 illustrates another embodiment of how to perform the image analysis of step 2. The convolutional neural network generates regression layers (gray value images) that indicate, for each pixel, the distance to the nucleus (FIG. 16 upper-right image) or the distance to the membrane (FIG. 16 lower-left image). Large distances are shown in white, while small distances are shown in black. A comparison of FIGS. 15 and 16 shows that the image analysis of the embodiment of FIG. 16 generates thicker membrane objects but smaller nucleus objects than does the embodiment of FIG. 15.

**In** one embodiment, the convolutional neural network includes a series of convolution layers from the input image 17 towards a bottleneck layer with very low spatial size (1 to 16 pixels), and a series of deconvolution layers towards the posterior layers that have the same size as the input image 17. This network architecture is called a U-Net. The training of the weights of the convolutional neural networks is performed by generating manual annotation layers for nuclei and membranes in multiple training images, and then adjusting by an optimization algorithm the network weights so that the generated posterior layers are most similar to the manually generated annotation layers.

**In** another embodiment, the annotation layers for nuclei and membranes are generated automatically and corrected manually in multiple training images. Epithelium regions and nuclei centers are manually annotated as regions and points, respectively. For each training image, the membrane segmentation is automatically generated by applying a region growing-like algorithm (e.g., watershed segmentation) seeded by the annotated nuclei centers and constrained by the extent of the annotated epithelium region. Given a training image, the nuclei segmentation is automatically generated by applying a blob detection algorithm (e.g., by the maximally-stable-extremal-regions MSER algorithm) and by selecting as nuclei only the detected blobs that contain an annotated nucleus center. The automatically generated membrane and nuclei segmentations are visually reviewed and manually corrected if necessary. The correction steps involve one of the following methods: rejecting incorrectly segmented membranes or nuclei, explicitly accepting correctly annotated membranes or nuclei, or refining the shapes of the membranes or nuclei. For each image with annotated membranes or nuclei, an annotation layer is created. In one embodiment, each pixel of the annotation layer is assigned a "1" if it belongs to the annotated object (membrane or nucleus); otherwise it is assigned a "0". In another embodiment, the pixels of the annotation layer represent the distance to the nearest annotated object. The network weights are adjusted by an optimization algorithm so that the generated posterior layers are most similar to the automatically generated membrane and nuclei annotation layers.

FIG. 17 illustrates step 3 in which individual cancer cell objects are detected that each include a cell membrane and a cell cytoplasm. A heuristic image analysis process uses watershed segmentation to segment the cell nuclei using the nucleus posterior layer generated by the convolutional neural network. The segmentation generates nucleus objects. Each nucleus object is assigned a unique identifier (UID). The individually identified nuclei are shown as dark objects in FIG. 17 (lower-right image). The detected nuclei are also displayed as overlays in the input image 17 (upper-left image) and in the posterior layers for nuclei (upper-right image) and membranes (lower-left image).

In one embodiment, the watershed segmentation involves a thresholding of the nucleus posterior layer with a predefined first size threshold. All single connected pixels that are above a first size threshold are considered to belong to a nucleus object. Nucleus objects with an area smaller than 16 um^2 are discarded. A UID is assigned to each nucleus object. In a subsequent step, the nucleus objects are grown towards smaller nucleus posteriors in which the added nucleus posterior pixels must be greater than a second predefined threshold.

FIG. 18 illustrates further segmentation steps in which nucleus objects are used to detect and improve the segmentation of the membranes. A region grow algorithm uses the detected nucleus objects as seeds to grow to the ridge of the membrane (approximate cell border) in the membrane posterior layer. Detected membranes (lower-right image) are shown as overlays in the input image 17 (upper-left image) and in the posterior layers for nuclei (upper-right image) and membranes (lower-left image).

FIG. 19 illustrates the detection and segmentation of membrane objects which are segmented by growing the region of the border pixels of detected cells outwards to the membrane probability layer and to a predefined membrane layer posterior threshold value. The thicker border regions become the membrane objects. Each membrane object is assigned the same UID as that of the associated the nucleus object.

The space between the membrane and the nucleus is assigned to the cytoplasm using the UID of the nucleus. For each membrane (FIG. 19 see top-left image) and cytoplasm (FIG. 19 see top-left image), the average optical densities of the DAB staining is exported to a file on a hard drive together with the UIDs. For each cell (defined as in including a nucleus, cytoplasm and membrane), the position of the center of gravity (x,y) of the cell within the slide is also exported. The file may reside on a hard disk, a solid state disk or a portion of dedicated RAM in a computer system.

FIG. 20 illustrates the results of the image analysis in an image analysis software environment. FIG. 20 (upper-left image) shows the segmentation of nucleus objects and membrane objects as an overlay on the digital image 17. FIG. 20 (lower-left image) shows the segmentation of a nucleus object as an overlay on an optical density representation of image 17. Dark optical density pixels are associated with a high amount of DAB, and bright optical density pixels are associated with a low amount of DAB. The DAB optical density of each image pixel is computed from the red-green-blue representation of the image pixel by transformation of the red-green-blue color space so that the brown DAB component

becomes an independent color, and by taking the logarithm of that brown color component. In the event that immunofluorescence (IF) imaging is used to determine the staining, then the HER2 channel would be acquired as a 12-bit, 16-bit or 32-bit gray scale image as opposed to using the red-green-blue color space. In IF, the nucleus is marked using DAPI (2-[4-(Aminoiminomethyl)phenyl]-1H-Indole-6-carboximidamide hydrochloride) as a first dye. The posterior layer of the nucleus is generated using the image of the first dye as input for a convolutional neural network. A second dye is linked to the diagnostic anti-HER2 antibody. The strength of the fluorescence signal from the second dye corresponds to the optical density (OD) of DAB. FIG. 20 (upper-right image) and FIG. 21 (top) show the image analysis script used to generate the segmented image within a Definiens Developer XD platform. FIG. 20 (lower-right image) and FIG. 21 (bottom) shows the exported measurements for all cell membrane objects and cytoplasm objects in image 17. In another embodiment, the image analysis script is encoded using another programming language, such as C++, C#, Java, Python or R.

**B. Calculation of predictive ADC score.**

Based on the optical density of the DAB staining within the membrane objects and the cytoplasm objects, a single-cell ADC score is computed for each cancer cell in the digital image 17. The single-cell ADC score is also based on the staining intensities of the DAB dye in the membrane objects and cytoplasm objects of neighboring cancer cells that are closer than a predefined distance to the cancer cell for which the single-cell ADC score is being computed. The score is predictive of a response of the cancer patient to an anti-HER2 ADC therapy.

FIG. 22 illustrates exemplary quantitative results of the optical density of staining from the image analysis of steps 2-3 in a schematic drawing using gray values of membrane and cytoplasm pixels. The steps of heuristic image analysis illustrated in FIGS. 15-20 are used to obtain the example segmentation of FIG. 22 into cell nuclei, cell membranes and cell cytoplasm. Bright gray values in FIG. 22 are associated with high DAB optical density, and therefore with a high amount of proteins targeted by the diagnostic antibody. Dark gray values are associated with a low DAB optical density. Brighter pixels correspond to a higher DAB optical density.

FIG. 23 lists the exemplary quantitative amounts of staining on the membranes and in the cytoplasms of the image of FIG. 22, which is reproduced in part in FIG. 23. The optical density of the brown DAB signal from the membranes of the first, second and third cells is 0.949, 0.369 and 0.498, respectively. The optical density of the brown DAB signal from the cytoplasms of the first, second and third cells is 0.796, 0.533 and 0.369, respectively. In the schematic image of FIG. 23, the first cancer cell 18 expresses a high amount of the target protein HER2 and would be very likely to be killed by the ADC payload entering the cell linked to the ADC antibody (effect 1 in FIG. 24). The second cancer cell 19 and third cancer cell 20 do not express sufficient amounts of the target protein HER2 to be killed directly by the anti-HER2 ADC. However, due to the vicinity of the second cancer cell 19 to the first cancer cell 18, the toxic payload released from the first cancer cell would also kill the second cancer cell 19 (effect 3 in FIG. 24). The third cancer cell 20 would remain active and could be the origin of a drug resistance mechanism, which could eventually cause the death of the patient.

FIG. 24 illustrates the mechanism by which an anti-HER2 ADC therapy kills cancer cells. For example, trastuzumab deruxtecan also uses this mechanism. In a first step, the ADC antibody (e.g., trastuzumab) binds to the target protein HER2 and inhibits the natural function of the target protein, which may lead to cell death. In a second step, the payload (e.g., a type I topoisomerase inhibitor) is internalized into the cell and kills the cell by the toxicity of the payload. This uptake of the payload depends on the amount of target protein on the membrane, and on the difference in the amount of target protein on the membrane and in the cytoplasm. After uptake, the payload can be released from the cell into the surrounding tissue. In a third step, the payload may enter nearby cells and may kill them as well. The spatial distribution of the payload in the tissue is spread by passive diffusion.

Traditional HER2 scoring reflects both the effect of inhibition of the target protein HER2 due to ADC binding, as well as the effect of the cytotoxic payload entering a cancer cell together with the ADC antibody. Thus, the traditional scoring for trastuzumab therapies does not reflect the importance of the presence of the HER2 protein in the cytoplasm and the effect of the cytotoxic payload that diffuses into the tissue after being released from the first killed cancer cell. In comparison, the novel predictive ADC Score measures the effect of the release of the cytotoxic payload on neighboring cancer cells.

In step 4 of the method of FIG. 14, a single-cell ADC score is determined for each cancer cell. FIG. 25 illustrates the calculation of the single-cell ADC score for each of the three cells shown in FIG. 23 and incorporates an exponential weighting factor based on cell separation to account for the uptake of the ADC payload into neighboring cells. The single-cell score can be calculated based on the formula shown in FIG. 26 or the formula recited in claim 4. The optical densities listed in FIG. 23 for the DAB signal from the cell membranes (0.949, 0.369 and 0.498) and from the cytoplasms (0.796, 0.533 and 0.369) are inputs into the calculation illustrated in FIG. 25. The first, second and third cells 18-20 have single-cell scores of 0.145, 0.012 and 0.064, respectively.

Thus, the single-cell ADC score incorporates the measurement of the amount of target protein on the cell membrane using the DAB optical density and an estimation of the amount of ADC payload uptake. As shown in FIG. 24, the uptake of the ADC payload for a first cell depends on both the amount of dye in its membrane and in its cytoplasm, as well as

on the amount of dye in the membrane and the cytoplasm of a second cell in the vicinity of the first cell. More specifically, the vicinity may be a circular disk with a predefined radius around the first cancer cell. In one embodiment, the single-cell ADC score for the first cancer cell is determined by a distance-weighted sum of the several powers of DAB optical densities of membrane and cytoplasm objects whose associated cancer cell centers are closer to the first cancer cell center than a predefined distance. In one embodiment, the predefined distance is 50um, as used in the calculation of FIG. 25. In another embodiment, the distance weighting involves computing the exponential of the scaled negative Euclidean distance from the first cancer cell center to the other cancer cell centers in the sum. In another embodiment, the powers in the sum are restricted to 0, 1, and 2 (constants, linear terms, squares).

FIG. 26 shows one embodiment of a formula for calculating the single-cell ADC score. The functions $a_{kl}$ in the formula depend on the distance $|r_j - r_i|$ from the cell j to the cell i. $ODM_j$ is the DAB optical density of the membrane of cell j, and $ODC_j$ is the DAB optical density of the cytoplasm of cell j. The constants A_ij , r_norm and d are the same for all types of cancer. However, the threshold for the score to determine whether the patient is eligible for the ADC therapy is not the same for different types of cancer.

In step 5 of the method for indicating how a particular cancer patient will respond to an ADC therapy, a response score is computed for the digital image 17 of tissue from the cancer patient based on the staining of the target protein HER2 such that the score indicates how the cancer patient will respond to the anti-HER2 ADC therapy. The response score is generated by aggregating all single-cell ADC scores of the tissue sample using a statistical operation. Thus, the score is computed based on a statistic of the single-cell ADC scores for all cancer cells detected in the digital image. In one embodiment, the statistic is a predefined quantile of the ADC payload uptake estimates of all cancer cells the image 17.

In step 6 of the method, the anti-HER2 ADC therapy is recommended to the cancer patient if the response score is larger than a predetermined threshold. The predetermined threshold in step 6 and the quantile in step 5 are determined by optimizing the positive predictive value, the negative predictive value, and the prevalence of a positive recommendation using a cohort of patients with known single-cell ADC scores and therapy response parameters.

In another example of the computation of the response score in step 5, a digital image$^s$ of a tissue sample of a cancer patient is acquired and stained with a diagnostic antibody (dAB). Image analysis is performed on the digital image$^s$ to detect N cancer cells $(c_i)_{0\leq<N}$. The diagnostic antibody (dAB) is Ventana PATHWAY anti-HER-2/neu (4B5). The optical density in the segmented membrane of cell $c_i$ is denoted as $OD_M^i$ , and the optical density in the segmented cytoplasm is denoted as $OD_C^i$ . The response score $S^s$ associated with digital image$^s$ indicates the likelihood that the cancer patient will respond to the anti-HER2 ADC therapy. The ADC binds specifically to the same HER2 protein as the diagnostic antibody. The response score $S^s$ can be expressed as:

[Formula 6]

$$S^s = \bigwedge_{0\leq i<N} [\, p(c_i) \,]$$

where $\Lambda\square$ is a aggregation operator over a set of cells detected in the digital image$^s$ . The aggregation operation is a single number representative of the positivity values $p(c_i)$ associated with the detected cells. In one example, the aggregation operation is the arithmetic mean. In another example, the aggregation operation is one of several kinds of averages, such as a harmonic mean or a geometric mean. In another example, the aggregation operation is the frequency of a sub-group of detected cells. In another example, the aggregation operation is a sum. In one embodiment, the aggregation operation is one of several kinds of averages of a subset of cells. In yet another example, the aggregation operation is a quantile.

The term $p(\square)$ denotes a positivity function that represents for each cell its probability of responding to the anti-HER2 ADC therapy. The positivity function for a cell is positive for an aggregation of dAB positivity values associated with the cell's neighboring cells. The positivity value $p(c_i)$ associated with a cell $c_i$ is expressed as:

[Formula 7]

$$p(c_i) = \bigvee_{(c_j)_{j\in\pi_i}} [\, p^{dAB}(c_j) \,]$$

where $\pi_i$ is the set of neighboring cancer cells in the proximity of the cell $c_i$, which includes cell $c_i$, defined as the cancer cells whose distance to the cell $c_i$ is smaller than a predefined constant. In one example, the distance between $c_i$ and $c_j$ is the Euclidean distance between the two cell centers. In another example, the distance is the minimal distance between the membranes of $c_i$ and $c_j$. In Formula 7, $p^{dAB}(c_j)$ is a positivity value associated with cell $c_j$ based on its optical density in the membrane and its optical density in its cytoplasm. In one example, $p^{dAB}(c_j)$ is defined as the function that associates 1 to cells for which

[Formula 8]

$$\alpha_1\left(OD_M^j\right)^{\gamma_1} + \alpha_2\left(OD_C^j\right)^{\gamma_2} + \alpha_3\left(h\left(OD_M^j, OD_C^j\right)\right)^{\gamma_3} > T_p$$

and 0 otherwise. In Formula 8, $\alpha_1$, $\alpha_2$, $\alpha_3$, $\gamma_1$, $\gamma_2$, $\gamma_a$ and $T_P$ are predefined constants, and h(□) is a function measuring the difference between $OD_M^j$ and $OD_C^j$. The predefined constants are determined by statistical analysis of a cohort of cancer patients with known treatment response to the anti-HER2 ADC therapy. In another example, $p^{dAB}(c_j)$ is expressed as:

[Formula 9]

$$p^{dAB}(c_j) = \alpha_1\left(OD_M^j\right)^{\gamma_1} + \alpha_2\left(OD_C^j\right)^{\gamma_2} + \alpha_3\left(h\left(OD_M^j, OD_C^j\right)\right)^{\gamma_3}$$

In one example, the function h(□) is equal to the difference between $OD_M^j$ and $OD_C^j$, expressed as $h\left(OD_M^j, OD_C^j\right) = OD_M^j \times \left(OD_M^j - OD_C^j\right)$. In another example, the function h(□) considers only the positive difference between $OD_M^j$ and $OD_C^j$, expressed as $h\left(OD_M^j, OD_C^j\right) = OD_M^j \times \max\left(0, OD_M^j - OD_C^j\right)$. In yet another example, the function h() is equal to the contrast between $OD_C^j$ and $OD_M^j$, expressed as

$$h\left(OD_M^j, OD_C^j\right) = OD_M^j \times \max\left(0, \frac{OD_M^j - OD_C^j}{OD_M^j + OD_C^j}\right).$$

**In** Formula 7, V□ is a aggregation operator over the set of neighboring cancer cells. The aggregation operation is a single number representative of the dAB positivity values associated with the neighboring cells. In one example, the aggregation operation is the weighted arithmetic mean of the dAB positivity, expressed as:

[Formula 10]

$$\bigvee_{\{c_j\}_{j\in\pi_i}} \left[p^{dAB}(c_j)\right] = \frac{\Sigma_{\{c_j\}_{j\in\pi_i}} w_{c_i}(c_j)\left[p^{dAB}(c_j)\right]}{\Sigma_{\{c_j\}_{j\in\pi_i}} w_{c_i}(c_j)}$$

where $w_{c_i}(c_j)$ denotes a weighting function that computes the likelihood of the cell $c_i$ being influenced by the neighboring cell $c_j$. In another example, the aggregation operation associates 1 to a cell $c_i$ if the sum of the dAB positivity values of its neighboring cells is above an arbitrary real constant $T_n$, and otherwise 0. In another example, the aggregation operation is a quantile, a frequency measure, or one of several kinds of averages, such as a harmonic mean or a geometric mean. In one example, the weighting function $w_{c_i}(c_j)$ is a Gaussian function of a spatial distance $d(c_i, c_j)$ between the cell $c_i$ and the

cell $c_j$, expressed as:

[Formula 11]

$$w_{c_i}(c_j) = exp\left[-\frac{d(c_i, c_j)^{\alpha}}{\sigma^{\alpha}}\right]$$

where $\sigma$ and $\alpha$ are predefined constants. In another example, the weighting function is a sigmoid of the spatial distance:

[Formula 12]

$$w_{c_i}(c_j) = 1 - \frac{1}{1 + exp\left[-\frac{(d(c_i, c_j) - \beta)^{\alpha}}{\sigma^{\alpha}}\right]}$$

where $\sigma$, $\alpha$ and $\beta$ are predefined constants. In another example, the weighting function is a decreasing function of the Euclidean distance between the cell $c_i$ and the cell $c_j$. In yet another example, the weighting function is a Heaviside function of the distance between the cell $c_i$ and the cell $c_j$ that associates 1 if the distance is smaller than an predefined constant $T_d$, and otherwise 0.

One particular example of the positivity function $p(c_i)$ associates 1 to the cell $c_i$ for which

[Formula 13]

$$\left(\sum_{(c_j)_{j\in\pi_i}} \left[OD_M^j > T_p\right]\right) > T_n$$

and otherwise 0.

**C. Validation of prediction method based on breast cancer patients.**

The accuracy of the novel predictive ADC score generated according to the method of FIG. 14 was validated based on a patient trial (J101 NCT02564900), which was a Phase I clinical trial with trastuzumab deruxtecan (DS-8201). The dataset of the J101 patient trial includes stained tissue images and therapy response rates for patients with multiple cancer types, including breast cancer and gastric cancer. A first validation of the predictive ADC score was based on data from 151 breast cancer patients with varying HER2 expression levels (1+, 2+, 3+). The response score was trained using pathologists' annotations, and the performance of the scoring method was validated on unseen data to ensure its generalization and robustness. The response score was blindly applied to the J101 data. The optical density OD (level of brown DAB stain intensity) was computed on detected membranes to derive features that were linked to survival prediction. The response score features were selected to maximize the objective response rate (ORR) in a positive group of patients and to minimize the (ORR) in a negative group of patients. The objective response rate is the proportion of patients in the group who exhibit either a partial response or a complete response to a therapy. A partial response is defined as tumor shrinkage between 30% and 100%. A complete response is tumor shrinkage of 100% and the elimination of the tumor.

The analytical validation explained below demonstrates a high correlation of the optical density values measured on membranes detected using image analysis according to the method of FIG. 14 (R=0.993) to that of the optical density measured on consolidated membranes annotated by pathologists (R=0.995). FIG. 27 is a diagram comparing how consistently pathologists annotate membranes compared to one another. The diagram shows the optical density of membrane staining identified by a first pathologist to the optical density identified by a second pathologist. Although the scale of the optical density in the diagram is listed from 0 to 300, the actual optical density is measured in a range from 0 to 220. The optical density is sometimes alternatively indicated in this application in a range from 0 to 1 by dividing the

measured optical density by 220. FIG. 28 is a diagram comparing how the membranes detected using image analysis correlate to the membranes identified by the pathologists. A comparison of the scatter plots of the two diagrams demonstrates that the image analysis of the method of FIG. 14 detects membranes almost as consistently as do the pathologists. Moreover, the response score derived from the image analysis was largely consistent with the pathologists' HER2 IHC test scoring, but also showed a broad quantitative overlap between the IHC and ISH staining categories. Scoring using the method of FIG. 14 showed a direct linear relationship between the objective response rate ORR and an increased HER2 expression across the entire range of assay.

HER2-targeting therapies are currently not approved for "HER2 Low" patients whose cancer is assigned a score of (i) >0 and <1+ for the expression of HER2 from immunohistochemical (IHC) staining, (ii) 1+ for the expression of HER2 from immunohistochemical staining, and (iii) both 2+ for the expression of HER2 from immunohistochemical (ISH) staining and negative (-) for the expression of HER2 from in situ hybridization staining.

FIG. 29 illustrates the low correlation between quality of response and having an HER2 IHC 2+ score as opposed to having an HER2 IHC 1+ score. Breast cancer patients from the J101 trial in the HER2-negative cohort having a score of IHC 2+ did not all have consistently better responses than patients in the cohort having a score of IHC 1+. Patients represented by longer bars to the right in the diagram exhibited a larger reduction in tumor size than did patients to the left. Patients to the right whose tumor size shrunk by more than 30% are considered to have shown a response to the anti-HER2 ADC therapy. Patients whose tumors both shrunk by less than 30% and grew by less than 30% are considered to have a stable disease. And patients whose tumor size has increased by more than 30% are considered to have a progressive disease. Of the 22 patients shown in the diagram of FIG. 29 to have exhibited a response to the therapy, nine have a score of IHC 1+, and thirteen have a score of IHC 2+. Thus, the HER2 IHC score is not a good predictor of whether a patient shows a response to the therapy.

FIG. 30 illustrates the response (change in tumor size) for 168 patients from the DESTINY-Breast01 study who had a HER2-positive response score. FIG. 30 illustrates that about 85% of the patients scored as HER2-positive showed a response to the therapy (>30% tumor reduction), 15% had a stable disease, and only one patient had a progressive disease. Thus, a HER2 IHC score of 3+ and 2+ with ISH+ was a better predictor of a response to an anti-HER2 ADC therapy than a score of 2+ and 1+ was a predictor of no response to the anti-HER2 ADC therapy.

FIG. 31 illustrates that a cohort of 65 of the 151 J101 breast cancer patients who fell within the HER2-negative category of IHC scoring still exhibited a favorable objective response rate ORR of 42% to the anti-HER2 ADC therapy. In this HER2-negative cohort of J101 patients (n=65) for which HER2-targeting therapies are not currently approved, 42% of the patients still responded to the anti-HER2 ADC therapy with a median progression-free survival (mPFS) period of 11 months. Thus, scoring using the method of FIG. 14 can be used to identify within the HER2-negative category patients who will exhibit a favorable response to the anti-HER2 ADC therapy. FIG. 31 also shows that the objective response rate was 56% for a cohort of 72 patients categorized as HER-positive using IHC scoring, and those patients responded to therapy with a mPFS of 14.1 months.

FIG. 32 illustrates further stratification of the 65 breast cancer patients in the conventional HER2-negative category using the scoring using the method of FIG. 14. By adjusting the cut-off of the novel predictive ADC score, 40 patients of the 65 HER2-negative group were categorized as "QCS Positive". This cohort of 40 patients had an ORR of 52%, and the patients responded to the anti-HER2 ADC therapy with a mPFS of 14.5 months. Thus, scoring using the method of FIG. 14 was able to identify a subgroup of HER2-negative patients whose novel response scores were above a predetermined threshold (based on staining intensity above a predetermined cut-off) indicating a favorable response to the anti-HER2 ADC therapy.

FIG. 33 shows the results of scoring all of the 151 J101 breast cancer patients using the method of FIG. 14. The upper left figure shows an exemplary result of QCS image analysis that has detected the cell centers of each epithelial cell (little dots), the border of the nucleus, and the border of each cell (membrane). The space between the border of the nucleus and the border of the cell is designated as cytoplasm.

The upper right graph of FIG. 33 shows the distribution of membrane staining of the 151 J101 patients. The height of each bar represents the median tumor cell membrane optical density of the HER2-stained tissue image of each patient. The shading of the bars represents the visual HER2 scoring for each patient by a pathologist. The indicated "cut-off" at >8.04 of membrane optical density was determined on the basis of the survival analysis of the full J101 cohort of patients. The bottom graph of FIG. 33 is a histogram which represents the cellular OD scores and their distribution for a single patient.

FIG. 34A is a more detailed version of the upper right graph of FIG. 33 showing the stratification of the 151 J101 patients into 120 "QCS Positive" (QCS+) patients and 31 "QCS Negative" (QCS-) patients. The group of 120 patients were scored as "QCS Positive" (QCS+) because at least 90% of the cells in their tissue samples exhibited an optical density of HER2 membrane staining above a predetermined threshold of 0.0365 (8.04/220). The group of 120 "QCS Positive" (QCS+) patients exhibited an objective response rate ORR of 56% and a mean progression-free survival (mPFS) period of 14.1 months, as indicated in the table of FIG. 34B.

The novel response score also identified the "QCS Negative" group of 31 patients which had an objective

response rate ORR of 26% and a mean progression-free survival (mPFS) period of 9 months. Thus, the scoring method of FIG. 14 identified a larger portion of the 151 J101 breast cancer patients who could benefit from the anti-HER2 ADC therapy than did conventional HER2 IHC scoring by a pathologist. The ability to identify patients from the conventional HER2-negative IHC scoring group who can nevertheless benefit from the anti-HER2 ADC therapy is critical because there is a high unmet need for effective therapies by patients in this HER2-negative group.

The staining intensity cut-off of the single-cell scores used to generate the response score is adjusted so as to divide the J101 patients into a first group that has a high ORR and a second group that has a low ORR. The best stratification of the J101 patients into a first group with the highest ORR and a second group with the lowest ORR was achieved by lowering the staining intensity threshold of the single-cell scores to 0.0365 (8.04/220) so as to include a larger portion of tumor cells that express at least a minimal amount of HER2. This staining intensity threshold of 8.04/220 contrasts with the current clinical guidelines that set a higher staining cut-off to include only a minority of cells that express a higher level of HER2.

The bar graph of FIG. 34A shows the conventional HER2 IHC score for each of the 151 J101 breast cancer patients, which is indicated by the shading of the bar. For example, the bar for a patient with a score of IHC 3+ is the second darkest gray, and the bar for a patient with a score of IHC 1+ is white. It is evident that all patients with the same HER2 IHC score are not grouped together, other than patients with HER2 IHC 3+ scores that correspond to the highest membrane optical densities. However, patients with much lower membrane optical densities also were likely to respond well to the anti-HER2 ADC therapy. The group of patients that has an ORR of 56% (n=120) is defined as being "HER Positive". Patients in this group were identified as having at least 90% of the cells in their tissue slides exhibit an optical density of HER2 staining greater than the predetermined intensity threshold of 0.0365 (8.04/220), which is lower than the intensity threshold used for a conventional HER2 IHC score of 2+.

In another embodiment, the cells counted as having at least the predetermined threshold of staining intensity also include cells that are within the vicinity of cells with staining above the threshold, but which themselves have staining below the threshold. Thus, the response score also takes into account the spatial heterogeneity of stained cells by characterizing cells as either exhibiting membrane staining above a predetermined optical density threshold (positive cell) or lying within a predetermined distance from a positive cell.

FIG. 35 is a graph of Kaplan-Meier curves of progression-free survival for two groups of J101 breast cancer patients in the HER2-negative cohort identified using the method of FIG. 14 for an embodiment that also considers cells that neighbor stained cells. The method generates scores indicative of the survival probability for the 65 breast cancer patients in the HER2-negative cohort. The upper curve shows the group of patients with better survival outcomes, which corresponds to patients for which 95% of the cells either (i) exhibited an optical density of HER2 staining of at least a minimum threshold, or (ii) were located within a minimum distance from a cell exhibiting the minimal staining.

In the method, all epithelial cells in each tissue sample are identified that exhibited a mean optical density on their membrane greater than 0.04077 (8.97/220); those cells are designated "p1". Then all epithelial cells in the tissue sample are identified that exhibited a mean optical density on the membrane of less than 0.04077, but that are within a distance of 20um (microns) from a p1 cell; those cells are designated "p2". Then the percentage of "p12" cells is computed as (number of p1 + number of p2)/(number of all epithelial cells in the tissue sample). A patient has a higher survival probability and is on the upper Kaplan-Meier curve if the percentage of p12 cells is greater than 95%.

The patients on the upper curve had a mean progression free survival period of 16 months, and the patients on the lower curve had a mean progression free survival period of 9 months. The patients on the upper curve with longer survivability and better response to the anti-HER2 ADC therapy have a more homogenous distribution of HER2-positive cells among HER2-negative cells. If most of the HER2-positive cells are clumped together and not distributed in the tumor tissue within a threshold minimum distance from HER2-negative cells (in this example 20um), then the anti-HER2 ADC that targets the HER2-positive cells does not come close enough to the HER2-negative cells to have any significant bystander effect. The novel predictive QCS score identifies patients with homogenous HER2 expression who will exhibit an improved response from the effect on neighboring cancer cells of the ADC releasing its cytotoxic payload into HER2-positive cells. In order to achieve a response to the anti-HER2 ADC therapy similar to that shown in the upper Kaplan-Meyer curve, the clinician would administer the anti-HER2 ADC therapy to each patient whose novel response score was above 95% p12 cells in their tissue sample.

FIG. 36 is a graph of Kaplan-Meier curves of progression-free survival for two groups of patients from the entire 151 J101 patients using the method of FIG. 14 for an embodiment that also considers p2 cells within a minimum distance of p1 cells. The method generates scores indicative of the survival probability for the 151 breast cancer patients of the J101 trial. The upper curve shows the group of patients with better survival outcomes, which corresponds to patients for which 95% of the cells either (i) exhibited an optical density of HER2 staining of at least a minimum threshold of 0.0481 (10.59244/220), or (ii) were located within a minimum distance of 20um from a cell exhibiting the minimal staining. The patients on the upper curve had a mean progression free survival period of 19 months, and the patients on the lower curve had a mean progression free survival period of 11 months.

FIG. 37 shows other parameters and features used in the method of FIG. 14 to generate the score indicative of the

survival probability of each cancer patient by aggregating all single-cell ADC scores of the tissue sample. The lower 10 features of FIG. 37 are ranked according to the best stratification (measured by the log-rank p-value) between the two curves that was obtained using the particular parameters of the labeled feature.

The upper 9 features of FIG. 37 are ranked according to the average objective response rate of the patients in the upper curve obtained using the particular parameters of the labeled feature. Feature #1 categorizes patients based on the percentage of positive tumor cells, where positivity is defined by a membrane optical density of greater than 5. Feature #2 ranks patients based on the percentage of positive tumor cells, where positivity is defined by a cell optical density of greater than 5 (average optical density of both cytoplasm and membrane). Feature #3 is a bystander score that stratifies patients using positive/negative cell classification, where a positive cell has a membrane optical density greater than 25 and where the score considers all cells within a radius of 100um. Feature #4 is a bystander score that categorizes patients using positive/negative cell classification, where a positive cell has a membrane optical density greater than 10 and where the score considers all cells within a radius of 50um. Feature #5 is a bystander score that categorizes patients using positive/negative cell classification, where a positive cell has a membrane optical density greater than 25 and where the score considers all cells within a radius of 50um. Feature #6 ranks patients by computing the difference between membrane optical density to the cytoplasm optical density for each tumor cell, and then taking the 15% quantile value of the resulting histogram. Feature #7 is a bystander score that categorizes patients using positive/negative cell classification, where a positive cell has a membrane optical density greater than 10 and where the score considers all cells within a radius of 10um. Feature #8 stratifies patients by calculating, for each tumor cell, the membrane optical density * (membrane optical density - cytoplasm optical density), and then taking the 10% quantile value of the resulting histogram. Feature #9 ranks patients by calculating, for each tumor cell, the membrane optical density * (max(0,membrane OD -cytoplasm OD)), and then taking the 10% quantile value of the resulting histogram.

The method of FIG. 14 for predicting a patient's response to an anti-HER2 ADC therapy can optionally be based on the presence of stromal tumor infiltrating lymphocytes (TILs) in addition to the staining characteristics of cancer cell objects. The validation explained below demonstrates that the modified scoring method of FIG. 14 also based on TILs showed an even better correspondence between the objective response rate (ORR) and increased HER2 expression plus TIL prevalence. Tumor infiltrating lymphocytes (TILs) are comprised mainly of cytotoxic (CD8+) T cells and helper (CD4+) T cells. CD8+ T lymphocytes correlate with favorable clinical outcomes, and patients with higher numbers of stromal TILs in their breast lesions exhibit better survival when treated with trastuzumab. Combining TIL prevalence with image analysis-based features extracted from HER2 staining improves the accuracy of the patient score generated by the method of FIG. 14 for predicting how a cancer patient will response to an anti-HER2 ADC therapy, especially for breast cancer patients having tumors with low expression levels of HER2.

In an additional step in the method of FIG. 14, deep learning-based image analysis is performed on digital images of tissue immunohistochemically stained with an anti-Her2 diagnostic antibody linked to a dye. A convolutional neural network is trained to detect TILs in the HER2 stained IHC images. Even though the TILs are not themselves HER2 stained, a deep learning-based model was developed to detect TILS directly from HER2 stained IHC images. For the TIL detection model, a separate subset of regions of interest were annotated for TILs. Point annotations were used to indicate TILs, which in the absence of functional staining are characterized as round to polygonal, relatively small cells with little cytoplasm and a nucleus with homogeneous texture. Only TILs located in tumor-associated stroma and intra-epithelial TILs were annotated and detected. The TIL detection model was applied to non-epithelial areas within the annotated tumor core regions. The TIL centers were detected by thresholding the obtained posterior maps followed by non-maxima suppression.

In another additional step in the method of FIG. 14, a TIL score is computed for each HER2 stained IHC image. Thus, in addition to the HER2 staining-based score, the method uses the density of TILs detected in the stained tissue as a biomarker. The model computes three densities: (i) the density of all TILs within the entire annotated tumor region, (ii) the density of TILs in the segmented tumor-associated stroma, and (iii) the intra-epithelial TIL density.

In order to improve the accuracy of the predictive ADC Score, TIL-based features were used in combination with the features based on HER2 staining. The optimal combination of features is the one that best divides the patient cohort into a group with longer progression free survival (PFS) and a group with shorter PFS as indicated by log-rank test p-values for a Kaplan-Meier curve of all patients in the cohort. The bottom table of FIG. 37 includes three TIL-based features and seven HER2-staining features that are ranked by the stratification that each feature individually achieves between the two curves of a Kaplan-Meier graph as indicated by the log-rank p-value.

FIG. 38 is a table of three additional features and compares their log-rank test p-values and their predicted objective response rates (ORR) for longer and shorter surviving patients. The table of FIG. 38 includes a TIL-based feature, a feature based on the density of HER2+ tumor cells, and feature based on HER2+ cells within a defined neighborhood calculated on the J101 dataset of a cohort of 151 breast cancer patients. The p-values in the table were determined by cross-validation in which the model cutoff was trained in a training set, and then applied in a validation set. The resulting cross-validated model is abbreviated as CM. For example, for the TIL density feature, the cut-off for stratifying the patients into high (QCS+) and low (QCS-) CM groups is 168. This means that if the density of tumor infiltrating

lymphocytes, as measured by the number of TILs in the stroma divided by the area of the tumor core (in mm^2), exceeds 168/mm^2, then the patient belongs to the CM-high group. For the HER2+ cell density feature, the cut-off is 1672. For this feature, a patient belongs to the CM-high group if the density of positive tumor cells, as measured by the number of tumor cells having a membrane optical density greater than 15 divided by the area of the tumor core (in mm^2), exceeds 1672/mm^2. For the HER2+ neighborhood score, the cut-off is 37. The cut-off is applies by first calculating, for all tumor cells, the continuous bystander score using a radius of 25um to generate a histogram. Then if the 5% quantile of the histogram of all cells is larger than 37, the patient belongs to the CM-high group. The TIL density feature achieved the best stratification of the 151 patients and resulted in a log-rank test p-value of 0.007. The feature for HER2+ cell density yielded a Kaplan-Meier, log-rank p-value of 0.011, and the feature for the HER2+ neighborhood score resulted in a p-value of 0.014.

FIGS. 39A-C show Kaplan-Meier curves for the three features listed in the table of FIG. 38. FIG. 39A is a Kaplan-Meier curve for TIL density; FIG. 39B is a Kaplan-Meier curve for HER2+ cell density; and 39C is a Kaplan-Meier curve for the HER2+ neighborhood score. As indicated by the p-values and the Kaplan-Meier curves, the TIL density feature provides the best stratification between patients with longer and shorter progression free survival (PFS) times when applied to the entire 151 breast cancer patients. The Kaplan-Meier curve of FIG. 39C for the HER2+ neighborhood score feature is similar to the Kaplan-Meier curve of FIG. 36 for the feature bystander_pot_cut1_20, which considers unstained bystander cells within a radius of 20um of each HER2-stained tumor cell. The HER2+ neighborhood score feature uses a neighborhood radius of 25um. In comparison, the continuous bystander feature "bystander_pot_cut1_20" of FIG. 36 uses a radius of sigma=20um and an alpha=2 and determines a weighted sum of the membrane optical densities of all tumor cells around a given cell, including the given cell itself. The distance-dependent weighting is calculated by "exp(-0.5*(dist/sigma)^2)".

FIGS. 40A, 40B and 40C show Kaplan-Meier curves for the three features listed in the table of FIG. 38 when applied just to the 72 patients designated as HER2 positive from among the 151 breast cancer patients. Again, the TIL density feature achieved the best stratification with a p-value of 0.00095. The HER2+ cell density feature had a p-value of 0.092, and the HER2+ neighborhood score feature had a p-value of 0.046.

FIGS. 41A, 41B, and 41C show Kaplan-Meier curves for the three features listed in the table of FIG. 38 when applied just to the 65 patients designated as HER2 negative from among the 151 breast cancer patients. For the HER2-negative cohort, however, the TIL density feature provided the worst stratification between longer and shorter progression free survival (PFS). The p-value for the TIL density Kaplan-Meier curve is 0.31. The HER2+ neighborhood score achieved the best stratification with a p-value of 0.0061. The stratification achieved by the HER2+ cell density feature was nearly as good with a p-value of 0.0064. The Kaplan-Meier curve of FIG. 41C for the HER2+ neighborhood score feature is similar to the Kaplan-Meier curve of FIG. 35.

The results of the Kaplan-Meier curves of FIGS. 39-41 indicate that using TIL-based features in combination with the features based on HER2 staining improves the accuracy of the method of FIG. 14 to identify patients who will respond favorably to an anti-HER2 ADC therapy. However, using TIL-based features does not improve the accuracy of identifying patients, from among a subgroup of HER2-negative patients, who are likely to show a favorable response to an anti-HER2 ADC therapy. This suggests that tumor infiltrating lymphocytes (TILs) are not essential to achieve an immediate favorable response of the patient to the anti-HER2 ADC therapy, but the presence of TILs retards the overall progression of the cancer.

D. Validation of prediction method based on gastric cancer patients.

The J101 trial with trastuzumab deruxtecan (DS-8201) also included patients with gastric cancer. Analysis of the dataset of the J101 trial showed that there was a low correlation between quality of response to the anti-HER2 ADC therapy and conventional HER2 IHC scoring. The predicted response using the QCS scoring of the method of FIG. 14 deviated significantly from the results of conventional HER2 IHC scoring.

FIG. 42 shows the results of scoring for a cohort of 32 J101 gastric cancer patients using the method of FIG. 14. The QCS score in this embodiment is based on the median of the mean membrane optical density of all tumor cells in the sample. The statistical operation to aggregate the single-cell ADC scores is the "median". Each single-cell ADC-score is generated by the average (mean) of the DAB optical density in the cell membrane. Patients are ordered according to an increasing QCS score. FIG. 42 shows that the HER2 IHC scores of 0, 1+, 2+ and 3+ do not correspond well with the QCS score. The bar chart of FIG. 42 also shows the clinical outcomes of the 32 gastric cancer patients, which are listed above each bar as complete response (CR), partial response (PR), stable disease (SD), and progressive disease (PD). FIG. 42 shows results for gastric cancer patients similar to those for breast cancer patients in FIG. 34A.

FIG. 43 is a table of six features used in the method of FIG. 14 to generate the score indicative of the survival probability of each gastric cancer patient who received the anti-HER2 ADC therapy by aggregating single-cell ADC scores of the tissue sample. The table of FIG. 43 compares the log-rank test p-values achieved by the features in stratifying patients based on progression free survival (PFS). The table also indicates the predicted objective response rates (ORR)

for the cohort of longer surviving patients (patients in the upper branch of the Kaplan-Meier curve). The ORR is expressed as the positive predictive value (PPV) with respect to the ORR. The positive predictive value (PPV) is the number of patients who are correctly predicted as responders (by observed ORR) divided by all responders. The ORR is measured by RECIST using the response categories CR, PR for ORR=true and SD, PD for ORR=false. FIG. 43 shows features used to stratify gastric cancer patients similar to the features of FIGS. 37-38 used to stratify breast cancer patients. The feature that provided the best stratification of longer and shorter surviving patients was membOD_density_10 (density of tumor cells having a membrane optical density (membOD) larger than 10 per square mm of tumor area), which was based on the optical density of membrane staining of epithelial cells. The membOD_density_10 feature achieved a stratification with a p-value of 0.00594.

FIG. 44 is a table of seven HER2-staining-based features of the model used to stratify patients who received the anti-HER2 ADC therapy based on overall survival (OS) as opposed to progression free survival (PFS). Thus, the list of features is optimized for p-values based on OS as opposed to PFS. The Kurtosis and Skewness features provided the best stratification of patients based on overall survival and achieved p-values of 0.0002.

FIG. 45 is a Kaplan-Meier curve for the feature membOD_density_10 listed in the table of FIG. 43. Based on this feature, a patient is QCS-positive and will more likely benefit from the HER2 ADC therapy if the density of tumor cells having a membrane optical density greater than 10 in the epithelium is greater than 810/mm^2. The feature membOD_density_10 divided the 32 gastric cancer patients based on PFS into a longer surviving cohort of 23 patients and a shorter surviving cohort of 9 patients with a p-value of 0.00594.

FIG. 46 is a Kaplan-Meier curve for the feature membOD_density_10 listed in the table of FIG. 44. The feature membOD_density_10 divided the 32 gastric cancer patients based on OS into a longer surviving cohort of 23 patients and a shorter surviving cohort of 9 patients with a p-value of 0.00731.

## Claims

1. An antibody drug conjugate (ADC) that includes an ADC payload and an ADC antibody that targets a protein on a cancer cell, wherein the protein is human epidermal growth factor receptor 2 (HER2), for use in a method of treating cancer, the method comprising administering a therapy involving the ADC to a cancer patient whose treatment score exceeds a predetermined threshold, wherein the treatment score is generated by:

   staining a tissue sample of the cancer patient immunohistochemically using a dye linked to a diagnostic antibody, wherein the diagnostic antibody binds to the protein on the cancer cells in the tissue sample;
   acquiring a digital image of the tissue sample;
   detecting cancer cells in the digital image;
   computing for each cancer cell a single-cell ADC score based on the staining intensities of the dyes in the membrane and the cytoplasm of the cancer cell, and the staining intensities of the dyes in the membranes and/or cytoplasms of other cancer cells that are closer than a predefined distance to the cancer cell; and
   generating a treatment score by aggregating all single-cell ADC scores of the tissue sample using a statistical operation;
   wherein the staining intensity of each membrane is computed based on an average optical density of a dye signal in pixels of the membrane, and/or wherein the staining intensity of each cytoplasm is computed based on the average optical density of the dye signal in pixels of the cytoplasm;
   wherein the single cell ADC score for a cell i is calculated as:

      a sum of all cells j with $|r_j - r_i|<d$ $\{a_{20}(|r_j - r_i|) \times ODM_j^2 + a_{11}(|r_j - r_i|) \times ODM_j \times ODC_j + a_{02}(|r_j - r_i|) \times ODC_j^2 + a_{00}(|r_j - r_i])\}$,
      wherein the functions $a_{kl}$ depend on a distance $|r_j - r_i|$ of each cell j to each cell i, wherein $ODM_j$ is an optical density of the dye signal in the membrane of cell j, and wherein $ODC_j$ is an optical density of the dye signal in the cytoplasm of cell j;
      wherein the functions $a_{kl}$ depend on the distance $|r_j - r_i|$ of the cell j to the cell i in the relation: $a_{kl}(|r_j - r_i|) = A_{kl} \times \exp(- |r_j - r_i| / r_{norm})$ with predefined constant coefficients $A_{oo}$, $A_{1o}$, $A_{o1}$, $A_{11}$, $A_{20}$, $A_{o2}$; and
      wherein the coefficients $A_{oo}$, $A_{1o}$, $A_{o1}$, $A_{11}$, $A_{20}$, $A_{o2}$, d and $r_{norm}$ are determined by optimizing the correlation of the response score with a therapy response of a cohort of training patients.

2. The ADC for the use of claim 1, wherein the detecting of cancer cells involves detecting for each cancer cell the pixels that belong to the membrane and/or the pixels that belong to the cytoplasm.

3. The ADC for the use of claim 1, wherein the dye signal is a brown diaminobenzidine (DAB) dye signal in pixels of the

membrane, and/or brown DAB dye signal in pixels of the cytoplasm.

**4.** The ADC for the use of claim 1, wherein the aggregating of all single-cell ADC scores is taken from the group consisting of: determining a mean, determining a median, and determining a quantile with a predefined percentage.

**5.** The ADC for the use of any one of claims 1-4, wherein the ADC is Trastuzumab Deruxtecan (DS-8201).

**6.** The ADC for the use of any one of claims 1-4, wherein the ADC antibody is Trastuzumab.

**7.** The ADC for the use of any one of claims 1-4, wherein the ADC payload is topoisomerase I inhibitor.

**8.** The ADC for the use of any one of claims 1-4, wherein the diagnostic antibody is Ventana anti-HER2/neu 4B5.

**9.** The ADC for the use of any one of claims 1-4, wherein the dye is 3,3'-Diaminobenzidine (DAB).

**10.** The ADC for the use of any one of claims 1-9, wherein the cancer patient has a cancer selected from the group consisting of: breast cancer, gastric cancer, colorectal cancer, lung cancer, esophageal cancer, head-and-neck cancer, esophagogastric junction cancer, biliary tract cancer, Paget's disease, pancreatic cancer, ovarian cancer, uterine cancer sarcoma, bladder cancer, prostate cancer, urothelial cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, sarcoma, osteosarcoma, and melanoma;
for example a cancer selected from the group consisting of: breast cancer, gastric cancer, colorectal cancer and lung cancer.

**11.** The ADC for the use of any one of claims 1-10, wherein the ADC is an anti-HER2 antibody conjugated to a drug-linker via a thioether bond, wherein the drug-linker is represented by the following formula:

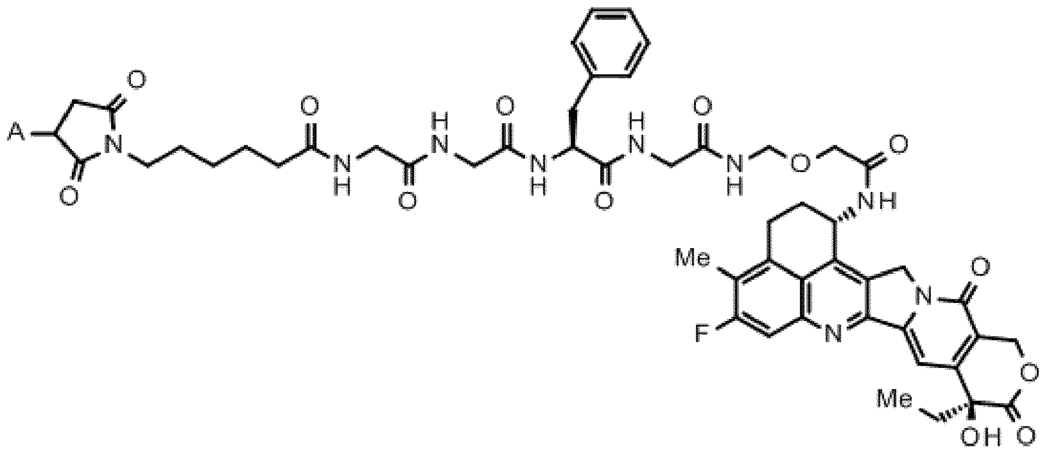

,

and

wherein A represents a connecting position to the anti-HER2 antibody;
for example wherein the anti-HER2 antibody comprises:

a heavy chain comprising CDRH1 consisting of an amino acid sequence represented by SEQ ID NO: 4, CDRH2 consisting of an amino acid sequence represented by SEQ ID NO: 5, and CDRH3 consisting of an amino acid sequence represented by SEQ ID NO: 6; and
a light chain comprising CDRL1 consisting of an amino acid sequence represented by SEQ ID NO: 7, CDRL2 consisting of an amino acid sequence consisting of amino acid residues 1 to 3 of SEQ ID NO: 8, and CDRL3 consisting of an amino acid sequence represented by SEQ ID NO: 9;

for example an anti-HER2 antibody comprising:

a heavy chain variable region consisting of the amino acid sequence represented by SEQ ID NO: 10; and
a light chain variable region consisting of the amino acid sequence represented by SEQ ID NO: 11.

**12.** The ADC for the use of claim 11, wherein the ADC includes an anti-HER2 antibody comprising:

a heavy chain consisting of the amino acid sequence represented by SEQ ID NO: 12; and
a light chain consisting of the amino acid sequence represented by SEQ ID NO: 3; or comprising:
a heavy chain consisting of the amino acid sequence represented by SEQ ID NO: 2; and
a light chain consisting of the amino acid sequence represented by SEQ ID NO: 3.

**Patentansprüche**

**1.** Antikörper-Wirkstoff-Konjugat (ADC), das eine ADC-Nutzlast und einen ADC-Antikörper beinhaltet, der ein Protein auf einer Krebszelle angreift, wobei das Protein ein humaner epidermaler Wachstumsfaktorrezeptor 2 (HER2) ist, zur Verwendung in einem Verfahren zur Krebsbehandlung, wobei das Verfahren das Verabreichen einer Therapie mit dem ADC an einen Krebspatienten involviert, dessen Behandlungsscore einen vorbestimmten Schwellenwert überschreitet, wobei der Behandlungsscore wie folgt generiert wird:

Färben einer Gewebeprobe des Krebspatienten immunhistochemisch mit einem Farbstoff, der an einen diagnostischen Antikörper gekoppelt ist, wobei der diagnostische Antikörper an das Protein auf den Krebszellen in der Gewebeprobe bindet;
Aufnehmen eines digitalen Bildes der Gewebeprobe;
Detektieren von Krebszellen im digitalen Bild;
Berechnen eines Einzelzell-ADC-Scores für jede Krebszelle basierend auf den Färbeintensitäten der Farbstoffe in der Membran und im Zytoplasma der Krebszelle sowie den Färbeintensitäten der Farbstoffe in den Membranen und/oder Zytoplasmen anderer Krebszellen, die sich näher als eine vordefinierte Entfernung zur Krebszelle befinden; und
Generieren eines Behandlungsscores durch Aggregation aller Einzelzell-ADC-Scores der Gewebeprobe mittels einer statistischen Operation;
wobei die Färbeintensität jeder Membran auf der Grundlage einer durchschnittlichen optischen Dichte eines Farbstoffsignals in Pixeln der Membran berechnet wird und/oder wobei die Färbeintensität jedes Zytoplasmas auf der Grundlage der durchschnittlichen optischen Dichte des Farbstoffsignals in Pixeln des Zytoplasmas berechnet wird;
wobei der Einzelzell-ADC-Score für eine Zelle i wie folgt berechnet wird:

die Summe aller Zellen j mit $|r_j - r_i| < d\{a_{20}(|r_j - r_i|) \times ODM_j^2 + a_{11}(|r_j - r_i|) \times ODM_j \times ODC_j + a_{02}(|r_j - r_i|) \times ODC_j^2 + a_{00}(|r_j - r_i|)\}$,

wobei die Funktionen $a_{k1}$ von einem Abstand $|r_j - r_i|$ jeder Zelle j zu jeder Zelle i abhängen, wobei $ODM_j$ eine optische Dichte des Farbstoffsignals in der Membran der Zelle j ist und wobei $ODC_j$ die optische Dichte des Farbstoffsignals im Zytoplasma der Zelle j ist;
wobei die Funktionen $a_{k1}$ von der Distanz $|r_j - r_i|$ der Zelle j zur Zelle i in der folgenden Beziehung abhängen: $a_{kl}(|r_j - r_i|) = A_{k1} \times \exp(-|r_j - r_i|/r_{norm})$ mit vordefinierten konstanten Koeffizienten $A_{00}$, $A_{10}$, $A_{01}$, $A_{11}$, $A_{20}$, $A_{02}$; und
wobei die Koeffizienten $A_{00}$, $A_{10}$, $A_{01}$, $A_{11}$, $A_{20}$, $A_{o2}$, d und $r_{norm}$ durch Optimieren der Korrelation des Antwort-Scores mit einer Therapieantwort einer Kohorte von Trainingspatienten bestimmt werden.

**2.** ADC zur Verwendung nach Anspruch 1, wobei das Detektieren von Krebszellen Detektieren der zur Membran gehörenden Pixel für jede Krebszelle und/oder der zum Zytoplasma gehörenden Pixel involviert.

**3.** ADC zur Verwendung nach Anspruch 1, wobei das Farbstoffsignal ein braunes Diaminobenzidin- (DAB)- Farbstoffsignal in Pixeln der Membran und/oder ein braunes DAB-Farbstoffsignal in Pixeln des Zytoplasmas ist.

**4.** ADC zur Verwendung nach Anspruch 1, wobei das Aggregieren aller Einzelzell-ADC-Scores aus der Gruppe erfolgt, bestehend aus: Bestimmen eines Mittelwerts, Bestimmen eines Medianwerts und Bestimmen eines Quantils mit einem vordefinierten Prozentsatz.

**5.** ADC zur Verwendung nach einem der Ansprüche 1-4, wobei das ADC Trastuzumab Deruxtecan (DS-8201) ist.

**6.** ADC zur Verwendung nach einem der Ansprüche 1-4, wobei der ADC-Antikörper Trastuzumab ist.

**7.** ADC zur Verwendung nach einem der Ansprüche 1-4, wobei die Nutzlast des ADC ein Topoisomerase-I-Inhibitor ist.

**8.** ADC zur Verwendung nach einem der Ansprüche 1-4, wobei der diagnostische Antikörper Ventana Anti-HER2/neu 4B5 ist.

**9.** ADC zur Verwendung nach einem der Ansprüche 1-4, wobei der Farbstoff 3,3'-Diaminobenzidin (DAB) ist.

**10.** ADC zur Verwendung nach einem der Ansprüche 1-9, wobei der Krebspatient an einer Krebserkrankung leidet, die aus der Gruppe ausgewählt ist, bestehend aus: Brustkrebs, Magenkrebs, Darmkrebs, Lungenkrebs, Speiseröhrenkrebs, Kopf-Hals-Krebs, Krebs des ösophagogastralen Übergangs, Gallenwegskrebs, Morbus Paget, Bauchspeicheldrüsenkrebs, Eierstockkrebs, Gebärmutterkrebs-Sarkom, Blasenkrebs, Prostatakrebs, Urothelkarzinom, gastrointestinalem Stromatumor, Gebärmutterhalskrebs, Plattenepithelkarzinom, Peritonealkrebs, Leberkrebs, hepatozellulärem Krebs, Endometriumkarzinom, Nierenkrebs, Vulvakrebs, Schilddrüsenkrebs, Peniskrebs, Leukämie, malignem Lymphom, Plasmozytom, Myelom, Glioblastom, Sarkom, Osteosarkom und Melanom;
zum Beispiel eine Krebsart, ausgewählt aus der Gruppe bestehend aus: Brustkrebs, Magenkrebs, Darmkrebs und Lungenkrebs.

**11.** ADC zur Verwendung nach einem der Ansprüche 1-10, wobei das ADC ein Anti-HER2-Antikörper ist, der über eine Thioetherbindung an einen Wirkstoff-Linker konjugiert ist, wobei der Wirkstoff-Linker durch die folgende Formel dargestellt wird:

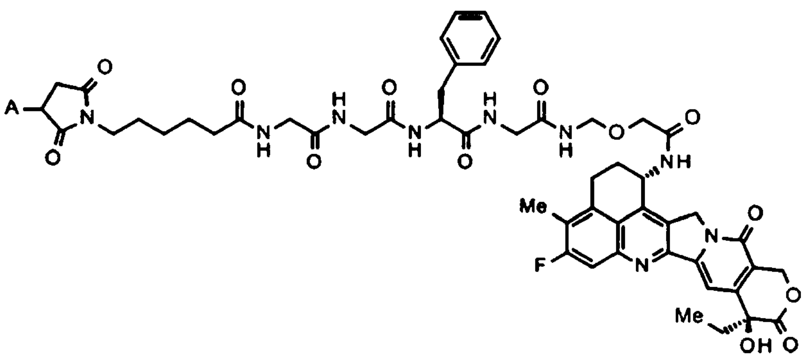

,

und

wobei A eine Verbindungsstelle zum Anti-HER2-Antikörper darstellt;
wobei der Anti-HER2-Antikörper zum Beispiel Folgendes umfasst:

eine schwere Kette umfassend CDRH1, bestehend aus einer Aminosäuresequenz, die durch SEQ ID NO: 4 dargestellt wird, CDRH2, bestehend aus einer Aminosäuresequenz, die durch SEQ ID NO: 5 dargestellt wird, und CDRH3, bestehend aus einer Aminosäuresequenz, die durch SEQ ID NO: dargestellt wird 6; und eine leichte Kette umfassend CDRL1, bestehend aus einer Aminosäuresequenz, die durch SEQ ID NO: 7 dargestellt wird, CDRL2, bestehend aus einer Aminosäuresequenz, die aus Aminosäureresten 1 bis 3 von SEQ ID NO: 8 besteht, und CDRL3, bestehend aus einer Aminosäuresequenz, die durch SEQ ID NO: dargestellt wird 9;

zum Beispiel ein Anti-HER2-Antikörper, der Folgendes umfasst:

eine variable Region der schweren Kette, bestehend aus der Aminosäuresequenz, die durch SEQ ID Nr. 10 dargestellt wird; und
eine variable Region der leichten Kette, bestehend aus der Aminosäuresequenz, die durch SEQ ID NO: 11 dargestellt wird.

**12.** ADC zur Verwendung nach Anspruch 11, wobei der ADC einen Anti-HER2-Antikörper beinhaltet, der Folgendes umfasst:

eine schwere Kette, bestehend aus der Aminosäuresequenz, die durch SEQ ID NO: 12 dargestellt wird; und eine leichte Kette, bestehend aus der Aminosäuresequenz, die durch SEQ ID NO: 3 dargestellt wird; oder umfassend:

eine schwere Kette, bestehend aus der Aminosäuresequenz, die durch SEQ ID NO: 2 dargestellt wird; und eine leichte Kette, bestehend aus der Aminosäuresequenz, die durch SEQ ID NO: 3 dargestellt wird.

**Revendications**

1. Conjugué anticorps-médicament (ADC) qui inclut une charge utile d'ADC et un anticorps d'ADC qui cible une protéine sur une cellule cancéreuse, dans lequel la protéine est le récepteur 2 du facteur de croissance épidermique humain (HER2), destiné à être utilisé dans un procédé de traitement du cancer, le procédé comprenant l'administration d'une thérapie impliquant l'ADC à un patient atteint de cancer dont le score de traitement dépasse un seuil prédéterminé, dans lequel le score de traitement est généré par :

   la coloration immunohistochimique d'un échantillon de tissu du patient atteint de cancer à l'aide d'un colorant lié à un anticorps de diagnostic, dans lequel l'anticorps de diagnostic se lie à la protéine présente sur les cellules cancéreuses de l'échantillon de tissu ;
   l'acquisition d'une image numérique de l'échantillon de tissu ;
   la détection de cellules cancéreuses dans l'image numérique ;
   le calcul, pour chaque cellule cancéreuse, d'un score d'ADC unicellulaire sur la base des intensités de coloration des colorants dans la membrane et le cytoplasme de la cellule cancéreuse, et des intensités de coloration des colorants dans les membranes et/ou les cytoplasmes d'autres cellules cancéreuses qui sont plus proches qu'une distance prédéfinie de la cellule cancéreuse ; et
   la génération d'un score de traitement en agrégeant tous les scores d'ADC unicellulaires de l'échantillon de tissu à l'aide d'une opération statistique ;
   dans lequel l'intensité de coloration de chaque membrane est calculée sur la base d'une densité optique moyenne d'un signal de colorant dans les pixels de la membrane, et/ou dans lequel l'intensité de coloration de chaque cytoplasme est calculée sur la base de la densité optique moyenne du signal de colorant dans les pixels du cytoplasme ;
   dans lequel le score d'ADC unicellulaire pour une cellule i est calculé comme suit : une somme de toutes les cellules j avec $|r_j - r_i| < d\{a_{20}(|r_j - r_i|) \times ODM_j^2 + a_{11}(|r_j - r_i|) \times ODM_j \times ODC_j + a_{02}(|r_j - r_i|) \times ODC_j^2 + a_{00}(|r_j - r_i|)\}$,
   dans lequel les fonctions $a_{kl}$ dépendent d'une distance allant $|r_j - r_i|$ de chaque cellule j à chaque cellule i, dans lequel $ODM_j$ représente la densité optique du signal de colorant dans la membrane de la cellule j, et dans lequel $ODC_j$ représente la densité optique du signal de colorant dans le cytoplasme de la cellule j ;
   dans lequel les fonctions $a_{kl}$ dépendent de la distance allant $|r_j - r_i|$ de la cellule j à la cellule i selon la relation : $a_{kl}(|r_j - r_i|) = A_{kl} \times \exp(-|r_j - r_i|/r_{norm})$ avec des coefficients constants prédéfinis $A_{00}$, $A_{10}$, $A_{01}$, $A_{11}$, $A_{20}$, $A_{02}$ ; et
   dans lequel les coefficients $A_{00}$, $A_{10}$, $A_{01}$, $A_{11}$, $A_{20}$, $A_{o2}$, d et $r_{norm}$ sont déterminés en optimisant la corrélation du score de réponse avec une réponse thérapeutique d'une cohorte de patients en formation.

2. ADC destiné à être utilisé selon la revendication 1, dans lequel la détection de cellules cancéreuses implique la détection, pour chaque cellule cancéreuse, des pixels qui appartiennent à la membrane et/ou des pixels qui appartiennent au cytoplasme.

3. ADC destiné à être utilisé selon la revendication 1, dans lequel le signal de colorant est un signal de colorant de diaminobenzidine (DAB) brun dans les pixels de la membrane et/ou un signal de colorant de DAB brun dans les pixels du cytoplasme.

4. ADC destiné à être utilisé selon la revendication 1, dans lequel l'agrégation de tous les scores d'ADC unicellulaires est prise à partir du groupe consistant en : la détermination d'une moyenne, la détermination d'une médiane et la détermination d'un quantile avec un pourcentage prédéfini.

5. ADC destiné à être utilisé selon l'une quelconque des revendications 1-4, dans lequel l'ADC est le trastuzumab déruxtécan (DS-8201).

6. ADC destiné à être utilisé selon l'une quelconque des revendications 1-4, dans lequel l'anticorps d'ADC est le trastuzumab.

7. ADC destiné à être utilisé selon l'une quelconque des revendications 1-4, dans lequel la charge utile d'ADC est un inhibiteur de la topoisomérase I.

8. ADC destiné à être utilisé selon l'une quelconque des revendications 1-4, dans lequel l'anticorps de diagnostic est Ventana anti-HER2/neu 4B5.

9. ADC destiné à être utilisé selon l'une quelconque des revendications 1-4, dans lequel le colorant est la 3,3'-diaminobenzidine (DAB).

10. ADC destiné à être utilisé selon l'une quelconque des revendications 1-9, dans lequel le patient atteint de cancer présente un cancer choisi parmi le groupe consistant en : cancer du sein, cancer de l'estomac, cancer colorectal, cancer du poumon, cancer de l'œsophage, cancer de la tête et du cou, cancer de la jonction cesogastrique, cancer des voies biliaires, maladie de Paget, cancer du pancréas, cancer de l'ovaire, sarcome de l'utérus, cancer de la vessie, cancer de la prostate, cancer urothélial, tumeur stromale gastro-intestinale, cancer du col de l'utérus, carcinome épidermoïde, cancer du péritoine, cancer du foie, carcinome hépatocellulaire, cancer de l'endomètre, cancer du rein, cancer de la vulve, cancer de la thyroïde, cancer du pénis, leucémie, lymphome malin, plasmocytome, myélome, glioblastome multiforme, sarcome, ostéosarcome et mélanome ;
par exemple, un cancer choisi parmi le groupe consistant en : cancer du sein, cancer de l'estomac, cancer colorectal et cancer du poumon.

11. ADC destiné à être utilisé selon l'une quelconque des revendications 1-10, dans lequel l'ADC est un anticorps anti-HER2 conjugué à un lieur de médicament via une liaison thioéther, dans lequel le lieur de médicament est représenté par la formule suivante :

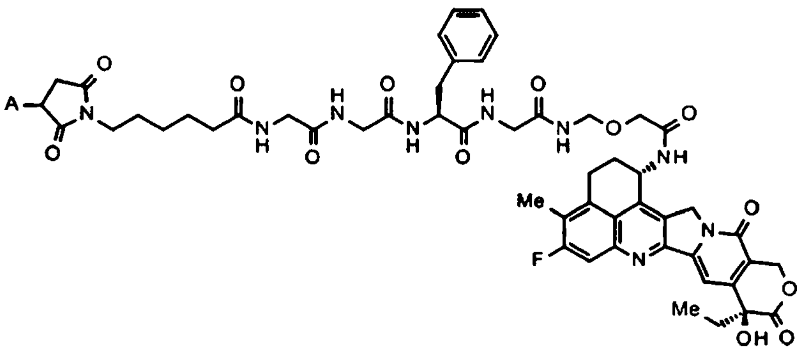

,

et

dans lequel A représente une position de liaison à l'anticorps anti-HER2 ;
par exemple, dans lequel l'anticorps anti-HER2 comprend :

une chaîne lourde comprenant CDRH1 consistant en une séquence d'acides aminés représentée par SEQ ID NO : 4, CDRH2 consistant en une séquence d'acides aminés représentée par SEQ ID NO : 5 et CDRH3 consistant en une séquence d'acides aminés représentée par SEQ ID NO : 6 ; et
une chaîne légère comprenant CDRL1 consistant en une séquence d'acides aminés représentée par SEQ ID NO : 7, CDRL2 consistant en une séquence d'acides aminés consistant en des résidus d'acides aminés allant 1 à 3 de SEQ ID NO : 8, et CDRL3 consistant en une séquence d'acides aminés représentée par SEQ ID NO : 9 ;

par exemple un anticorps anti-HER2 comprenant :

une région variable de chaîne lourde consistant en la séquence d'acides aminés représentée par SEQ ID NO : 10 ; et
une région variable de chaîne légère consistant en la séquence d'acides aminés représentée par SEQ ID

NO : 11.

**12.** ADC destiné à être utilisé selon la revendication 11, dans lequel l'ADC inclut un anticorps anti-HER2 comprenant :

une chaîne lourde consistant en la séquence d'acides aminés représentée par SEQ ID NO : 12 ; et
une chaîne légère consistant en la séquence d'acides aminés représentée par SEQ ID NO : 3 ; ou comprenant :

une chaîne lourde consistant en la séquence d'acides aminés représentée par SEQ ID NO : 2 ; et
une chaîne légère consistant en la séquence d'acides aminés représentée par SEQ ID NO : 3.

M E L A A L C R W G L L L L A L L P P G A A S T Q V C T G T D M K L R L P A S
P E T H L D M L R H L Y Q G C Q V V Q G N L E L T Y L P T N A S L S F L Q D
I Q E V Q G Y V L I A H N Q V R Q V P L Q R L R I V R G T Q L F E D N Y A L
A V L D N G D P L N N T T P V T G A S P G G L R E L Q L R S L T E I L K G G
V L I Q R N P Q L C Y Q D T I L W K D I F H K N N Q L A L T L I D T N R S R
A C H P C S P M C K G S R C W G E S S E D C Q S L T R T V C A G G C A R C K
G P L P T D C C H E Q C A A G C T G P K H S D C L A C L H F N H S G I C E L
H C P A L V T Y N T D T F E S M P N P E G R Y T F G A S C V T A C P Y N Y L
S T D V G S C T L V C P L H N Q E V T A E D G T Q R C E K C S K P C A R V C
Y G L G M E H L R E V R A V T S A N I Q E F A G C K K I F G S L A F L P E S
F D G D P A S N T A P L Q P E Q L Q V F E T L E E I T G Y L Y I S A W P D S
L P D L S V F Q N L Q V I R G R I L H N G A Y S L T L Q G L G I S W L G L R
S L R E L G S G L A L I H H N T H L C F V H T V P W D Q L F R N P H Q A L L
H T A N R P E D E C V G E G L A C H Q L C A R G H C W G P G P T Q C V N C S
Q F L R G Q E C V E E C R V L Q G L P R E Y V N A R H C L P C H P E C Q P Q
N G S V T C F G P E A D Q C V A C A H Y K D P P F C V A R C P S G V K P D L
S Y M P I W K F P D E E G A C Q P C P I N C T H S C V D L D D K G C P A E Q
R A S P L T S I I S A V V G I L L V V V L G V V F G I L I K R R Q Q K I R K
Y T M R R L L Q E T E L V E P L T P S G A M P N Q A Q M R I L K E T E L R K
V K V L G S G A F G T V Y K G I W I P D G E N V K I P V A I K V L R E N T S
P K A N K E I L D E A Y V M A G V G S P Y V S R L L G I C L T S T V Q L V T
Q L M P Y G C L L D H V R E N R G R L G S Q D L L N W C M Q I A K G M S Y L
E D V R L V H R D L A A R N V L V K S P N H V K I T D F G L A R L L D I D E
T E Y H A D G G K V P I K W M A L E S I L R R R F T H Q S D V W S Y G V T V
W E L M T F G A K P Y D G I P A R E I P D L L E K G E R L P Q P P I C T I D
V Y M I M V K C W M I D S E C R P R F R E L V S E F S R M A R D P Q R F V V
I Q N E D L G P A S P L D S T F Y R S L L E D D D M G D L V D A E E Y L V P
Q Q G F F C P D P A P G A G G M V H H R H R S S S T R S G G G D L T L G L E
P S E E E A P R S P L A P S E G A G S D V F D G D L G M G A A K G L Q S L P
T H D P S P L Q R Y S E D P T V P L P S E T D G Y V A P L T C S P Q P E Y V
N Q P D V R P Q P P S P R E G P L P A A R P A G A T L E R P K T L S P G K N
G V V K D V F A F G G A V E N P E Y L T P Q G G A A P Q P H P P P A F S P A
F D N L Y Y W D Q D P P E R G A P P S T F K G T P T A E N P E Y L G L D V P
V

(SEQ ID No. 1: Amino acid sequence of HER2 protein)

FIG. 1

E V Q L V E S G G G L V Q P G G S L R L S C A A S G F N I K D T Y I H W V R
Q A P G K G L E W V A R I Y P T N G Y T R Y A D S V K G R F T I S A D T S K
N T A Y L Q M N S L R A E D T A V Y Y C S R W G G D G F Y A M D Y W G Q G T
L V T V S S A S T K G P S V F P L A P S S K S T S G G T A A L G C L V K D Y
F P E P V T V S W N S G A L T S G V H T F P A V L Q S S G L Y S L S S V V T
V P S S S L G T Q T Y I C N V N H K P S N T K V D K K V E P K S C D K T H T
C P P C P A P E L L G G P S V F L F P P K P K D T L M I S R T P E V T C V V
V D V S H E D P E V K F N W Y V D G V E V H N A K T K P R E E Q Y N S T Y R
V V S V L T V L H Q D W L N G K E Y K C K V S N K A L P A P I E K T I S K A
K G Q P R E P Q V Y T L P P S R E E M T K N Q V S L T C L V K G F Y P S D I
A V E W E S N G Q P E N N Y K T T P P V L D S D G S F F L Y S K L T V D K S
R W Q Q G N V F S C S V M H E A L H N H Y T Q K S L S L S P G K

(SEQ ID No. 2: Amino acid sequence of a heavy chain of the anti-HER2 antibody)

FIG. 2

D I Q M T Q S P S S L S A S V G D R V T I T C R A S Q D V N T A V A W Y Q Q
K P G K A P K L L I Y S A S F L Y S G V P S R F S G S R S G T D F T L T I S
S L Q P E D F A T Y Y C Q Q H Y T T P P T F G Q G T K V E I K R T V A A P S
V F I F P P S D E Q L K S G T A S V V C L L N N F Y P R E A K V Q W K V D N
A L Q S G N S Q E S V T E Q D S K D S T Y S L S S T L T L S K A D Y E K H K
V Y A C E V T H Q G L S S P V T K S F N R G E C .

(SEQ ID No. 3: Amino acid sequence of a light chain of the anti-HER2 antibody)

FIG. 3

G F N I K D T Y

(SEQ ID No. 4: Amino acid sequence of CDRH1 of the anti-HER2 antibody)

FIG. 4

I Y P T N G Y T

(SEQ ID No. 5: Amino acid sequence of CDRH2 of the anti-HER2 antibody)

FIG. 5

S R W G G D G F Y A M D Y

(SEQ ID No. 6: Amino acid sequence of CDRH3 of the anti-HER2 antibody)

FIG. 6

Q D V N T A

(SEQ ID No. 7: Amino acid sequence of CDRL1 of the anti-HER2 antibody)

FIG. 7

S A S F L Y S

(SEQ ID No. 8: Amino acid sequence of CDRL2 (SAS) of the anti-HER2 antibody)

FIG. 8

Q Q H Y T T P P T

(SEQ ID No. 9: Amino acid sequence of CDRL3 of the anti-HER2 antibody)

FIG. 9

E V Q L V E S G G G L V Q P G G S L R L S C A A S G F N I K D T Y I H W V R
Q A P G K G L E W V A R I Y P T N G Y T R Y A D S V K G R F T I S A D T S K
N T A Y L Q M N S L R A E D T A V Y Y C S R W G G D G F Y A M D Y W G Q G T
L V T V S S

(SEQ ID No. 10: Amino acid sequence of a heavy chain variable region of the anti-HER2 antibody)

FIG. 10

D I Q M T Q S P S S L S A S V G D R V T I T C R A S Q D V N T A V A W Y Q Q
K P G K A P K L L I Y S A S F L Y S G V P S R F S G S R S G T D F T L T I S
S L Q P E D F A T Y Y C Q Q H Y T T P P T F G Q G T K V E I K

(SEQ ID No. 11: Amino acid sequence of a light chain variable region of the anti-HER2 antibody)

FIG. 11

E V Q L V E S G G G L V Q P G G S L R L S C A A S G F N I K D T Y I H W V R
Q A P G K G L E W V A R I Y P T N G Y T R Y A D S V K G R F T I S A D T S K
N T A Y L Q M N S L R A E D T A V Y Y C S R W G G D G F Y A M D Y W G Q G T
L V T V S S A S T K G P S V F P L A P S S K S T S G G T A A L G C L V K D Y
F P E P V T V S W N S G A L T S G V H T F P A V L Q S S G L Y S L S S V V T
V P S S S L G T Q T Y I C N V N H K P S N T K V D K K V E P K S C D K T H T
C P P C P A P E L L G G P S V F L F P P K P K D T L M I S R T P E V T C V V
V D V S H E D P E V K F N W Y V D G V E V H N A K T K P R E E Q Y N S T Y R
V V S V L T V L H Q D W L N G K E Y K C K V S N K A L P A P I E K T I S K A
K G Q P R E P Q V Y T L P P S R E E M T K N Q V S L T C L V K G F Y P S D I
A V E W E S N G Q P E N N Y K T T P P V L D S D G S F F L Y S K L T V D K S
R W Q Q G N V F S C S V M H E A L H N H Y T Q K S L S L S P G

(SEQ ID No. 12: Amino acid sequence of a heavy chain of the anti-HER2 antibody)

FIG. 12

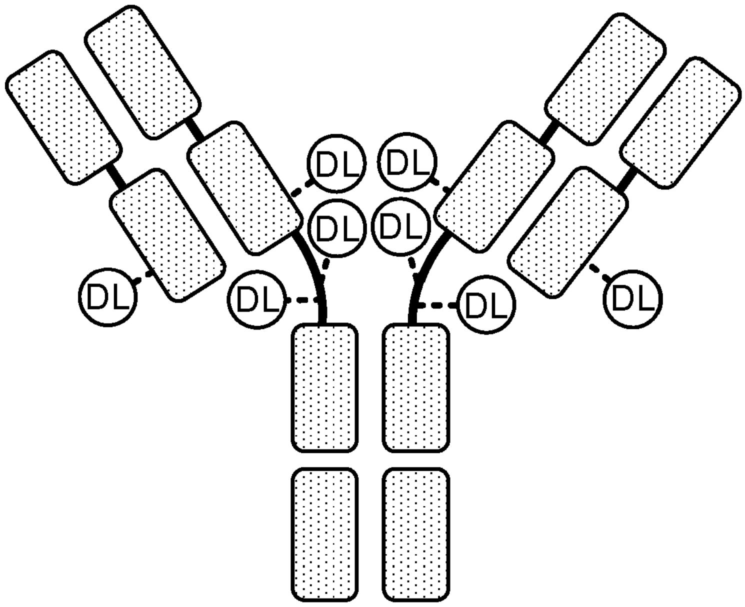


(Humanized anti-HER2 IgG1 mAb[1-3])

FIG. 13

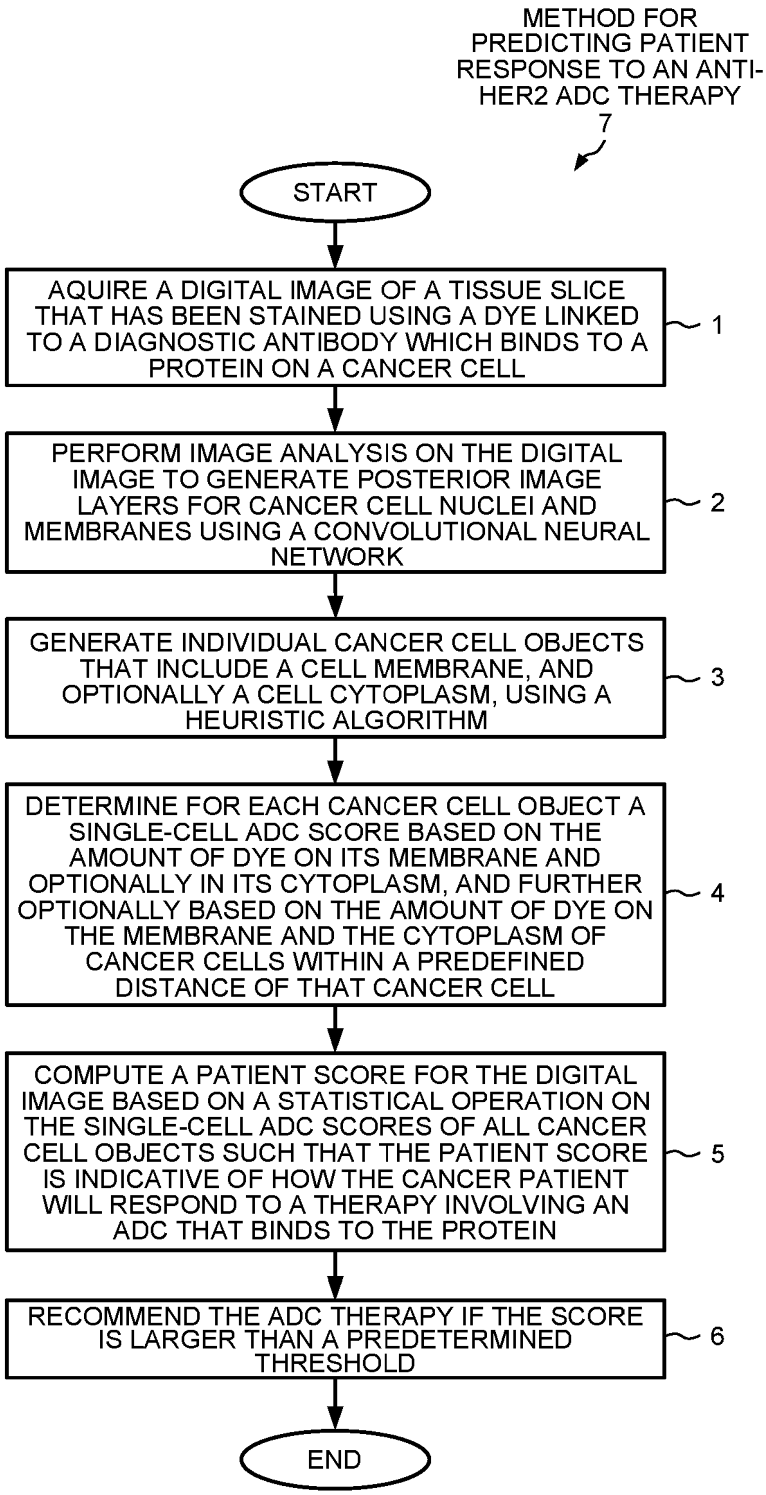
METHOD FOR PREDICTING PATIENT RESPONSE TO AN ANTI-HER2 ADC THERAPY
7
START
AQUIRE A DIGITAL IMAGE OF A TISSUE SLICE THAT HAS BEEN STAINED USING A DYE LINKED TO A DIAGNOSTIC ANTIBODY WHICH BINDS TO A PROTEIN ON A CANCER CELL
1
PERFORM IMAGE ANALYSIS ON THE DIGITAL IMAGE TO GENERATE POSTERIOR IMAGE LAYERS FOR CANCER CELL NUCLEI AND MEMBRANES USING A CONVOLUTIONAL NEURAL NETWORK
2
GENERATE INDIVIDUAL CANCER CELL OBJECTS THAT INCLUDE A CELL MEMBRANE, AND OPTIONALLY A CELL CYTOPLASM, USING A HEURISTIC ALGORITHM
3
DETERMINE FOR EACH CANCER CELL OBJECT A SINGLE-CELL ADC SCORE BASED ON THE AMOUNT OF DYE ON ITS MEMBRANE AND OPTIONALLY IN ITS CYTOPLASM, AND FURTHER OPTIONALLY BASED ON THE AMOUNT OF DYE ON THE MEMBRANE AND THE CYTOPLASM OF CANCER CELLS WITHIN A PREDEFINED DISTANCE OF THAT CANCER CELL
4
COMPUTE A PATIENT SCORE FOR THE DIGITAL IMAGE BASED ON A STATISTICAL OPERATION ON THE SINGLE-CELL ADC SCORES OF ALL CANCER CELL OBJECTS SUCH THAT THE PATIENT SCORE IS INDICATIVE OF HOW THE CANCER PATIENT WILL RESPOND TO A THERAPY INVOLVING AN ADC THAT BINDS TO THE PROTEIN
5
RECOMMEND THE ADC THERAPY IF THE SCORE IS LARGER THAN A PREDETERMINED THRESHOLD
6
END

FIG. 14

Digital image of a HER2/neu (4B5) stained tissue section

17

Posterior layer for cell nuclei, generated by a CNN

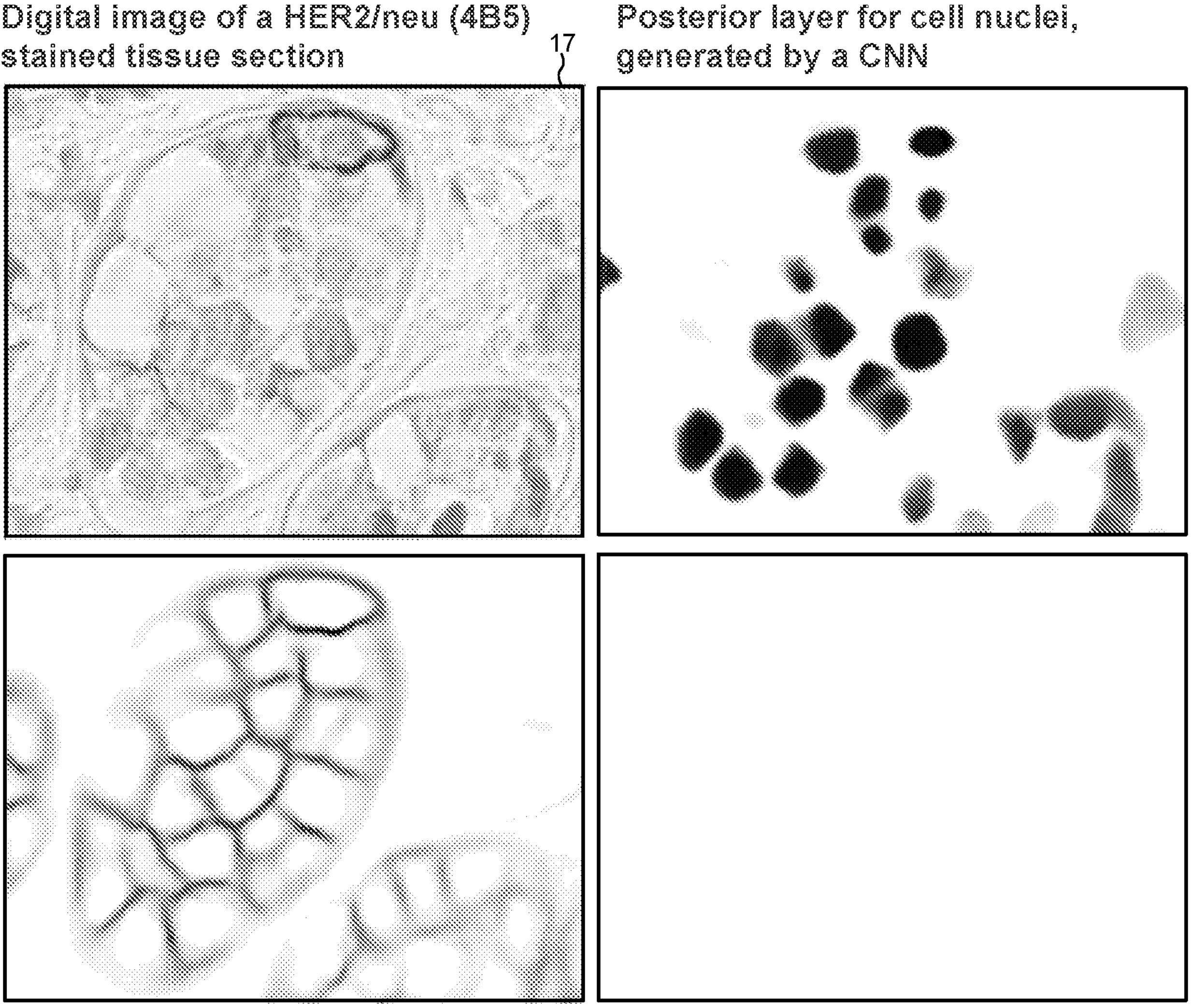

Posterior layer for cell membranes generated by the CNN

Unique Identifiers for cells, initially empty

In the images, dark corresponds to high probability, and white corresponds to low probability

FIG. 15

Digital image of a HER2/neu (4B5) stained tissue section

Posterior layer for cell nuclei, generated by a first CNN

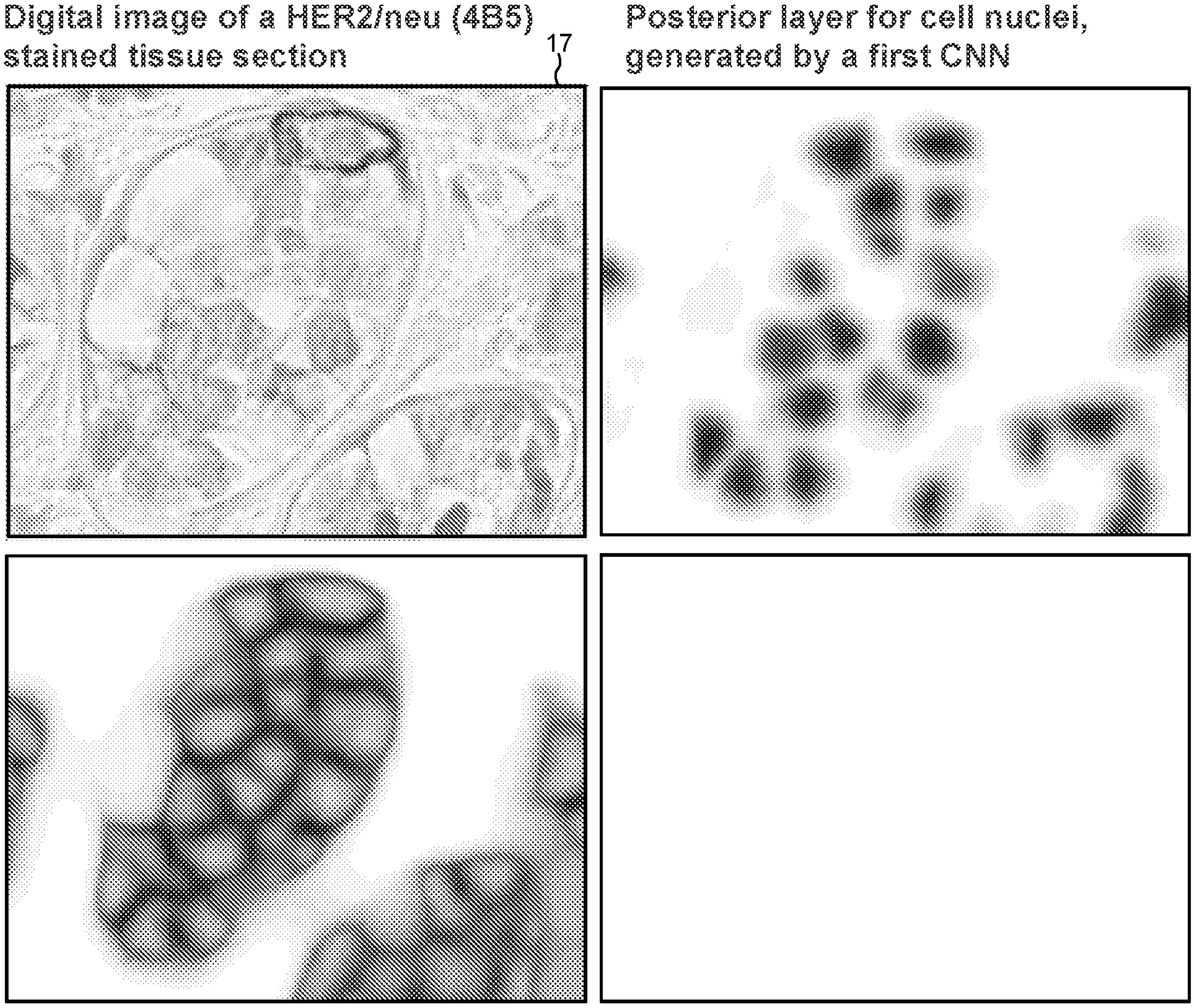


Posterior layer for cell membranes generated by a second CNN

Unique Identifiers for cells, initially empty

In the images, dark corresponds to high probability, and white corresponds to low probability

FIG. 16

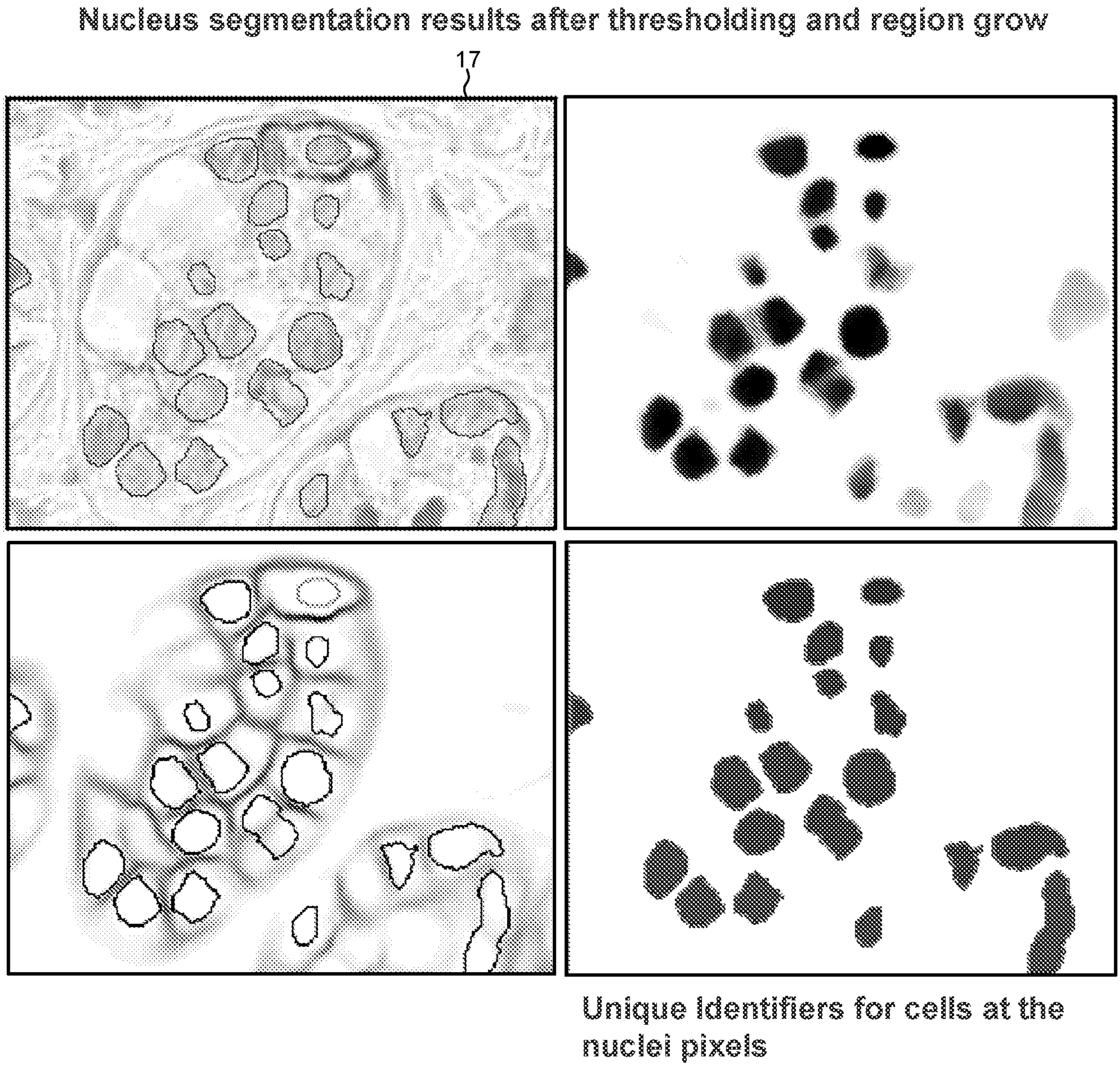
Nucleus segmentation results after thresholding and region grow
17
Unique Identifiers for cells at the nuclei pixels

FIG. 17

Cell segmentation results by region growing of each nucleus with restrictions:
(1) not to grow downhill in the membrane posterior,
(2) and use a minimal object density constraint for the cell border

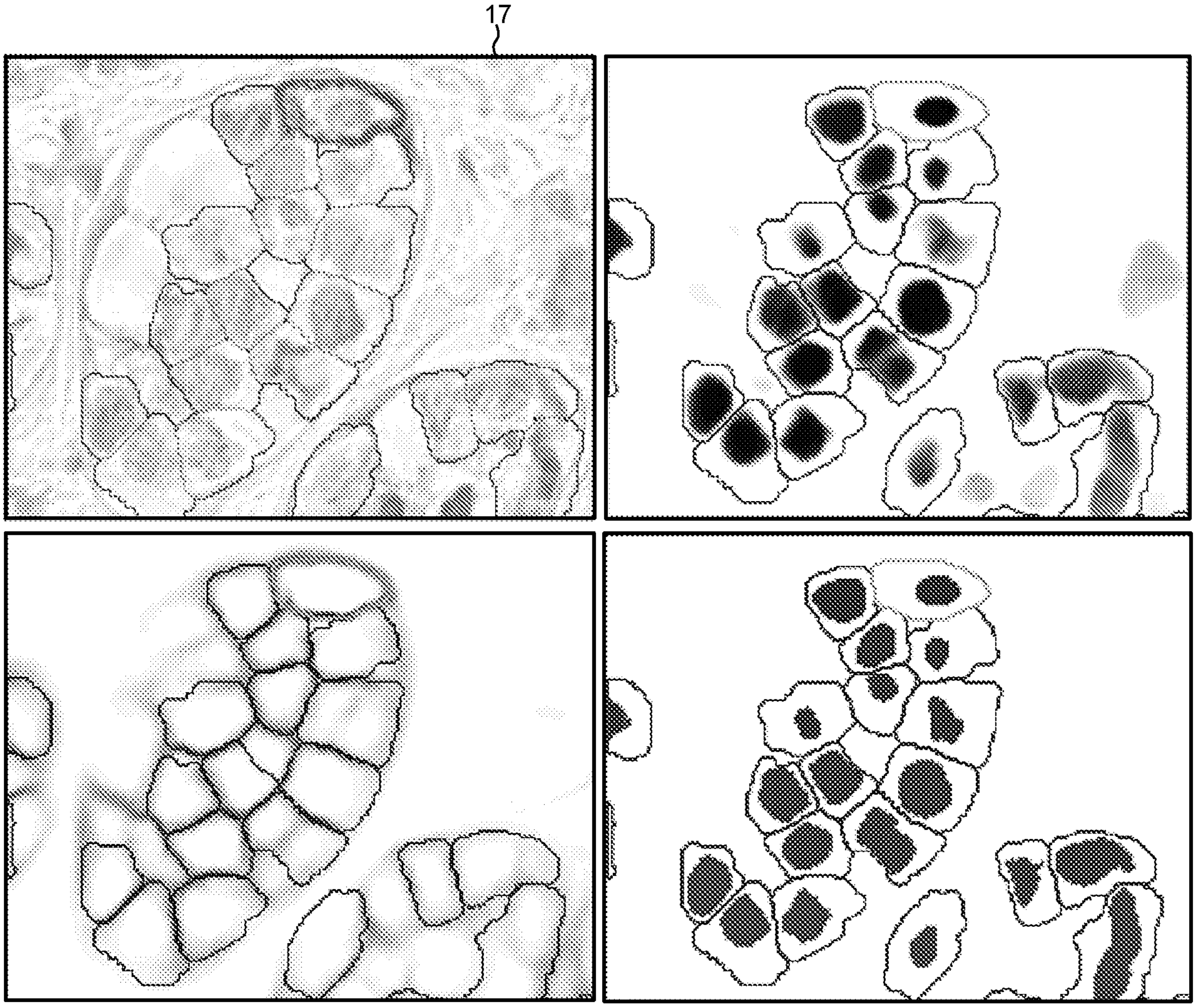


The unique identifier (UID) for each membrane object is the same as that of the associated nucleus object.

FIG. 18

Cytoplasm and membrane segmentation results by identification of cell border pixels as membrane, and then region growing of the membrane into pixels with membrane posterior >20%.

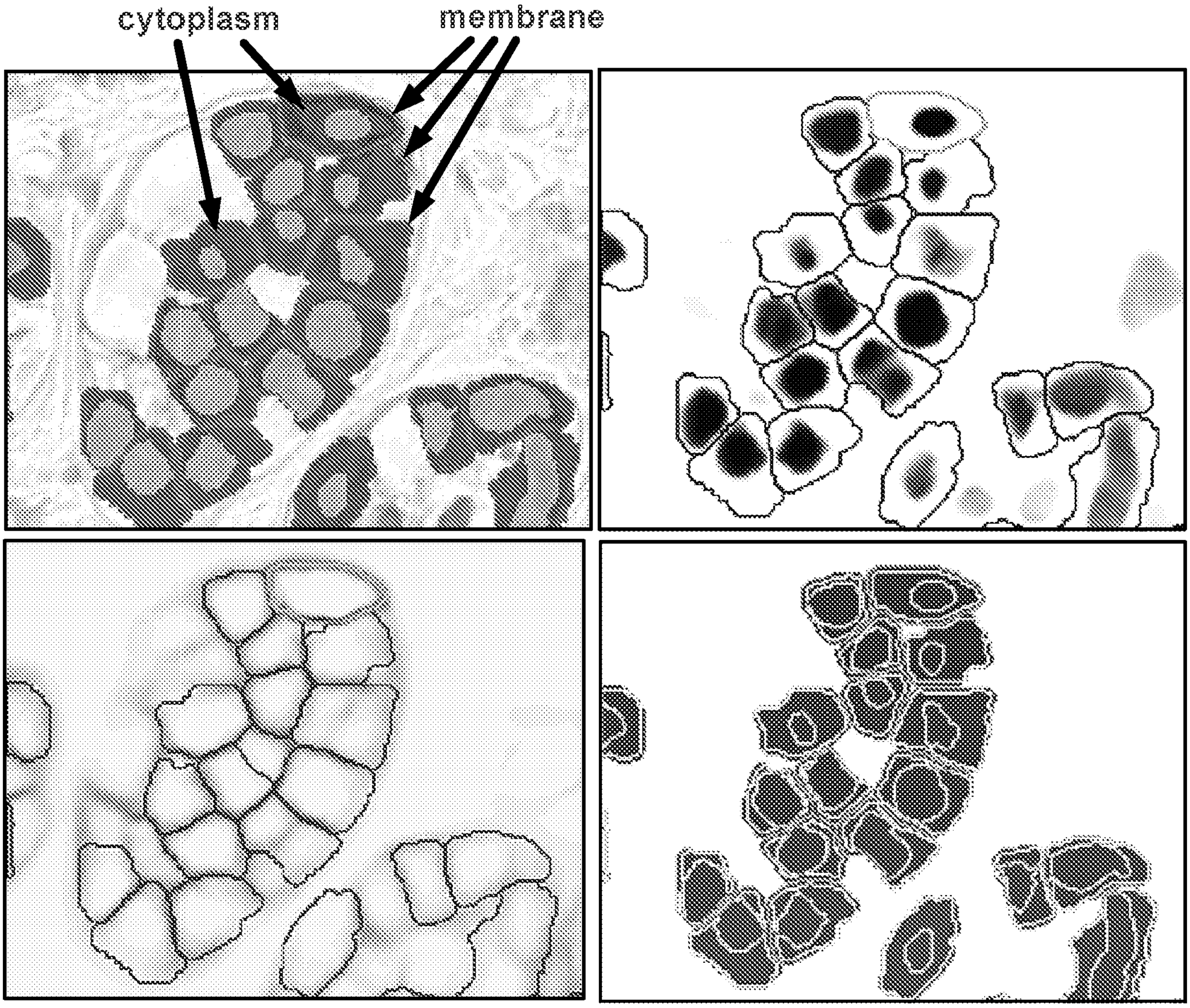


Unique Identifiers for cells at the nuclei, cytoplasm and membrane pixels

FIG. 19

17

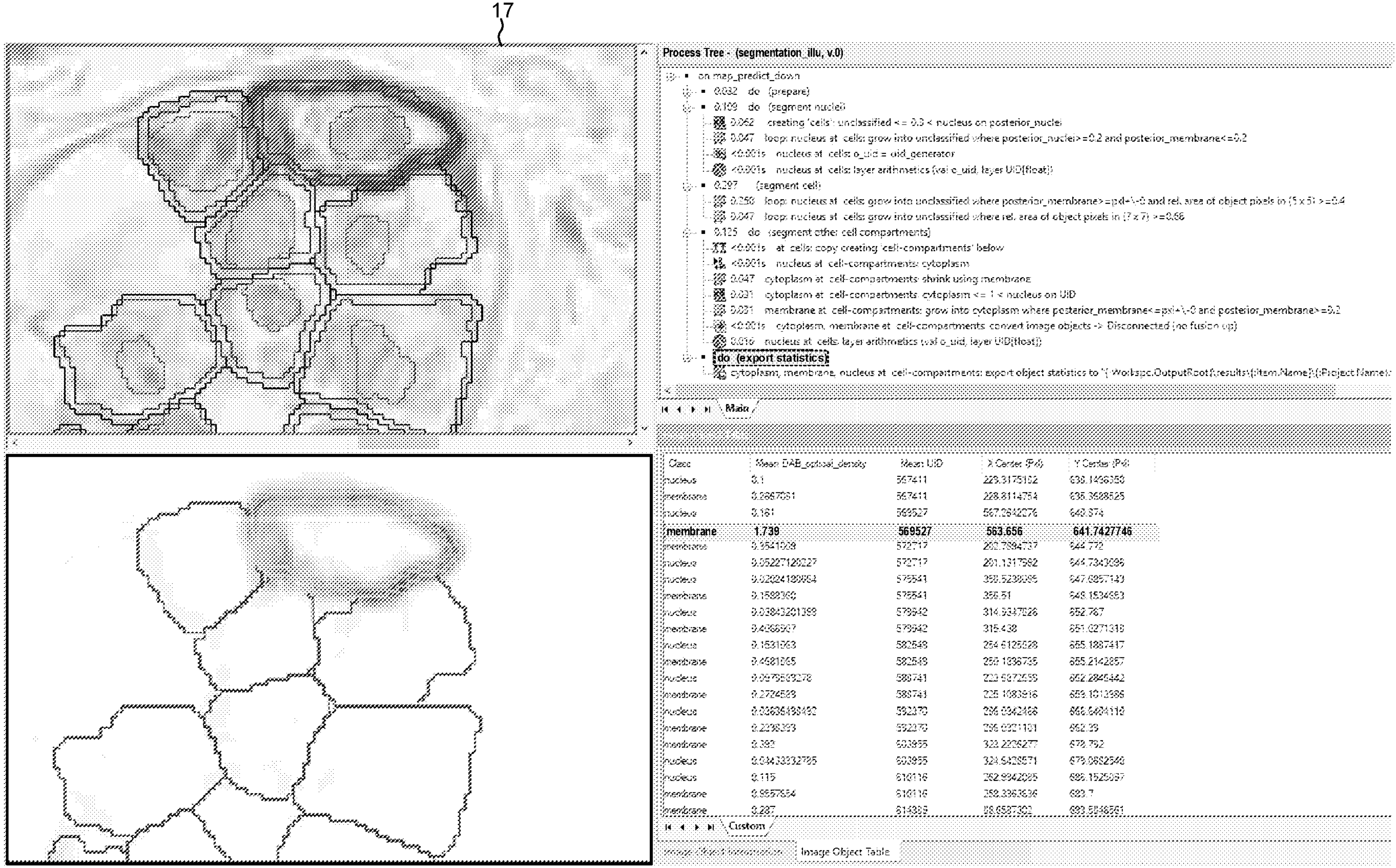


In the lower image, dark corresponds to high optical density, and bright corresponds to low optical density

FIG. 20

COGNITION NETWORK LANGUAGE SCRIPT IS USED TO SEGMENT CELLS INTO SEPARATE NUCLEUS, CYTOPLASM AND MEMBRANE.

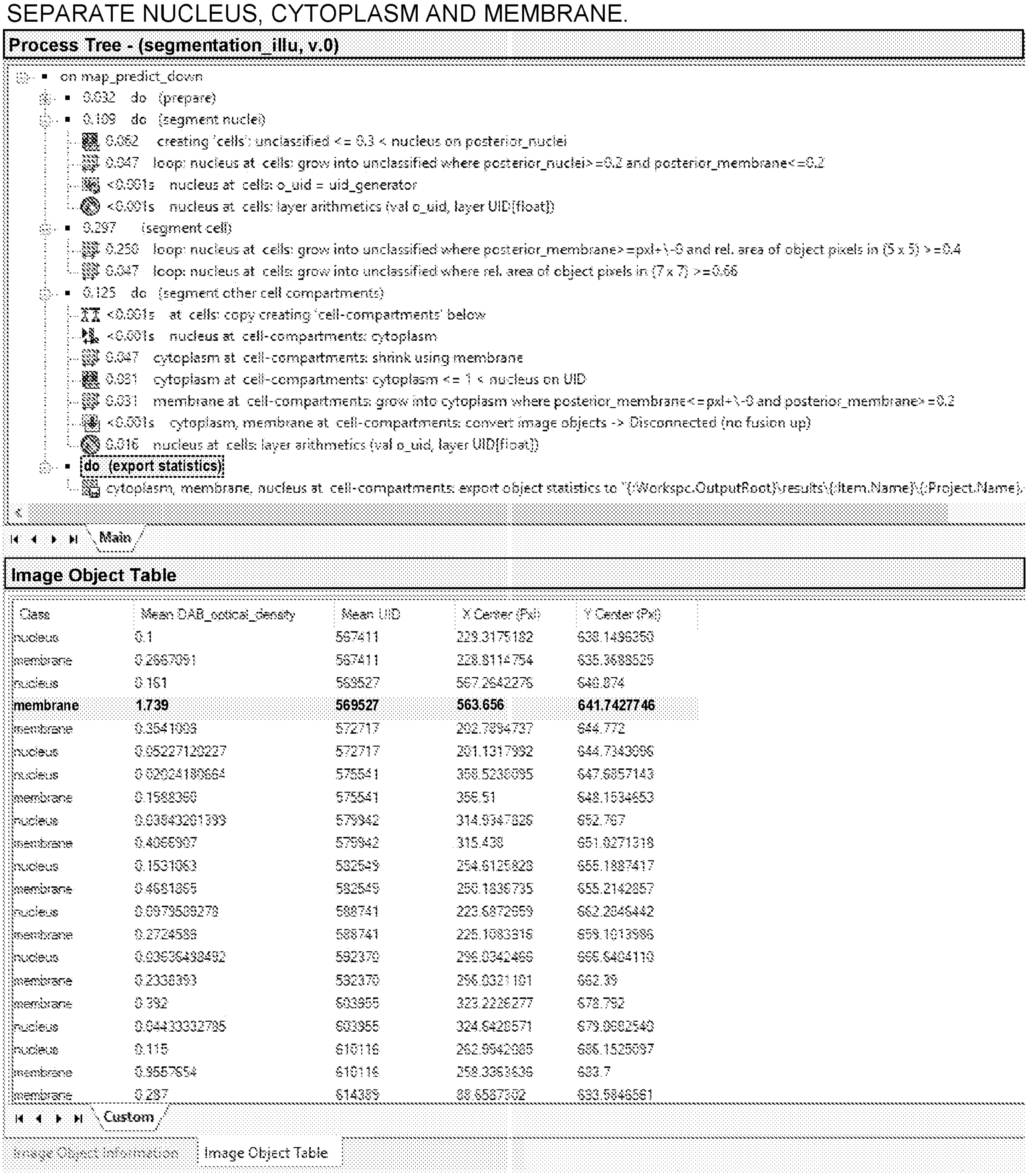


| Class | Mean DAB_optical_density | Mean UID | X Center (Pxl) | Y Center (Pxl) |
|---|---|---|---|---|
| nucleus | 0.1 | 567411 | 229.3175182 | 638.1496350 |
| membrane | 0.2667091 | 567411 | 228.8114754 | 635.3688525 |
| nucleus | 0.161 | 569527 | 567.2642276 | 640.874 |
| **membrane** | **1.739** | **569527** | **563.656** | **641.742746** |
| membrane | 0.3541008 | 572717 | 292.7894737 | 644.772 |
| nucleus | 0.05227120227 | 572717 | 291.1317992 | 644.7343096 |
| nucleus | 0.02024180664 | 575541 | 358.5238095 | 647.6857143 |
| membrane | 0.1588368 | 575541 | 356.51 | 648.1534653 |
| nucleus | 0.03843281393 | 579942 | 314.9347826 | 652.767 |
| membrane | 0.4066907 | 579942 | 315.438 | 651.0271318 |
| nucleus | 0.1531063 | 582549 | 254.6125828 | 655.1887417 |
| membrane | 0.4681865 | 582549 | 250.1836735 | 655.2142857 |
| nucleus | 0.09795089279 | 588741 | 223.6872659 | 662.2846442 |
| membrane | 0.2724589 | 588741 | 225.1083916 | 659.1013986 |
| nucleus | 0.03636492492 | 592370 | 296.0342466 | 665.6404110 |
| membrane | 0.2338393 | 592370 | 296.0321101 | 662.39 |
| membrane | 0.392 | 603955 | 323.2226277 | 678.792 |
| nucleus | 0.04433332795 | 603955 | 324.6428571 | 679.0682540 |
| nucleus | 0.115 | 610116 | 262.9542085 | 685.1525097 |
| membrane | 0.9557654 | 610116 | 258.3363636 | 683.7 |
| membrane | 0.287 | 614369 | 88.6587302 | 693.5846561 |

THE STATISTICS ARE TABULATED OF THE DAB OPTICAL DENSITY FOR ALL MEMBRANES AND CYTOPLASMS IN THE IMAGE .

FIG. 21

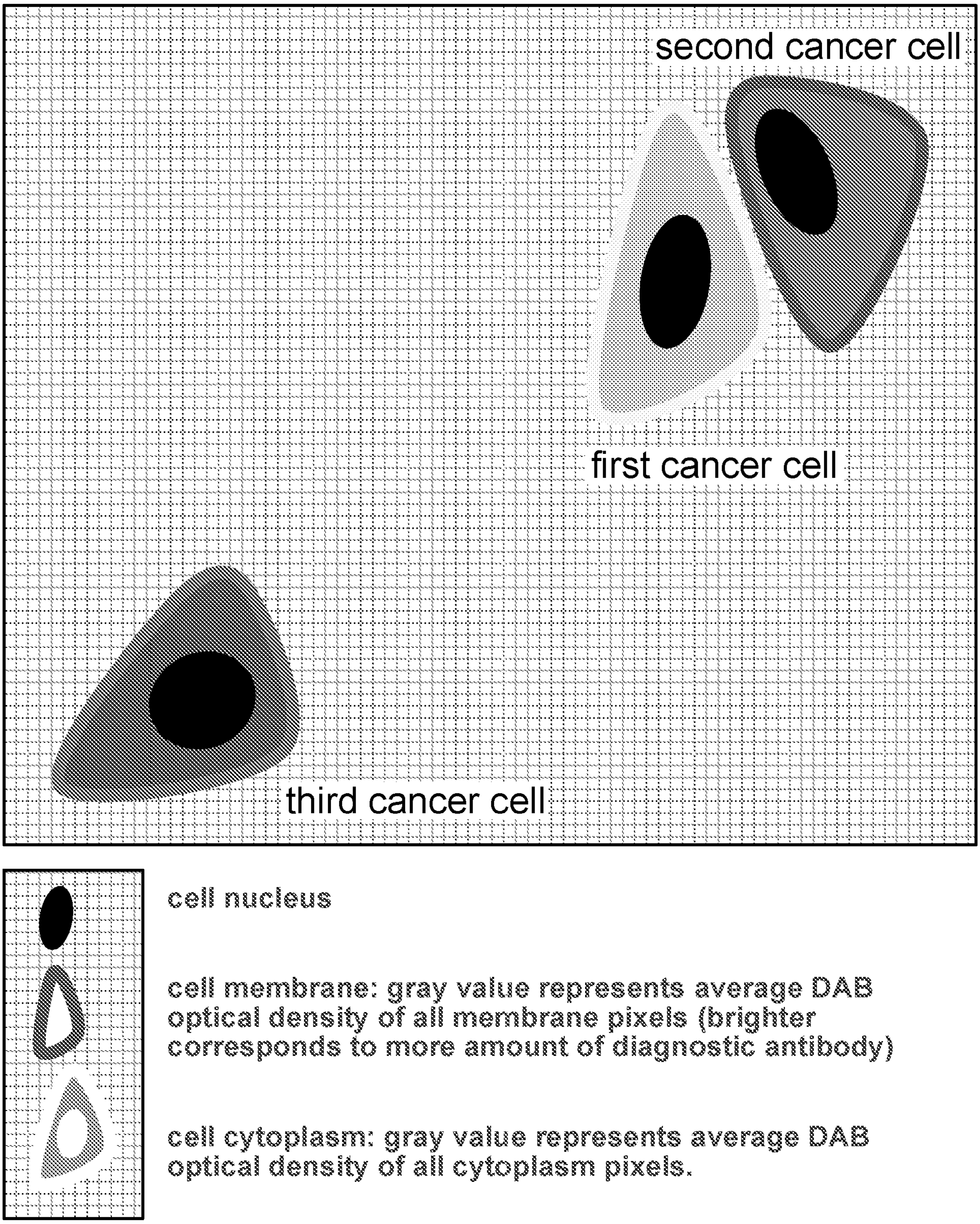
second cancer cell
first cancer cell
third cancer cell
cell nucleus
cell membrane: gray value represents average DAB optical density of all membrane pixels (brighter corresponds to more amount of diagnostic antibody)
cell cytoplasm: gray value represents average DAB optical density of all cytoplasm pixels.

FIG. 22

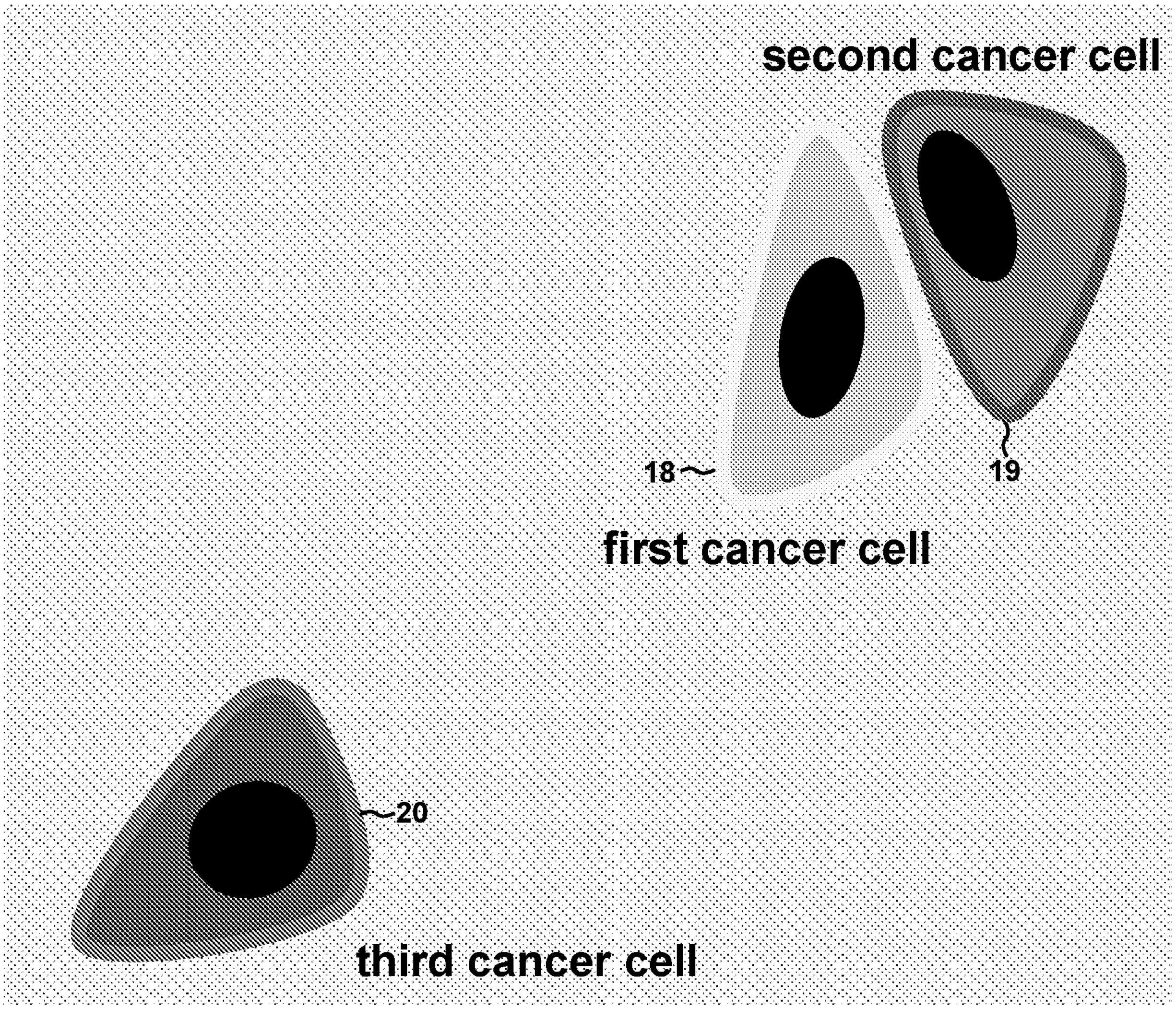


| UID | X [pixels] | Y [pixels] | Optical density membrane (ODM) | Optical density cytoplasm (ODC) |
|---|---|---|---|---|
| 1 | 480 | 190 | 242/255 = .949 | 203/255 = .796 |
| 2 | 590 | 130 | 94/255 = .369 | 136/255 = .533 |
| 3 | 140 | 500 | 127/255 = .498 | 94/255 = .369 |

FIG. 23

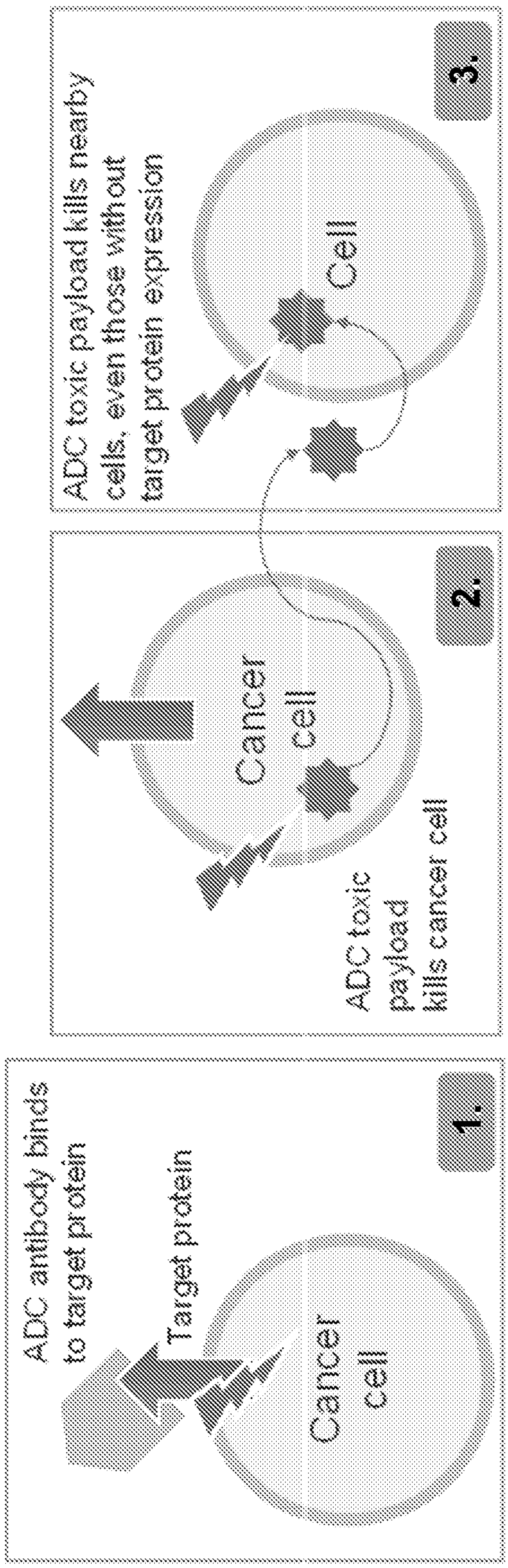
1.
ADC antibody binds to target protein
Target protein
Cancer cell
2.
ADC toxic payload kills cancer cell
Cancer cell
3.
ADC toxic payload kills nearby cells, even those without target protein expression
Cell

FIG. 24

## Cell statistics with single cell ADC scoring

r_1 – r_i : euclidean distance from center of gravity of the cell with UID 1 to the cell with UID i.

w_ij = exp ( - |r_i – r_j | / r_norm ) : exponential weighting factor to include ADC payload uptake from neighboring cells

Single cell ADC score for cell i = min (K1 , Sum_j with w_ij < 15% { w_ij x max ( 0, ODM_j x (K2 + ODM_j – ODC_j )) } )

| UID | X [pixels] | Y [pixels] | Optical density membrane (ODM) | Optical density cytoplasm (ODC) | max(0, ODM x(ODM-ODC)) | r_1-r_i | r_2-r_i | r_3-r_i | exp(-(r_1-r_i)/50) | exp(-(r_2-r_i)/50) | exp(-(r_3-r_i)/50) | Single-Cell ADC Uptake |
|---|---|---|---|---|---|---|---|---|---|---|---|---|
| 1 | 480 | 190 | 0.949019608 | 0.796078431 | 0.145144175 | 0 | 125.2996 | 460.1087 | 1 | 0.08159455 | 0.00010082 | 0.145144175 |
| 2 | 590 | 130 | 0.368627451 | 0.533333333 | 0 | 125.2996 | 0 | 582.5805 | 0.08159455 | 1 | 8.705E-06 | 0.011842974 |
| 3 | 140 | 500 | 0.498039216 | 0.368627451 | 0.064452134 | 460.1087 | 582.5805 | 0 | 0.00010082 | 8.705E-06 | 1 | 0.064452134 |

r_norm=50

FIG. 25

GENERATING A SCORE FOR EACH CELL WHICH REFLECTS THE LIKELIHOOD THAT A CANCER CELL IS DAMAGED IN FUNCTION OR KILLED

Single Cell ADC score = Weighted sum of this cell and neighboring cells of a bilinear combination of the amounts of Target Protein on the cells membrane and cytoplasm, the weights depend on the distance of the neighboring cells to this cell.

Mathematics for cell i with position $r_i$ on the slide and average membrane (cytoplasm) optical density values $ODM_i$ ($ODC_i$)

$$single_cell_score_i = Sum_j \text{ with } |r_j - r_i|<d \{ a_{20}(|r_j - r_i|) \times ODM_j^2 + a_{11}(|r_j - r_i|) \times ODM_j \times ODC_j + a_{02}(|r_j - r_i|) \times ODC_j^2 + a_{00}(|r_j - r_i|) \}$$

with the functions $a_{kl}$ depending on the distance $|r_j - r_i|$ of the cell j to the cell i, e.g., $a_{kl}(|r_j - r_i|) = A_{kl} \times \exp(- |r_j - r_i|/r_{norm})$ with $A_{kl}$ and $r_0$ constants determined by optimized therapy success prediction.

FIG. 26

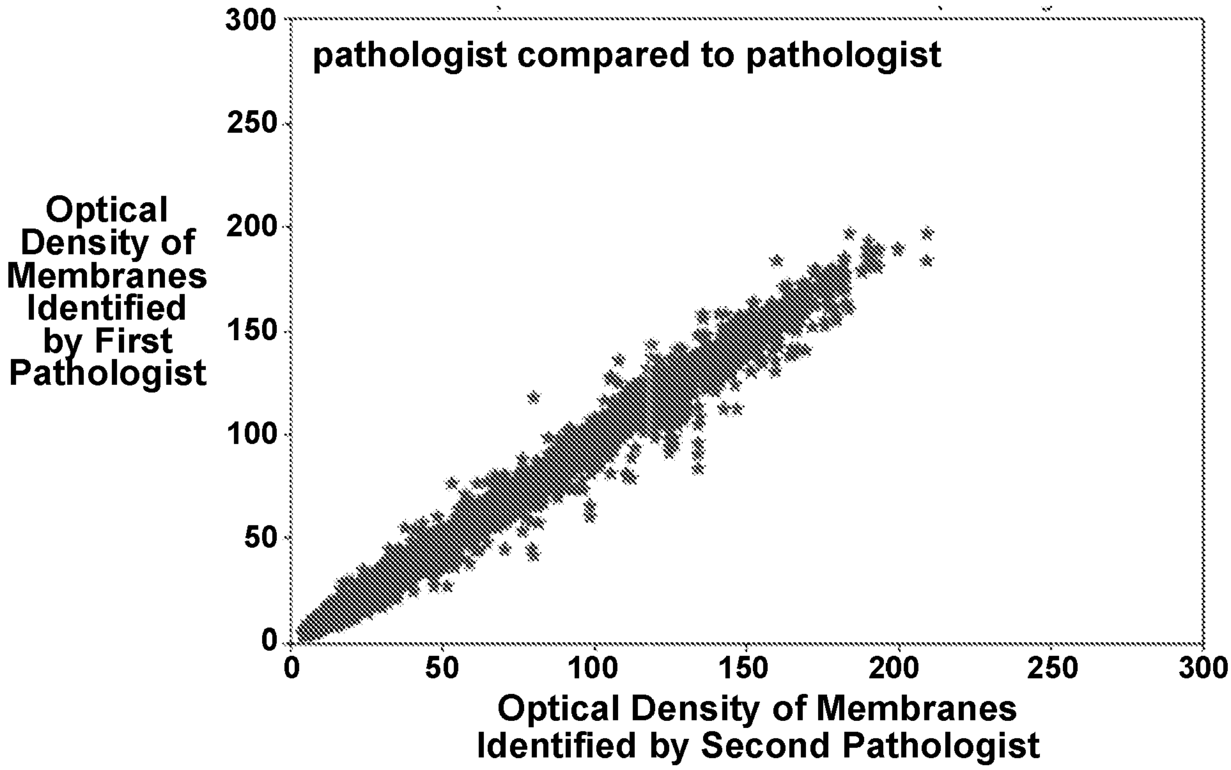
pathologist compared to pathologist
Optical Density of Membranes Identified by First Pathologist
Optical Density of Membranes Identified by Second Pathologist
0
50
100
150
200
250
300

FIG. 27

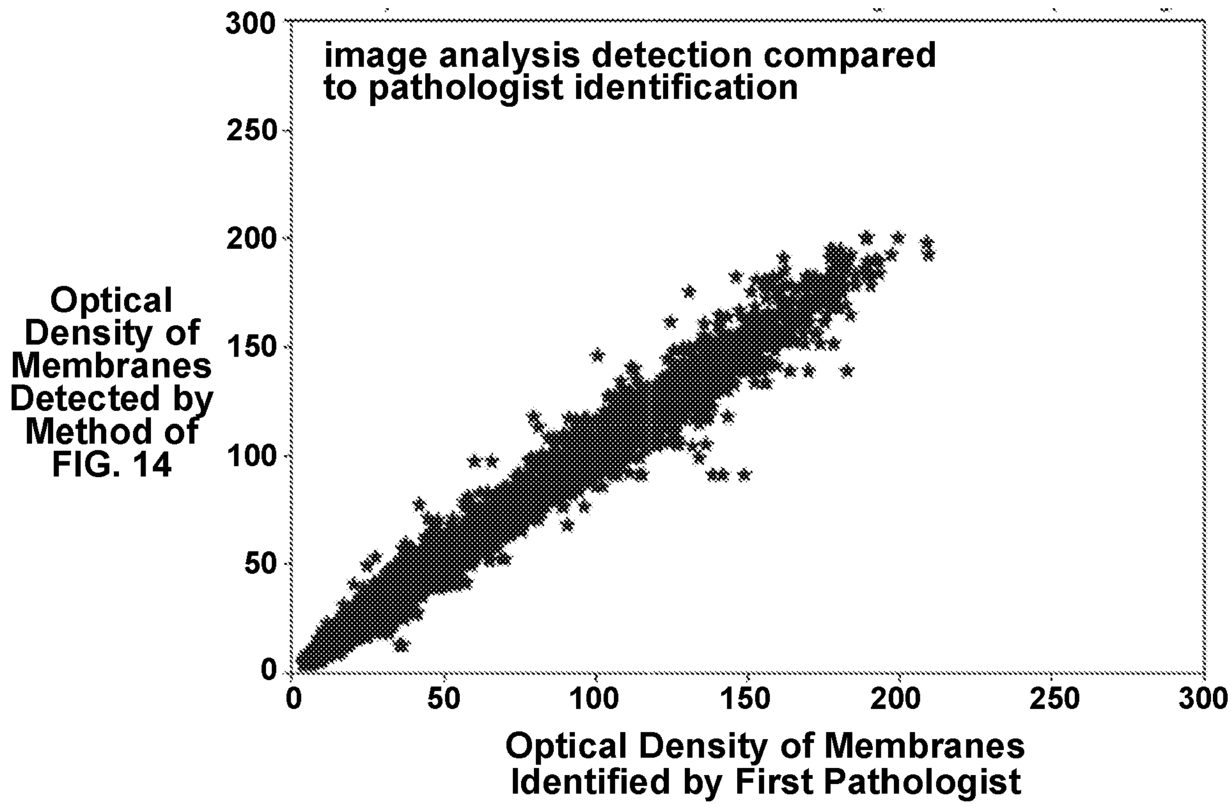
image analysis detection compared to pathologist identification
Optical Density of Membranes Detected by Method of FIG. 14
Optical Density of Membranes Identified by First Pathologist
0
50
100
150
200
250
300

FIG. 28

HER2-negative patients from J101 trial

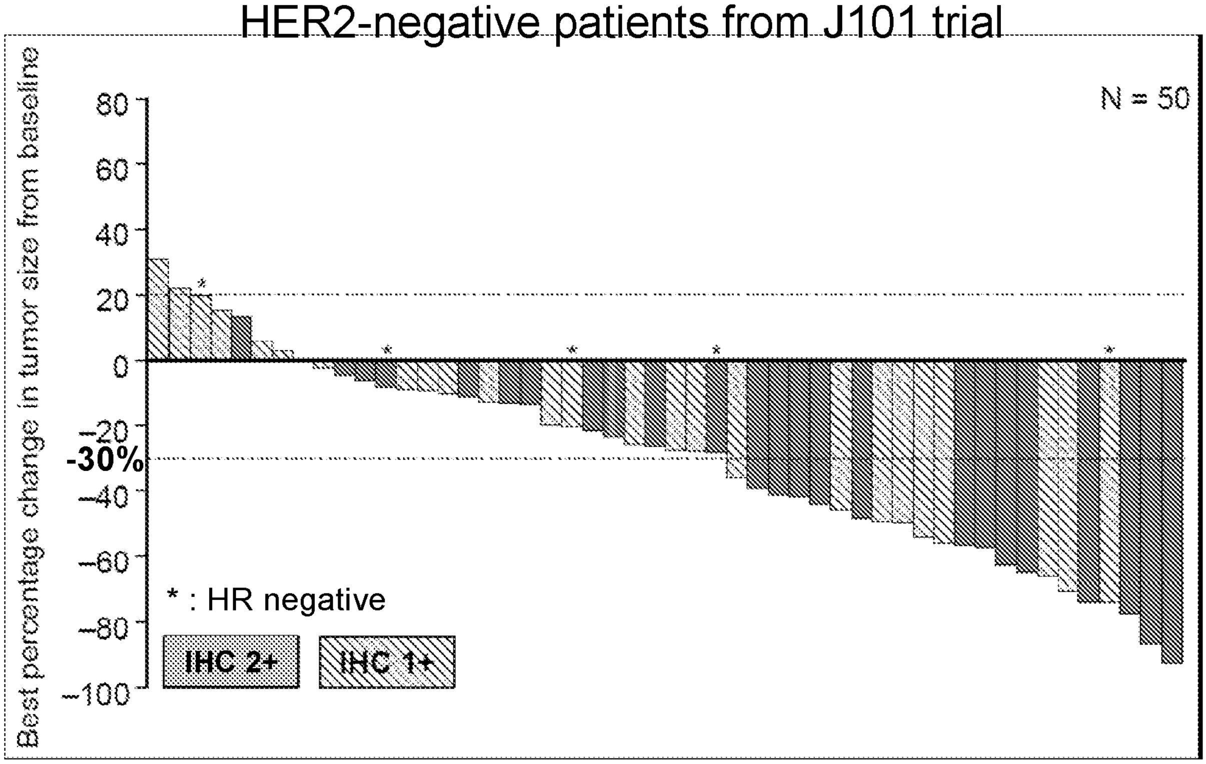


FIG. 29

HER2-positive patients from DESTINY BREAST 01 trial

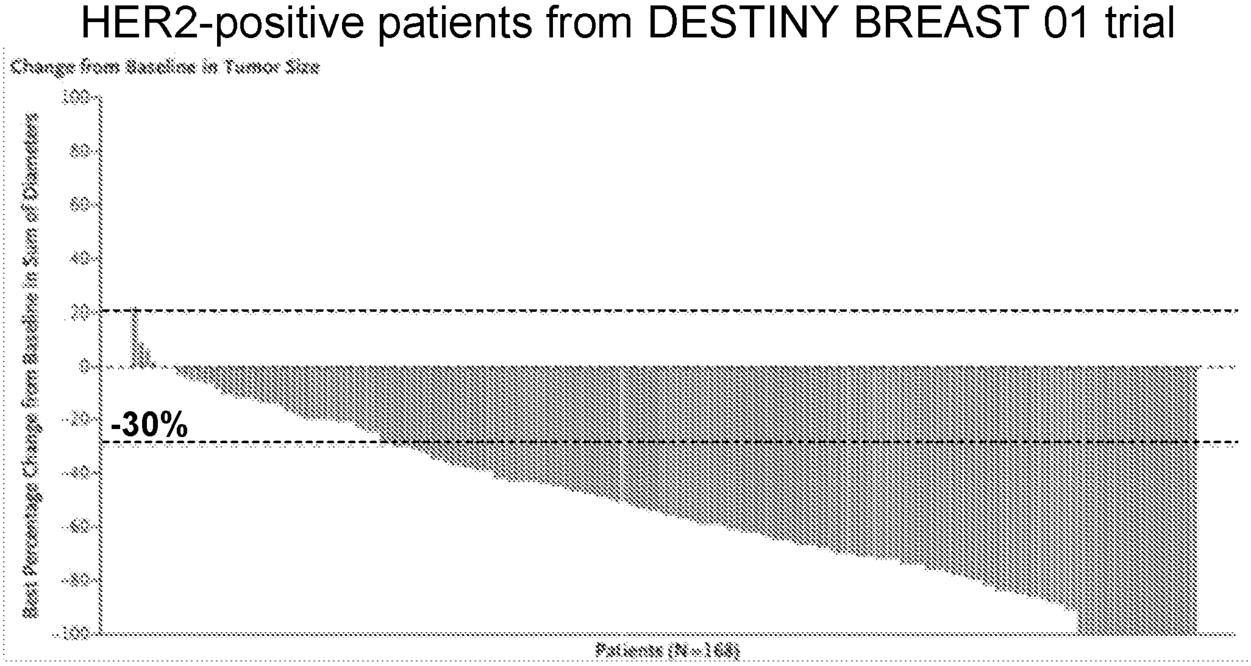


FIG. 30

## Conventional HER2 IHC scoring

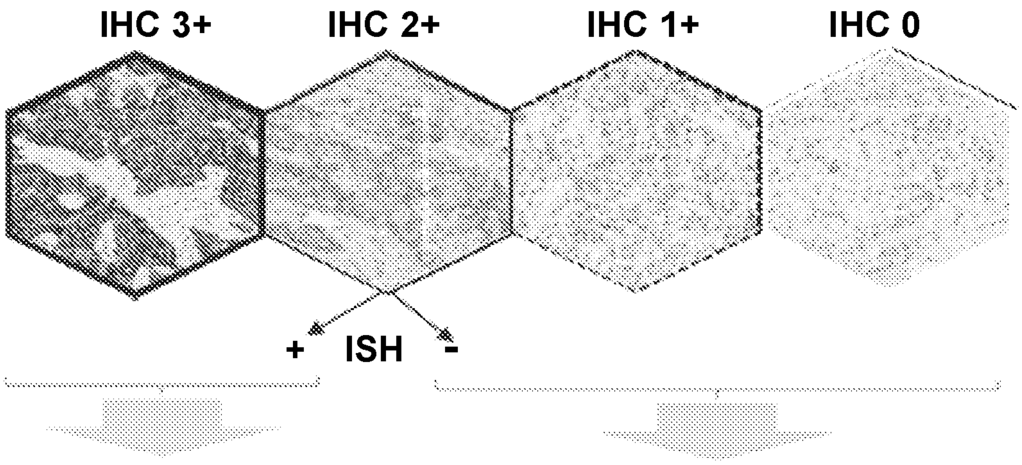


| | HER2 Positive (IHC 3+ & 2+ ISH+) | HER2 Negative (IHC 2+/ISH-, 1+, 0) |
|---|---|---|
| ORR | 56% | 42% |
| mPFS | 14.1 mos | 11.0 mos |
| n | 72 | 65 |

(N=137)

FIG. 31

| | QCS Positive (OD >cut-off) | QCS Negative (OD<cut-off) |
|---|---|---|
| ORR | 52% | 24% |
| mPFS | 14.5 mos | 8.6 mos |
| n | 40 | 25 |

FIG. 32

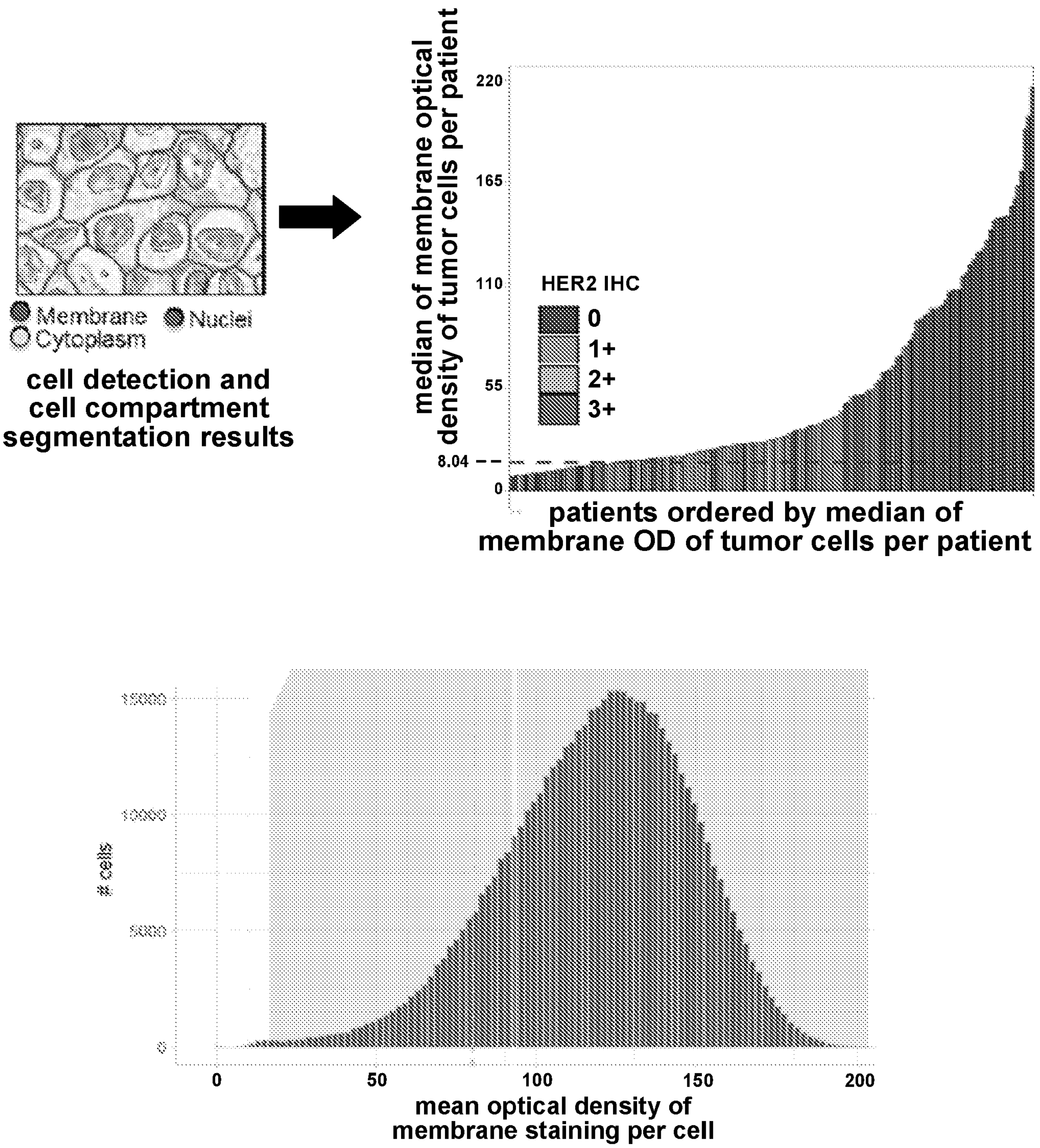
Membrane
Nuclei
Cytoplasm
cell detection and cell compartment segmentation results
median of membrane optical density of tumor cells per patient
220
165
110
55
8.04
0
HER2 IHC
0
1+
2+
3+
patients ordered by median of membrane OD of tumor cells per patient
15000
10000
5000
0
# cells
0
50
100
150
200
mean optical density of membrane staining per cell

FIG. 33

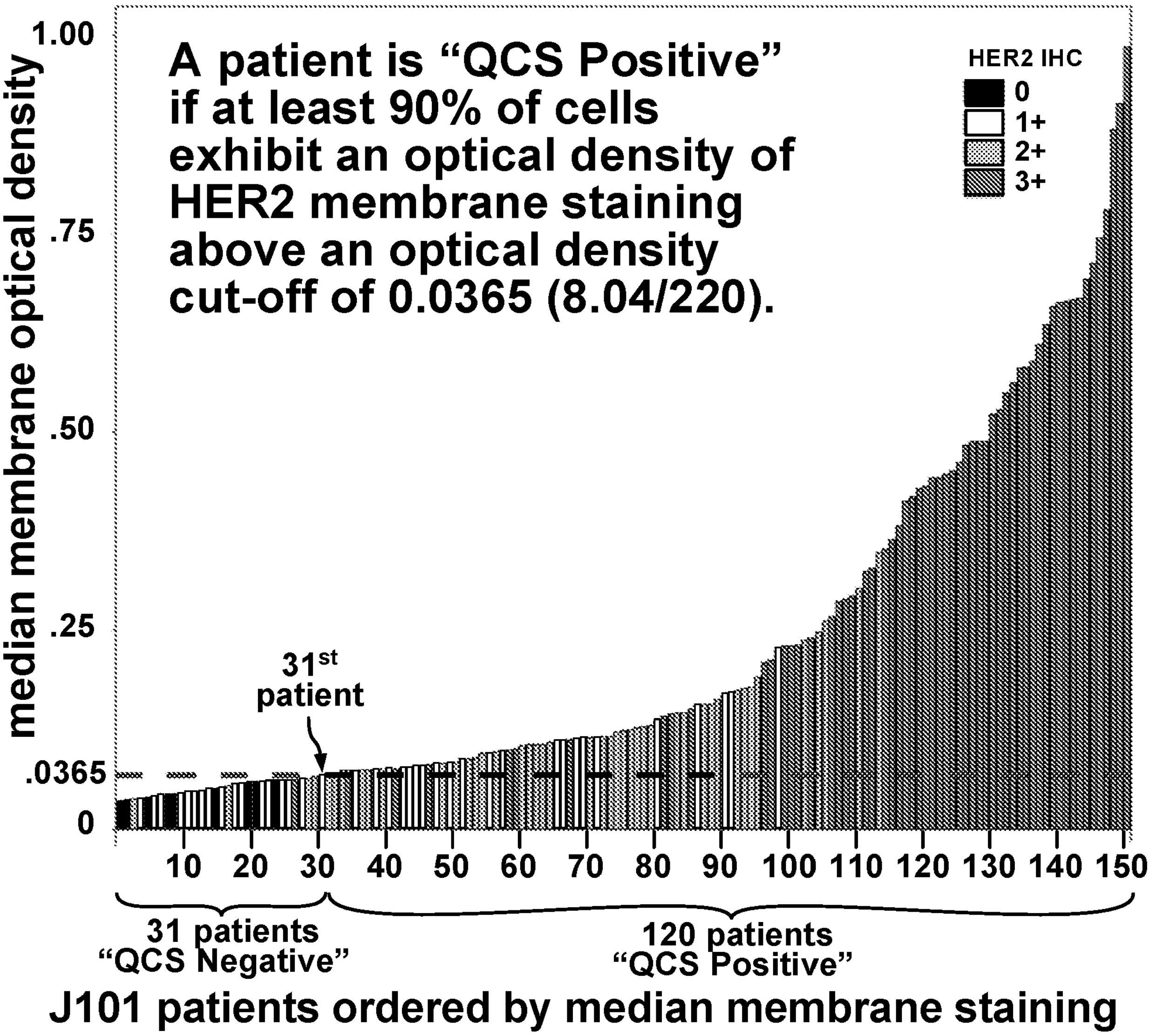


FIG. 34A

| | optical density less than cut-off QCS Positive | optical density greater than cut-off QCS Negative |
|---|---|---|
| ORR | 56% | 26% |
| mPFS | 14.1 mos | 9 mos |
| n | 120 | 31 |

(N=151)

FIG. 34B

For 65 patients in HER2-negative cohort

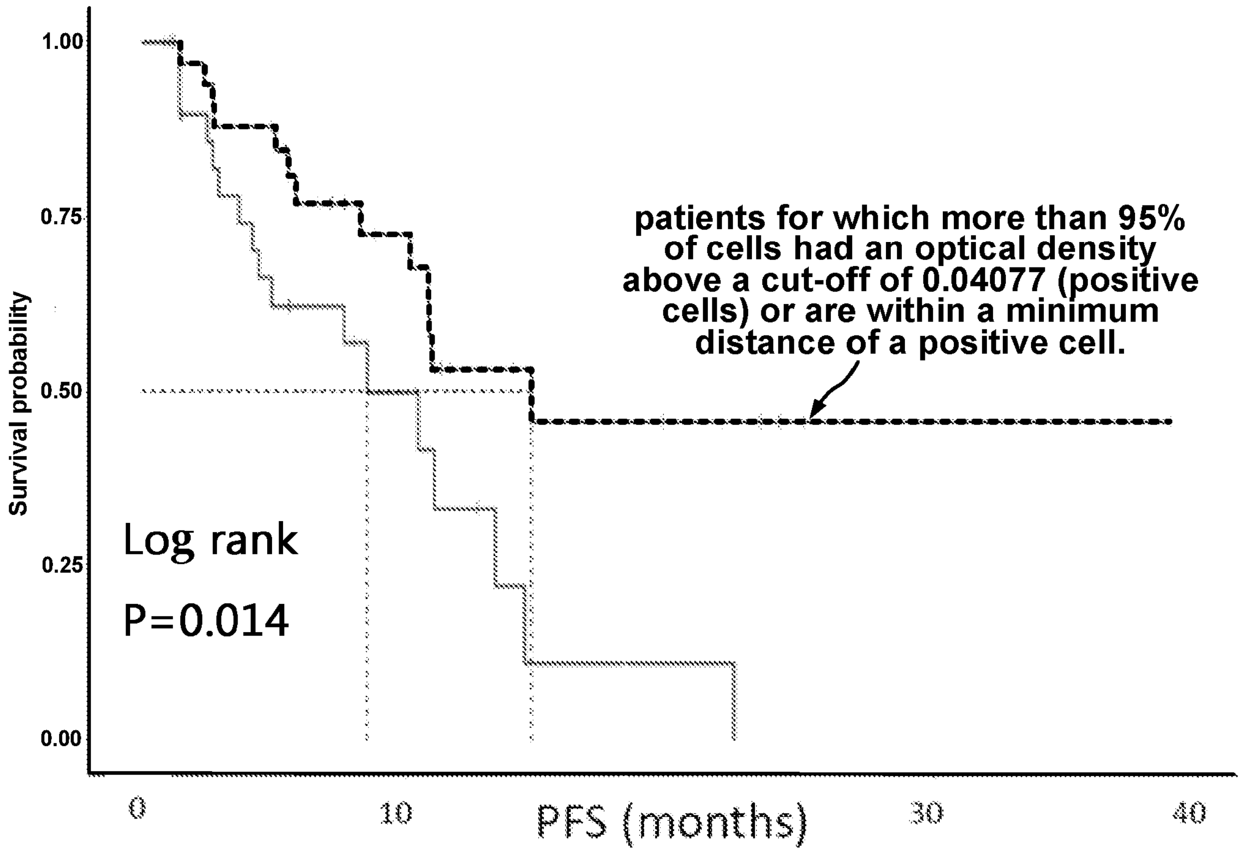


For patients corresponding to upper curve, 95% of cells either (1) exhibited an optical density of HER2 staining above a minimum threshold, or (2) are within a minimum distance of a cell exhibiting the minimal staining.

FIG. 35

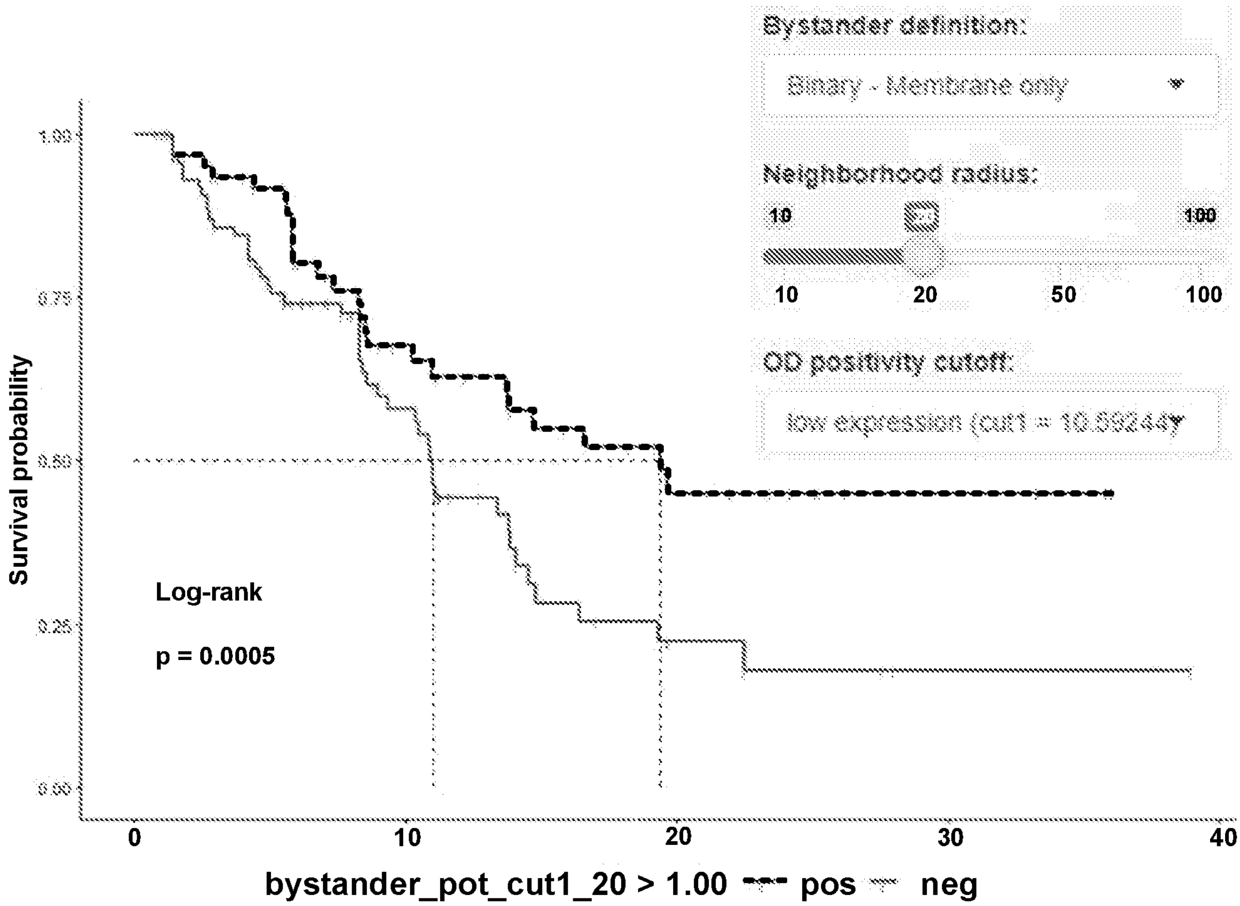
Bystander definition:
Binary - Membrane only
Neighborhood radius:
10
20
100
10
20
50
100
OD positivity cutoff:
low expression (cut1 = 10.59244)
1.00
0.75
0.50
0.25
0.00
Survival probability
Log-rank
p = 0.0005
0
10
20
30
40
bystander_pot_cut1_20 > 1.00
pos
neg

FIG. 36

**HER2+**: IHC 3+ or IHC 2+/ISH+

**HER2-**: IHC 0 or IHC 1+ or ICH2+/ISH-

Show 10 entries

Search:

| Rank | Feature | Cutoff | N pos | N neg | ORR pos | ORR neg | HR | HR p-value | log-rank p-value | mPFS pos | mPFS neg |
|---|---|---|---|---|---|---|---|---|---|---|---|
| 1 | membrane.perc.pos_cut_5 | 0.9987 | [illegible] | [illegible] | 0.63 | [illegible] | [illegible] | [illegible] | [illegible] | [illegible] | [illegible] |
| 2 | cell.perc.pos_cut_5 | 0.9984 | [illegible] | [illegible] | 0.63 | [illegible] | [illegible] | [illegible] | [illegible] | [illegible] | [illegible] |
| 3 | bystander_pot_cut2_100 | 0.9998 | [illegible] | [illegible] | 0.61 | [illegible] | [illegible] | [illegible] | [illegible] | [illegible] | [illegible] |
| 4 | bystander_pot_cut1_50 | 0.9998 | [illegible] | [illegible] | 0.61 | [illegible] | [illegible] | [illegible] | [illegible] | [illegible] | [illegible] |
| 5 | bystander_pot_cut2_50 | 0.9990 | [illegible] | [illegible] | 0.61 | [illegible] | [illegible] | [illegible] | [illegible] | [illegible] | [illegible] |
| 6 | mem_cyto.OD_diff_1_q15 | 2.2731 | [illegible] | [illegible] | 0.59 | [illegible] | [illegible] | [illegible] | [illegible] | [illegible] | [illegible] |
| 7 | bystander_pot_cut1_10 | 0.9896 | [illegible] | [illegible] | 0.59 | [illegible] | [illegible] | [illegible] | [illegible] | [illegible] | [illegible] |
| 8 | mem_cyto.OD_diff_3_q10 | 13.6300 | [illegible] | [illegible] | 0.59 | [illegible] | [illegible] | [illegible] | [illegible] | [illegible] | [illegible] |
| 9 | mem_cyto.OD_diff_4_q10 | 13.6300 | [illegible] | [illegible] | 0.59 | [illegible] | [illegible] | [illegible] | [illegible] | [illegible] | [illegible] |

| Rank | Feature | Cutoff | N pos | N neg | ORR pos | ORR neg | HR | HR p-value | log-rank p-value | mPFS pos | mPFS neg |
|---|---|---|---|---|---|---|---|---|---|---|---|
| 1 | TIL_HER2_CONTINEOUS | 271.8987 | [illegible] | [illegible] | [illegible] | [illegible] | [illegible] | [illegible] | 0.0000 | [illegible] | [illegible] |
| 2 | density_TILs_TC | 388.7609 | [illegible] | [illegible] | [illegible] | [illegible] | [illegible] | [illegible] | 0.0001 | [illegible] | [illegible] |
| 3 | density_sTILs_rel_to_TC | 273.0258 | [illegible] | [illegible] | [illegible] | [illegible] | [illegible] | [illegible] | 0.0001 | [illegible] | [illegible] |
| 4 | membrane.OD_q5 | 6.9696 | [illegible] | [illegible] | [illegible] | [illegible] | [illegible] | [illegible] | 0.0002 | [illegible] | [illegible] |
| 5 | bystander_1_20_q5 | 9.0662 | [illegible] | [illegible] | [illegible] | [illegible] | [illegible] | [illegible] | 0.0005 | [illegible] | [illegible] |
| 6 | bystander_2_20_q5 | 6.5920 | [illegible] | [illegible] | [illegible] | [illegible] | [illegible] | [illegible] | 0.0005 | [illegible] | [illegible] |
| 7 | bystander_pot_cut1_20 | 0.9860 | [illegible] | [illegible] | [illegible] | [illegible] | [illegible] | [illegible] | 0.0005 | [illegible] | [illegible] |
| 8 | cell.OD_q35 | 10.1557 | [illegible] | [illegible] | [illegible] | [illegible] | [illegible] | [illegible] | 0.0005 | [illegible] | [illegible] |
| 9 | cell.OD_q10 | 6.8046 | [illegible] | [illegible] | [illegible] | [illegible] | [illegible] | [illegible] | 0.0006 | [illegible] | [illegible] |
| 10 | cell.OD_q15 | 7.4176 | [illegible] | [illegible] | [illegible] | [illegible] | [illegible] | [illegible] | 0.0006 | [illegible] | [illegible] |

FIG. 37

| | feature | feature parameter | cutoff in model | log-rank p-value | CM group | objective response rate |
|---|---|---|---|---|---|---|
| A | TIL density | stromal TILs in tumor core | 168 | 0.007 | high | 49.2 |
| | | | | | low | 50.2 |
| B | HER2+ cell density | optical density cutoff: 15 density in tumor core | 1672 | 0.011 | high | 60.7 |
| | | | | | low | 40.7 |
| C | HER2+ neighborhood score | optical density in radius 25 µm | 37 | 0.014 | high | 57.3 |
| | | | | | low | 38.9 |

FIG. 38

# For 151 breast cancer patients in J101 trial

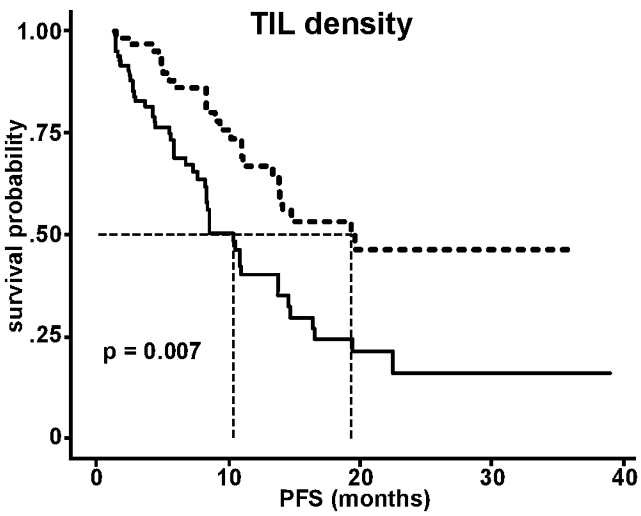


FIG. 39A

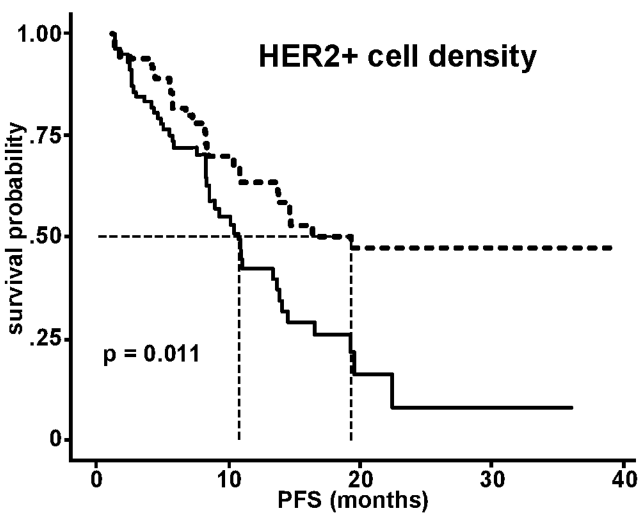


FIG. 39B

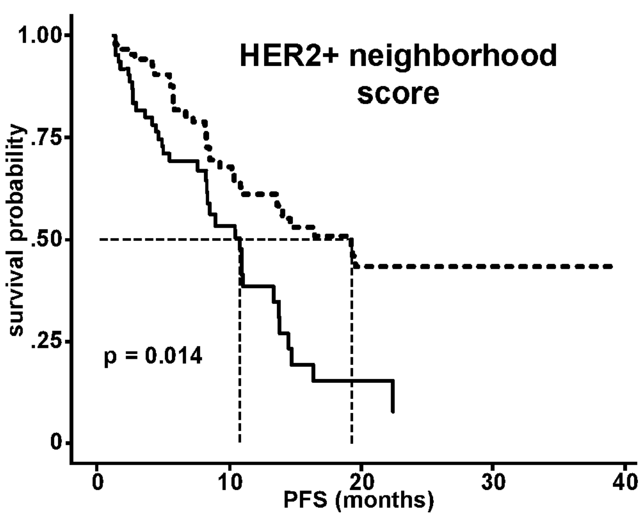


FIG. 39C

# For 72 patients in HER2-positive cohort

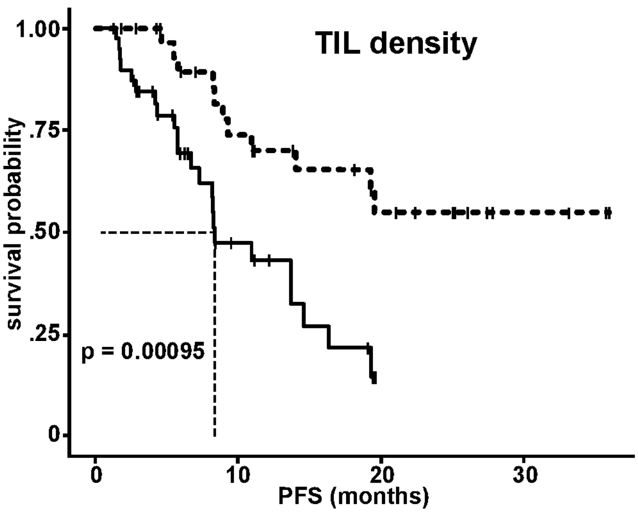


FIG. 40A

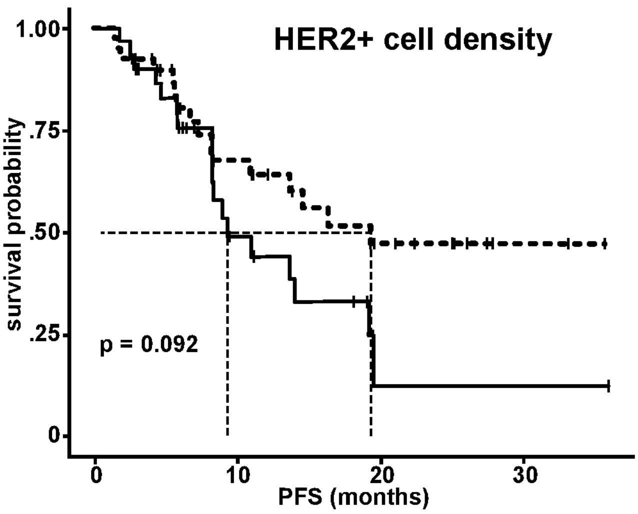


FIG. 40B

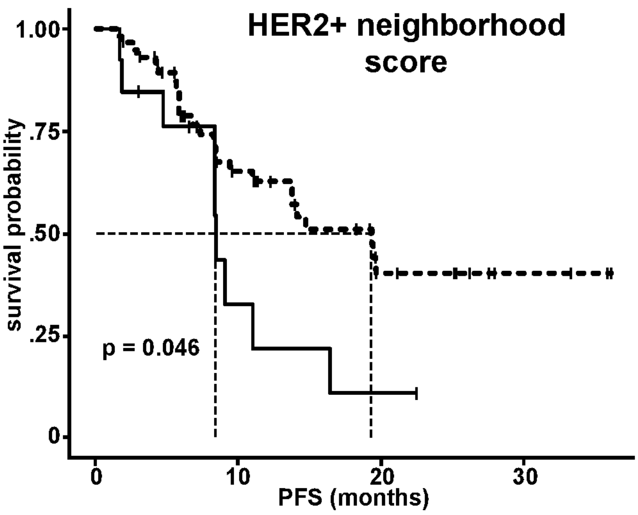


FIG. 40C

# For 65 patients in HER2-negative cohort

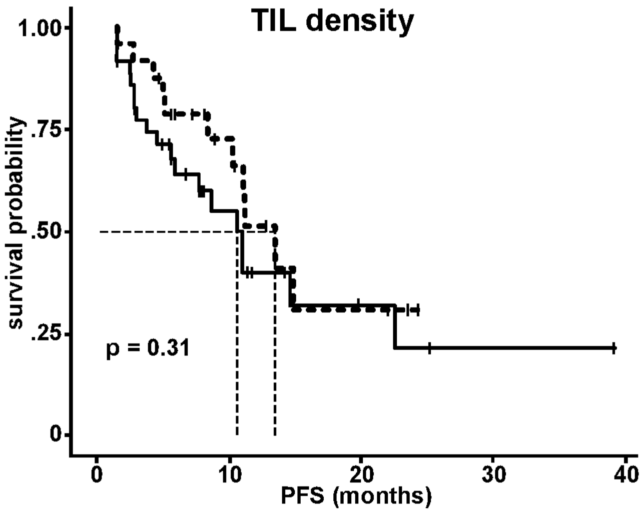


FIG. 41A

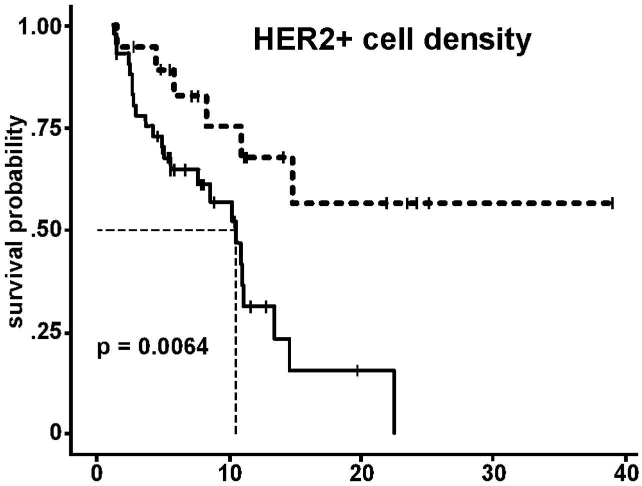


FIG. 41B

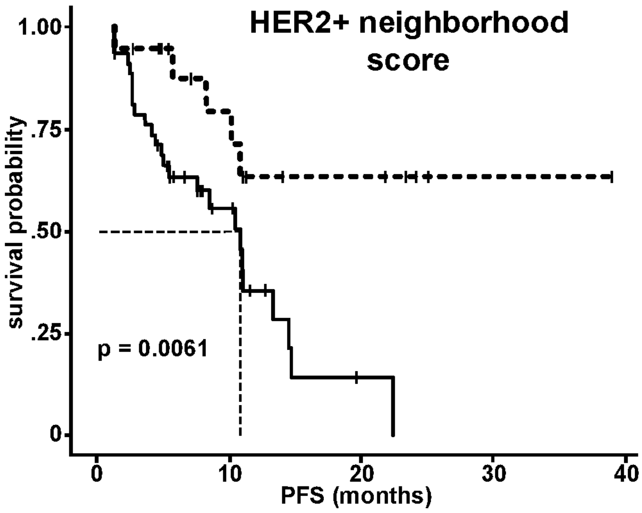


FIG. 41C

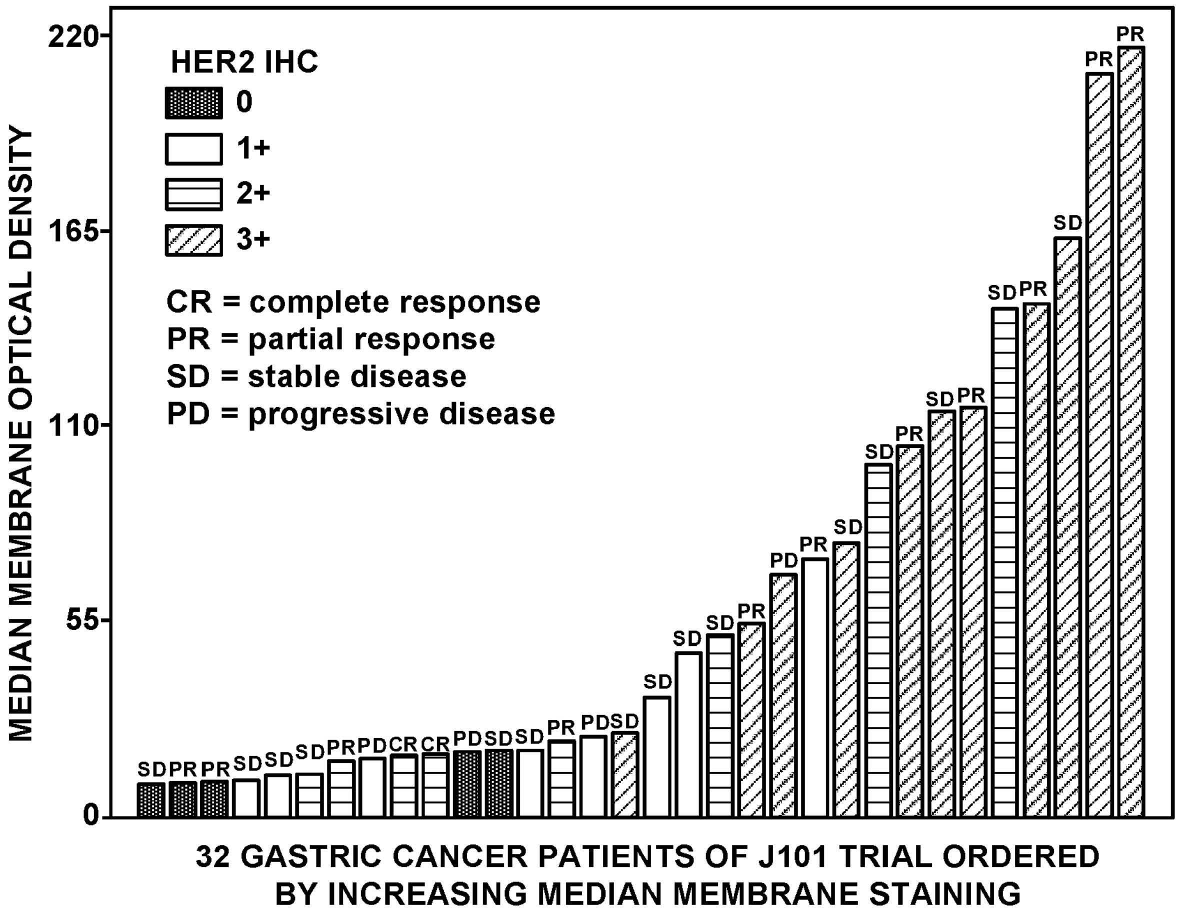


FIG. 42

| | feature | feature parameter | cutoff in model | log-rank p-value | PPV/ORR |
|---|---|---|---|---|---|
| 1 | membOD_q75 | optical density quantile | >=12.21 | 0.04 | 0.45 |
| 2 | membOD_Perc_pos_ 12 | Perc Pos Cells | >=26.17 | 0.048 | 0.45 |
| 3 | membOD_density_10 | Density Epi cells | >=810 | 0.006 | 0.47 |
| 4 | R25_cut15 | Bystander binary | >=35 | 0.04 | 0.45 |
| 5 | R75_mean | Bystander continuous | >=254 | 0.02 | 0.44 |
| 6 | Kurtosis | Histogram | <=41.45 | 0.03 | 0.45 |

FIG. 43

| | feature | feature parameter | cutoff in model | log-rank p-value | PPV/ORR |
|---|---|---|---|---|---|
| 1 | membOD_q75 | optical density quantile | >=12.21 | 0.02 | 0.45 |
| 2 | membOD_Perc_pos_12 | Perc Pos Cells | >=26.17 | 0.02 | 0.45 |
| 3 | membOD_density_10 | Density Epi cells | >=810 | 0.007 | 0.47 |
| 4 | R25_cut8 | Bystander binary | >=80 | 0.004 | 0.44 |
| 5 | R25_q90 | Bystander continuous | >=70.8 | 0.02 | 0.40 |
| 6 | Kurtosis | Histogram | <=37.88 | 0.0002 | 0.47 |
| 7 | Skewness | Histogram | <=6.03 | 0.0002 | 0.47 |

FIG. 44

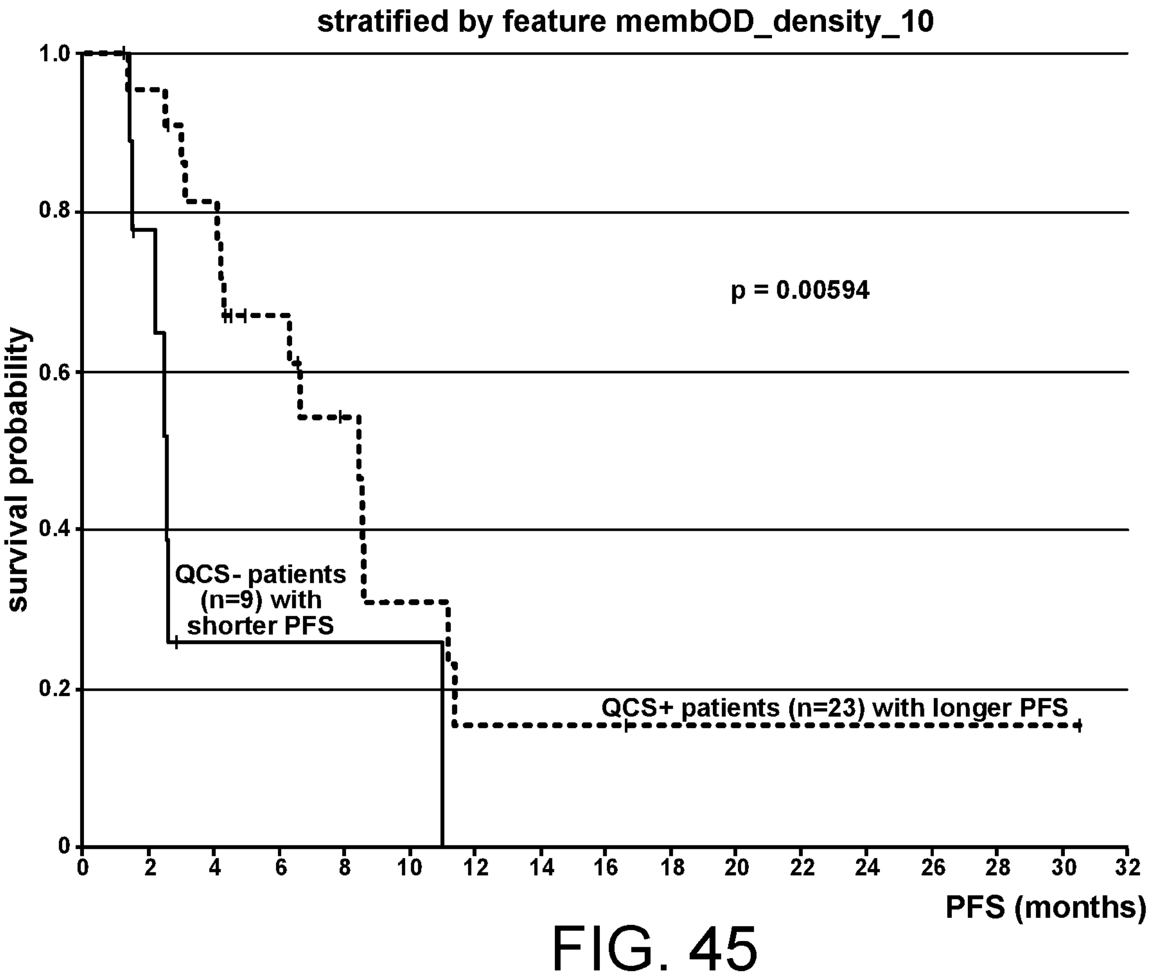


FIG. 45

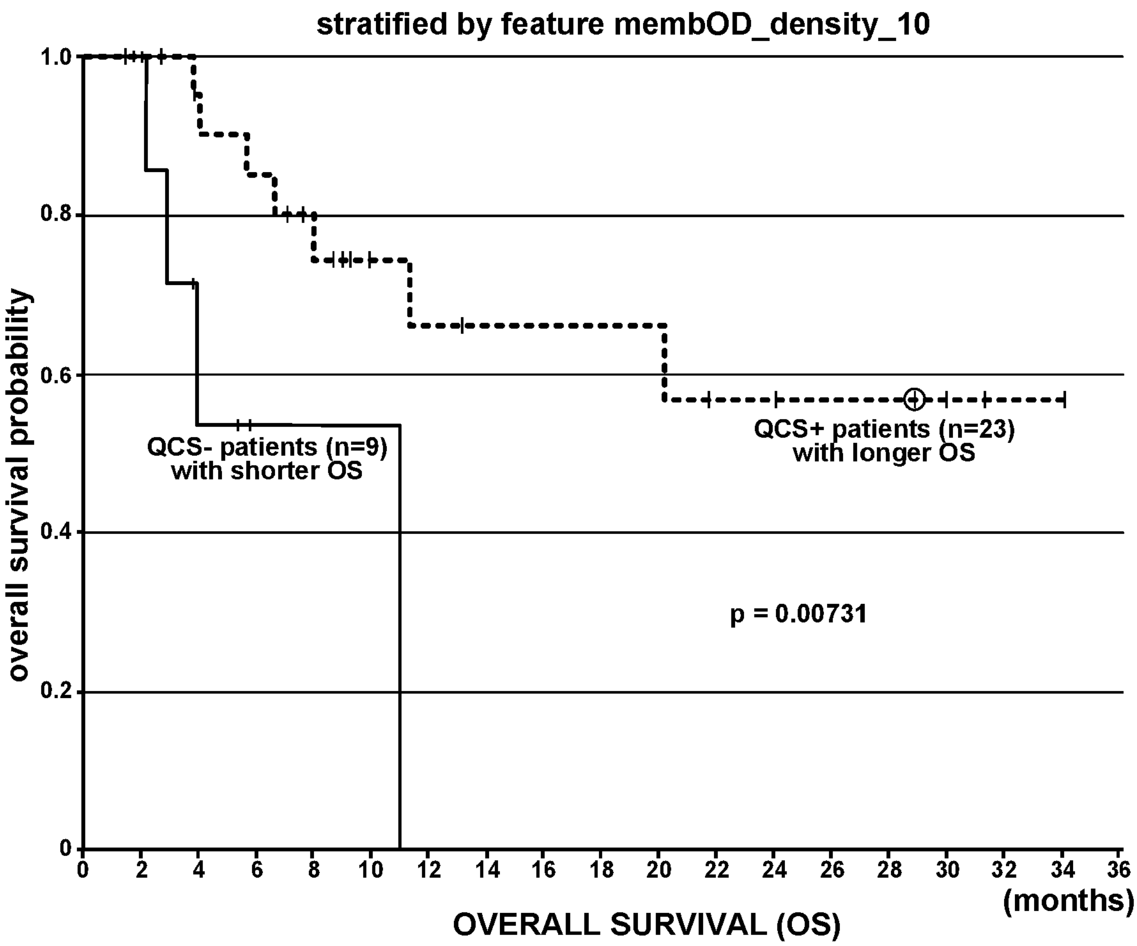
stratified by feature membOD_density_10
1.0
0.8
0.6
0.4
0.2
0
overall survival probability
QCS- patients (n=9)
with shorter OS
QCS+ patients (n=23)
with longer OS
p = 0.00731
0 2 4 6 8 10 12 14 16 18 20 22 24 26 28 30 32 34 36
(months)
OVERALL SURVIVAL (OS)

FIG. 46

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 63077604
- WO 2015115091 A
- WO 9007861 A
- US 5821337 A
- WO 9954342 A
- WO 0061739 A
- WO 0231140 A